(19) Europäisches Patentamt
European Patent Office
Office européen des brevets

(11) Veröffentlichungsnummer: **0 519 211 A1**

# (12) EUROPÄISCHE PATENTANMELDUNG

(21) Anmeldenummer: **92108143.6**

(22) Anmeldetag: **14.05.92**

(51) Int. Cl.5: **C07D 239/42**, C07D 239/46, C07D 239/48, C07D 239/52, C07D 401/12, A01N 43/54

(30) Priorität: **17.05.91 DE 4116089**

(43) Veröffentlichungstag der Anmeldung: **23.12.92 Patentblatt 92/52**

(84) Benannte Vertragsstaaten: **AT BE CH DE DK ES FR GB GR IT LI NL**

(71) Anmelder: **HOECHST AKTIENGESELLSCHAFT**
**Postfach 80 03 20**
**W-6230 Frankfurt am Main 80(DE)**

(72) Erfinder: **Schaper, Wolfgang, Dr.**
**Kapellenweg 5c**
**W-8901 Diedorf(DE)**
Erfinder: **Salbeck, Gerhard, Dr.**
**Königsberger Strasse 52**
**W-6239 Kriftel(DE)**
Erfinder: **Ehrhardt, Heinz, Dr.**
**Bergstrasse 21**
**W-8901 Rehling(DE)**
Erfinder: **Braun, Peter, Dr.**
**Pfarrer-Dorn-Strasse 13**
**W-6500 Mainz(DE)**
Erfinder: **Knauf, Werner, Dr.**
**Im Kirschgarten 24**
**W-6239 Eppstein/Ts(DE)**
Erfinder: **Sachse, Burkhard, Dr.**
**An der Ziegelei 30**
**W-6233 Kelkheim (Taunus)(DE)**
Erfinder: **Waltersdorfer, Anna, Dr.**
**Rauenthaler Weg 28**
**W-6000 Frankfurt am Main 71(DE)**
Erfinder: **Kern, Manfred, Dr.**
**Im Traminerweg 8**
**W-6501 Lörzweiler(DE)**
Erfinder: **Lümmen, Peter, Dr.**
**Rautenweg 1**
**W-6272 Niedernhausen(DE)**

(54) **Substituierte 4-Aminopyrimidine, Verfahren zu ihrer Herstellung und ihre Verwendung als Schädlingsbekämpfungsmittel.**

(57) Die Erfindung betrifft substituierte 4-Aminopyrimidine der allgemeinen Formel

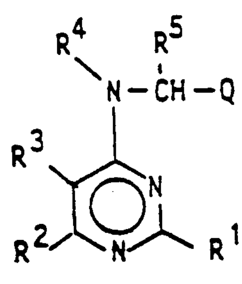

worin bedeuten:

- $R^1$ Wasserstoff, Halogen, Alkyl oder Cycloalkyl;
- $R^2$ Wasserstoff, Alkyl, Halogen, Trifluormethyl, Alkoxy, Halogenalkoxy, Alkoxyalkyl, Alkylthio, Alkylthioalkyl, Alkylamino, Dialkylamino oder Cycloalkylamino;
- $R^3$ Wasserstoff, Alkyl, Alkoxy, Halogenalkoxy, Alkylthio, Halogen, Nitro oder Dialkylamino;
- $R^4$ Wasserstoff oder gegebenenfalls substituiertes Carbamoyl;

EP 0 519 211 A1

$R^5$ Wasserstoff, $(C_1-C_8)$Alkyl oder $(C_3-C_6)$Cycloalkyl; und
Q die in der Beschreibung definierte Bedeutung hat, sowie deren Salze. Die Erfindung betrifft weiterhin Verfahren zu deren Herstellung und deren Verwendung als Schädlingsbekämpfungsmittel.

Die Erfindung betrifft neue substituierte 4-Aminopyrimidine, Verfahren zu ihrer Herstellung, sowie ihre Verwendung als Schädlingsbekämpfungsmittel, insbesondere als Insektizid, Akarizid, Nematozid und Fungizid.

Es ist bereits bekannt, daß bestimmte substituierte 4-Aminopyrimidine gute fungizide, akarizide und insektizide Wirkung besitzen (vgl. EP-OS 276406, EP-OS 196524, EP-OS 264217, EP-OS 57440, EP-OS 323757, EP-OS 356158, EP-OS 370704). Die biologische Wirkung dieser Verbindungen ist jedoch insbesondere bei niedrigen Aufwandmengen und Konzentrationen nicht in allen Anwendungsbereichen zufriedenstellend.

Gegenstand der Erfindung sind substituierte 4-Aminopyrimidine der allgemeinen Formel I

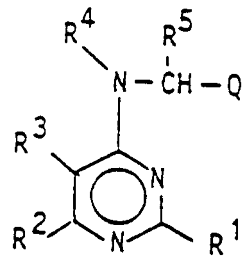

(I),

worin

$R^1$ Wasserstoff, Halogen, $(C_1\text{-}C_4)$Alkyl oder $(C_3\text{-}C_6)$Cycloalkyl bedeutet,

$R^2$ Wasserstoff, $(C_1\text{-}C_4)$Alkyl, Halogen, Trifluormethyl, $(C_1\text{-}C_4)$Alkoxy, $(C_1\text{-}C_4)$Halogenalkoxy, $(C_1\text{-}C_4)$-Alkoxy-$(C_1\text{-}C_4)$alkyl, $(C_1\text{-}C_4)$Alkylthio, $(C_1\text{-}C_4)$Alkylthio-$(C_1\text{-}C_4)$alkyl, $(C_1\text{-}C_4)$Alkylamino, $(C_1\text{-}C_4)$-Dialkylamino oder $(C_3\text{-}C_6)$Cycloalkylamino bedeutet,

$R^3$ Wasserstoff, $(C_1\text{-}C_4)$Alkyl, $(C_1\text{-}C_4)$Alkoxy, $(C_1\text{-}C_4)$Halogenalkoxy, $(C_1\text{-}C_4)$Alkylthio, Halogen, Nitro oder $(C_1\text{-}C_4)$Dialkylamino bedeutet, mit der Maßgabe, daß wenn Q keine der später aufgeführten Gruppen der Bedeutung $Q^1$ oder der allgemeinen Formeln II', II'', II''' oder II'''' ist und $R^3$ eine $(C_1\text{-}C_4)$Alkylgruppe oder ein Halogenatom bedeutet, $R^2$ nicht gleichzeitig $(C_1\text{-}C_4)$Alkyl, Halogen, $(C_1\text{-}C_4)$Alkoxy-$(C_1\text{-}C_4)$alkyl oder

$(C_1\text{-}C_4)$Alkylthio-$(C_1\text{-}C_4)$alkyl bedeutet, oder, falls Q die Bedeutung $Q^1$ hat und $R^2$ eine Ethylgruppe bedeutet, $R^3$ nicht gleichzeitig Halogen bedeutet, oder, falls Q die Bedeutung $Q_1$ hat und $R^4$ einen Rest der Formel

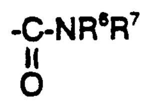

und $R^2$ $(C_1\text{-}C_4)$Alkyl oder Halogen bedeuten, $R^3$ nicht gleichzeitig $(C_1\text{-}C_4)$Alkyl oder Halogen ist, oder,

falls Q ein Rest der allgemeinen Bedeutung $Q^1$ oder der Formel II ist, für die E eine direkte Bindung oder Methylenoxy ist, oder der Formeln II', II'', II''' oder II'''' oder der Bedeutung $Q^3$ ist, für die $R^{14}$ keine Gruppe der allgemeinen Bedeutung IV ist, $R^2$ und $R^3$ auch zusammen mit den Kohlenstoffatomen an das sie gebunden sind, einen ungesättigten 5-gliedrigen Ring bilden können, der ein an den Pyrimidin-Ring gebundenes Sauerstoff- oder Schwefelatom enthält und der gegebenenfalls durch Alkyl oder Halogen substituiert ist,

$R^4$ Wasserstoff oder einen Rest

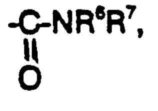

worin $R^6$ oder $R^7$ gleich oder verschieden sind und jeweils Wasserstoff, $(C_1\text{-}C_4)$Alkyl, Phenyl oder Phenyl-$(C_1\text{-}C_4)$alkyl bedeuten,

wobei die beiden vorgenannten Phenylgruppen unsubstituiert oder mit einem oder zwei Substituenten versehen sind und diese Substituenten jeweils Halogen, $(C_1\text{-}C_4)$Alkyl, $(C_1\text{-}C_4)$Halogenalkyl, $(C_1\text{-}C_4)$Alkoxy, $(C_1\text{-}C_4)$-Alkylthio oder Nitro sein können, oder $R^6$ und $R^7$ zusammen mit dem Stickstoffatom an das sie gebunden sind einen gesättigten oder ungesättigten 5- oder 6-gliedrigen Ring bilden, der zusätzlich weitere Stickstoffatome oder ein Sauerstoff- oder Schwefelatom enthalten kann, wobei dieser Ring mit einem Benzolring kondensiert und mit einem oder zwei Substituenten versehen sein kann und diese Substituenten jeweils $(C_1\text{-}C_4)$Alkyl, Trifluormethyl, Halogen, $(C_1\text{-}C_4)$Alkoxy, $(C_1\text{-}C_4)$Alkylthio oder Nitro sein können,

$R^5$ Wasserstoff, $(C_1\text{-}C_8)$Alkyl oder $(C_3\text{-}C_6)$Cycloalkyl bedeutet,

Q die Bedeutung $Q^1$ hat und

$Q^1$ $(C_1\text{-}C_{15})$Alkyl bedeutet, gegebenenfalls substituiert mit einem, zwei oder drei Halogenatomen, einer $(C_1\text{-}C_{15})$Alkoxygruppe,
einer $(C_4\text{-}C_8)$Cycloalkylalkoxygruppe, einer Dioxolanylgruppe, einer $(C_1\text{-}C_4)$Alkoxy-$(C_1\text{-}C_4)$-alkoxygruppe, einer Hydroxygruppe, einer Methoxycarbonylgruppe, einer $(C_3\text{-}C_6)$-Cycloalkylgruppe, einer 2-[2-($(C_1\text{-}C_4)$Alkoxy)ethoxy]ethoxygruppe, einer Pyranyloxygruppe, einer Imidazol-1-ylgruppe, einer Triazol-1-ylgruppe, einer Pyrazol-1-ylgruppe oder einer Gruppe -A-B-$R^8$, worin A Sauerstoff, Schwefel oder Imino, B Carbonyl, Thiocarbonyl oder Sulfonyl bedeutet und $R^8$ $(C_1\text{-}C_4)$Alkyl,
$(C_3\text{-}C_6)$Cycloalkyl, $(C_1\text{-}C_4)$Halogenalkyl, $(C_1\text{-}C_4)$Alkoxy, eine substituierte oder unsubstituierte Phenoxygruppe, eine substituierte oder unsubstituierte Phenylgruppe, eine substituierte oder unsubstituierte Anilinogruppe, eine 2,6-Dimethylmorpholin-4-yl-, 4-Methylpiperazin-1-yl-, Imidazol-1-yl-, Triazol-1-yl-, Pyrazol-1-ylgruppe oder eine Mono- oder Di-$(C_1\text{-}C_4)$alkylaminogruppe bedeutet, oder

Q die Bedeutung $Q^2$ hat und

$Q^2$ eine Gruppe der allgemeinen Formeln II, II', II'', II''' oder II'''' bedeutet

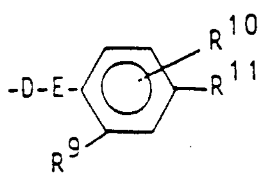

II ,

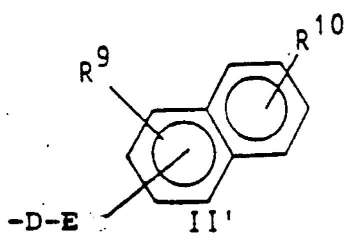

II' ,

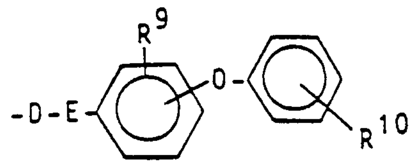

II''

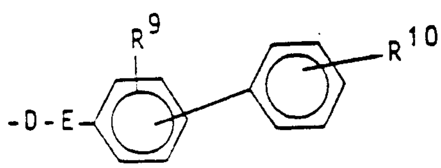

II'''

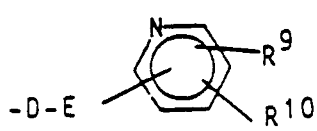

II'''' ,

worin

D eine $(C_1\text{-}C_6)$Alkylengruppe bedeutet,

E eine direkte Bindung, Sauerstoff oder Methylenoxy bedeutet,

$R^9$ und $R^{10}$ gleich oder verschieden sind und jeweils Wasserstoff, Halogen, $(C_1\text{-}C_4)$Alkyl, $(C_3\text{-}C_6)$-Cycloalkyl, $(C_1\text{-}C_4)$Halogenalkyl, $C_3$- oder $C_4$-Alkenyl, $(C_1\text{-}C_4)$Alkoxy, $(C_1\text{-}C_4)$Alkylthio oder Nitro bedeuten,

$R^{11}$ die für $R^9$ und $R^{10}$ angegebenen Bedeutungen hat, sowie für den Fall, daß E Sauerstoff bedeutet, zusätzlich eine Gruppe der Formel X-Y-$(GO)_n$-$R^{12}$ bedeutet, worin

X eine $(C_1\text{-}C_8)$Alkylengruppe oder eine $(C_1\text{-}C_8)$Alkylengruppe mit einem $(C_1\text{-}C_4)$-Alkoxysubstituenten bedeutet,

Y Sauerstoff, Schwefel oder eine Iminogruppe bedeutet,

G eine $(C_1\text{-}C_8)$Alkylengruppe oder eine $(C_1\text{-}C_4)$Alkylenoxy-$(C_1\text{-}C_4)$alkylengruppe bedeutet,

| | |
|---|---|
| n | Null oder 1 ist, |
| $R^{12}$ | $(C_1\text{-}C_4)$Alkyl, $C_3$- oder $C_4$-Alkenyl, $(C_4\text{-}C_6)$Alkadienyl, $C_3$- oder $C_4$-Alkinyl, Phenyl-$(C_1\text{-}C_3)$alkyl oder eine Gruppe der Formel $CH_2$-W bedeutet, worin |
| W | eine Gruppe der Formel CH = N-$OR^{13}$ bedeutet, worin |
| $R^{13}$ | Wasserstoff, $(C_1\text{-}C_4)$Alkyl, $C_3$- oder $C_4$-Alkenyl oder Phenyl-$(C_1\text{-}C_3)$alkyl bedeutet, |
| W | weiterhin eine Morpholinomethylgruppe oder eine heterocyclische Gruppe mit 5 bis 8 Ringatomen bedeutet, von denen 2 oder 3 Sauerstoff- oder Schwefelatome sind, und der besagte Heterocyclus gesättigt ist oder eine Doppelbindung im Ring besitzt und der besagte Heterocyclus unsubstituiert ist oder einen oder zwei Alkyl-, $(C_1\text{-}C_4)$Halogenalkyl- oder Phenylsubstituenten trägt, oder |
| Q | die Bedeutung $Q^3$ hat und |
| $Q^3$ | eine Gruppe der allgemeinen Formel III bedeutet |

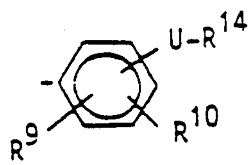

(III),

worin $R^9$ und $R^{10}$ die oben angegebenen Bedeutungen haben, und

| | |
|---|---|
| U | eine direkte Bindung, Sauerstoff, Schwefel, $(C_1\text{-}C_3)$Alkylen, oder $(C_1\text{-}C_3)$Alkylenoxy bedeutet, |
| $R^{14}$ | Phenyl oder einen Heterocyclus bedeutet, wobei jeder der beiden vorgenannten Reste unsubstituiert oder mit einem oder zwei Substituenten versehen sein kann und diese Substituenten gleich oder verschieden sind und jeweils Halogen, $(C_1\text{-}C_4)$Alkyl, $(C_1\text{-}C_4)$-Halogenalkyl, Nitro, $(C_1\text{-}C_4)$Alkoxy, $(C_1\text{-}C_4)$Alkylthio, $(C_1\text{-}C_4)$Alkoxy-$(C_1\text{-}C_4)$alkyl, Phenyl, Phenoxy, Halogenphenoxy oder $(C_1\text{-}C_4)$Alkylphenoxy bedeuten, |
| $R^{14}$ | weiterhin, für den Fall, daß U Sauerstoff ist, $(C_5\text{-}C_{10})$Alkyl, Allyl, Geranyl, Farnesyl, $(C_1\text{-}C_4)$Halogenalkyl,$(C_3\text{-}C_6)$Cycloalkylmethyl, eine Ethylgruppe, die in 2-Stellung durch $(C_1\text{-}C_4)$Alkoxy, $(C_1\text{-}C_4)$Alkoxy-$(C_1\text{-}C_4)$alkyl, $(C_1\text{-}C_4)$Alkylthio, $(C_1\text{-}C_4)$Alkylsulfonyl oder eine Phenoxygruppe, die gegebenenfalls mit einer oder zwei $(C_1\text{-}C_4)$Alkylgruppen substituiert sein kann, Glycidyl, Acetonyl, Halogenphenoxymethyldioxolanyl, 2,2-Diethoxyethyl, 1-Ethoxycarbonylmethyl, Trimethylsilylmethyl, 1-Pyridylethyl oder eine $(C_1\text{-}C_4)$Alkylgruppe, die mit einer $(C_1\text{-}C_4)$Alkoxyimino- oder einer Benzyloxyiminogruppe substituiert ist, substituiert ist, bedeutet, |
| $R^{14}$ | weiterhin, für den Fall, daß U Sauerstoff ist, eine Gruppe der allgemeinen Formel IV |

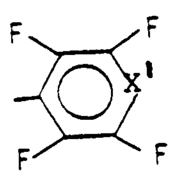

(IV)

bedeutet, worin

| | |
|---|---|
| X' | Stickstoff oder eine Gruppe $CR^{15}$ bedeutet, worin |
| $R^{15}$ | Wasserstoff, Halogen, $(C_1\text{-}C_4)$Alkyl, $(C_1\text{-}C_4)$Halogenalkyl, $(C_1\text{-}C_4)$Alkanoyl, Nitro, Cyano oder 1,3-Dioxolan-2-yl bedeutet, sowie ihre Salze und ihre Stereoisomeren. |

Bevorzugt sind solche Verbindungen der Formel I, worin

| | |
|---|---|
| $R^1$ | Wasserstoff, Methyl oder Halogen bedeutet, |
| $R^2$ | Wasserstoff, Methyl, Ethyl, Halogen, Trifluormethyl $(C_1\text{-}C_4)$Alkoxy, $(C_1\text{-}C_4)$Halogenalkoxy, Methoxymethyl, Dimethylamino oder Diethylamino bedeutet, |
| $R^3$ | Wasserstoff, $(C_1\text{-}C_4)$Alkyl, $(C_1\text{-}C_4)$Alkoxy, $(C_1\text{-}C_4)$Halogenalkoxy oder Halogen bedeutet, mit der Maßgabe, daß wenn Q keine der später aufgeführten Gruppen der Bedeutung $Q^1$ |

oder der allgemeinen Formeln II', II'', II''' oder II'''' ist und $R^3$ eine $(C_1\text{-}C_4)$Alkylgruppe oder ein Halogenatom bedeutet, $R^2$ nicht gleichzeitig Methyl, Ethyl, Halogen oder Methoxymethyl bedeutet, oder, falls Q die Bedeutung $Q^1$ hat und $R^2$ eine Ethylgruppe bedeutet, $R^3$ nicht gleichzeitig Halogen bedeutet, oder falls Q die Bedeutung $Q^1$ hat und $R^4$ ein Rest der Formel

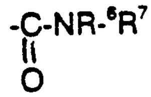

und $R^2$Methyl, Ethyl oder Halogen bedeuten, $R^3$ nicht gleichzeitig $(C_1\text{-}C_4)$Alkyl oder Halogen ist,

$R^4$ Wasserstoff oder einen Rest

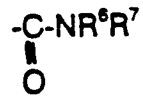

bedeutet,
worin $R^6$ oder $R^7$ gleich oder verschieden sind und jeweils Wasserstoff, $(C_1\text{-}C_4)$Alkyl, Phenyl oder Phenyl-$(C_1\text{-}C_4)$-alkyl bedeuten,
wobei die beiden vorgenannten Phenylgruppen unsubstituiert oder mit einem oder zwei Substituenten versehen sein können und diese Substituenten jeweils Halogen, $(C_1\text{-}C_4)$-Alkyl, $(C_1\text{-}C_4)$Halogenalkyl, $(C_1\text{-}C_4)$Alkoxy, $(C_1\text{-}C_4)$Alkylthio oder Nitro sein können, oder $R^6$ und $R^7$ zusammen mit dem Stickstoffatom an das sie gebunden sind einen gesättigten oder ungesättigten 5- oder 6-gliedrigen Ring bilden, der zusätzlich weitere Stickstoffatome oder ein Sauerstoff- oder Schwefelatom enthalten kann, wobei dieser Ring mit einem Benzolring kondensiert und mit einem oder zwei Substituenten versehen sein kann und diese Substituenten jeweils $(C_1\text{-}C_4)$Alkyl, Trifluormethyl, Halogen, $(C_1\text{-}C_4)$-Alkoxy, $(C_1\text{-}C_4)$Alkylthio oder Nitro sein können,

$R^5$ Wasserstoff, $(C_1\text{-}C_8)$Alkyl oder Cyclopropyl bedeutet,

Q die Bedeutung $Q^1$ hat und

$Q^1$ $(C_1\text{-}C_{15})$Alkyl bedeutet, gegebenenfalls substituiert mit einem, zwei oder drei Halogenatomen, einer $(C_1\text{-}C_{15})$Alkoxygruppe,
einer $(C_4\text{-}C_8)$Cycloalkylalkoxygruppe, einer Dioxolanylgruppe, einer $(C_1\text{-}C_4)$Alkoxy-$(C_1\text{-}C_4)$alkoxygruppe, einer Hydroxygruppe, einer Methoxycarbonylgruppe, einer $(C_3\text{-}C_6)$-Cycloalkylgruppe, einer 2-[2-($(C_1\text{-}C_4)$Alkoxy)ethoxy]ethoxygruppe, einer Pyranyloxygruppe, einer Imidazol-1-ylgruppe, einer Triazol-1-ylgruppe, einer Pyrazol-1-ylgruppe oder einer Gruppe -A-B-$R^8$, worin A Sauerstoff, Schwefel oder Imino, B Carbonyl, Thiocarbonyl oder Sulfonyl bedeutet und $R^8$ $(C_1\text{-}C_4)$Alkyl,
$(C_3\text{-}C_6)$Cycloalkyl, $(C_1\text{-}C_4)$Halogenalkyl, $(C_1\text{-}C_4)$Alkoxy, eine substituierte oder unsubstituierte Phenoxygruppe, eine substituierte oder unsubstituierte Phenylgruppe, eine substituierte oder unsubstituierte Anilinogruppe, eine 2,6-Dimethylmorpholin-4-yl-, 4-Methylpiperazin-1-yl-, Imidazol-1-yl-, Triazol-1-yl-, Pyrazol-1-ylgruppe oder eine Mono- oder Di-$(C_1\text{-}C_4)$alkylaminogruppe bedeutet, oder

Q die Bedeutung $Q^2$ hat und

$Q^2$ eine Gruppe der allgemeinen Formeln II, II', II'', II''' oder II'''' bedeutet, worin

D eine $(C_1\text{-}C_6)$Alkylengruppe bedeutet,

E eine direkte Bindung, Sauerstoff oder Methylenoxy bedeutet,

$R^9$ und $R^{10}$ gleich oder verschieden sind und jeweils Wasserstoff, Halogen, $(C_1\text{-}C_4)$Alkyl, $(C_3\text{-}C_6)$-Cycloalkyl, $(C_1\text{-}C_4)$Halogenalkyl, $C_3$- oder $C_4$-Alkenyl, $(C_1\text{-}C_4)$Alkoxy, $(C_1\text{-}C_4)$Alkylthio oder Nitro bedeuten,

$R^{11}$ die für $R^9$ und $R^{10}$ angegebenen Bedeutungen hat, sowie für den Fall, daß E Sauerstoff bedeutet, zusätzlich eine Gruppe der Formel X-Y-$(GO)_n$-$R^{12}$ bedeutet, worin

X eine $(C_1\text{-}C_8)$Alkylengruppe oder eine $(C_1\text{-}C_8)$Alkylengruppe mit einem $(C_1\text{-}C_4)$-Alkoxysubsituenten bedeutet,

| | |
|---|---|
| Y | Sauerstoff, Schwefel oder eine Iminogruppe bedeutet, |
| G | eine $(C_1-C_8)$Alkylengruppe oder eine $(C_1-C_4)$Alkylenoxy-$(C_1-C_4)$alkylengruppe bedeutet, |
| n | Null oder 1 ist, |
| $R^{12}$ | $(C_1-C_4)$Alkyl, $C_3$- oder $C_4$-Alkenyl, $(C_4-C_6)$Alkadienyl, $C_3$- oder $C_4$-Alkinyl, Phenyl-$(C_1-C_3)$alkyl oder eine Gruppe der Formel $CH_2$-W bedeutet, worin |
| W | eine Gruppe der Formel $CH=N-OR^{13}$ bedeutet, worin |
| $R^{13}$ | Wasserstoff, $(C_1-C_4)$Alkyl, $C_3$- oder $C_4$-Alkenyl oder Phenyl-$(C_1-C_3)$alkyl bedeutet, |
| W | weiterhin eine Morpholinomethylgruppe oder eine heterocyclische Gruppe mit 5 bis 8 Ringatomen bedeutet, von denen 2 oder 3 Sauerstoff- oder Schwefelatome sind, und der besagte Heterocyclus gesättigt ist oder eine Doppelbindung im Ring besitzt und der besagte Heterocyclus unsubstituiert ist oder einen oder zwei Alkyl-, $(C_1-C_4)$Halogenalkyl- oder Phenylsubstituenten trägt, oder |
| Q | die Bedeutung $Q^3$ hat und |
| $Q^3$ | eine Gruppe der allgemeinen Formel III bedeutet,worin $R^9$ und $R^{10}$ die oben angegebenen Bedeutungen haben, |
| U | eine direkte Bindung, Sauerstoff, Schwefel, $(C_1-C_3)$Alkylen oder $(C_1-C_3)$Alkylenoxy bedeutet, und |
| $R^{14}$ | Phenyl oder einen Heterocyclus bedeutet, wobei jeder der beiden vorgenannten Reste unsubstituiert oder mit einem oder zwei Substituenten versehen sein kann und diese Substituenten gleich oder verschieden sind und jeweils Halogen, $(C_1-C_4)$Alkyl, $(C_1-C_4)$-Halogenalkyl, Nitro, $(C_1-C_4)$Alkoxy, $(C_1-C_4)$Alkylthio, $(C_1-C_4)$Alkoxy-$(C_1-C_4)$alkyl, Phenyl, Phenoxy, Halogenphenoxy oder $(C_1-C_4)$Alkylphenoxy bedeuten, |
| $R^{14}$ | weiterhin, für den Fall, daß U Sauerstoff ist, $(C_5-C_{10})$Alkyl, Allyl, Geranyl, Farnesyl, $(C_1-C_4)$Halogenalkyl, $(C_3-C_6)$Cycloalkylmethyl, eine Ethylgruppe, die in 2-Stellung durch $(C_1-C_4)$Alkoxy, $(C_1-C_4)$Alkoxy-$(C_1-C_4)$alkyl, $(C_1-C_4)$Alkylthio, $(C_1-C_4)$-Alkylsulfonyl oder eine Phenoxygruppe, die gegebenenfalls mit einer oder zwei $(C_1-C_4)$Alkylgruppen substituiert sein kann, Glycidyl, Acetonyl, Halogenphenoxymethyldioxolanyl, 2,2-Diethoxyethyl, 1-Ethoxycarbonylmethyl, Trimethylsilylmethyl, 1-Pyridylethyl oder eine $(C_1-C_4)$Alkylgruppe, die mit einer $(C_1-C_4)$Alkoxyimino- oder einer Benzyloxyiminogruppe substituiert ist, substituiert ist, bedeutet, |
| $R^{14}$ | weiterhin, für den Fall daß U Sauerstoff ist, eine Gruppe der allgemeinen Formel IV bedeutet, worin |
| X' | Stickstoff oder eine Gruppe $CR^{15}$ bedeutet, worin |
| $R^{15}$ | Wasserstoff, Halogen, $(C_1-C_4)$Alkyl, $(C_1-C_4)$Halogenalkyl, $(C_1-C_4)$Alkanoyl, Nitro, Cyano oder 1,3-Dioxolan-2-yl bedeutet, sowie ihre Salze und ihre Stereoisomeren. |

Stärker bevorzugt sind solche Verbindungen der Formel I, worin

| | |
|---|---|
| $R^1$ | Wasserstoff, Methyl oder Halogen bedeutet, |
| $R^2$ | Wasserstoff, Methyl, Ethyl, Halogen, Trifluormethyl, $(C_1-C_4)$Alkoxy, $(C_1-C_4)$-Halogenalkoxy, Methoxymethyl, Dimethylamino oder Diethylamino bedeutet, |
| $R^3$ | Wasserstoff, $(C_1-C_4)$Alkyl, $(C_1-C_4)$Alkoxy, $(C_1-C_4)$Halogenalkoxy oder Halogen bedeutet, mit der Maßgabe, daß wenn Q keine der später aufgeführten Gruppen der Bedeutung $Q^1$ oder der allgemeinen Formeln II', II'', II''' oder II'''' ist und $R^3$ eine $(C_1-C_4)$Alkylgruppe oder ein Halogenatom bedeutet, $R^2$ nicht gleichzeitig Methyl, Ethyl, Halogen oder Methoxymethyl bedeutet, oder, falls Q die Bedeutung $Q^1$ hat und $R^2$ eine Ethylgruppe bedeutet, $R^3$ nicht gleichzeitig Halogen bedeutet, |
| $R^4$ | Wasserstoff bedeutet, |
| $R^5$ | Wasserstoff, $(C_1-C_4)$Alkyl oder Cyclopropyl bedeutet, |
| Q | die Bedeutung $Q^1$ hat und |
| $Q^1$ | $(C_1-C_{15})$Alkyl bedeutet, gegebenenfalls substituiert mit einem, zwei oder drei Halogenatomen, einer $(C_1-C_{15})$Alkoxygruppe, einer $(C_4-C_8)$Cycloalkylalkoxygruppe, einer Dioxolanylgruppe, einer $(C_1-C_4)$Alkoxy-$(C_1-C_4)$alkoxygruppe, einer Hydroxygruppe, einer Methoxycarbonylgruppe, einer $(C_3-C_6)$-Cycloalkylgruppe, einer 2-[2-($(C_1-C_4)$Alkoxy)ethoxy]ethoxygruppe, einer Pyranyloxygruppe, einer Imidazol-1-ylgruppe, einer Triazol-1-ylgruppe, einer Pyrazol-1-ylgruppe oder einer Gruppe $-A-B-R^8$, worin A Sauerstoff, Schwefel oder Imino, B Carbonyl, Thiocarbonyl oder Sulfonyl bedeutet und $R^8$ $(C_1-C_4)$Alkyl, $(C_3-C_6)$Cycloalkyl, $(C_1-C_4)$Halogenalkyl, $(C_1-C_4)$Alkoxy, eine substituierte oder unsubsti- |

tuierte Phenoxygruppe, eine substituierte oder unsubstituierte Phenylgruppe, eine substituierte oder unsubstituierte Anilinogruppe, eine 2,6-Dimethylmorpholin-4-yl-, 4-Methylpiperazin-1-yl-, Imidazol-1-yl-, Triazol-1-yl-, Pyrazol-1-ylgruppe oder eine Mono- oder Di-($C_1$-$C_4$)alkylaminogruppe bedeutet oder

| | |
|---|---|
| Q | die Bedeutung $Q^2$ hat und |
| $Q^2$ | eine Gruppe der allgemeinen Formeln II, II', II'', II''' oder II'''' bedeutet, worin |
| D | eine ($C_1$-$C_6$)Alkylengruppe bedeutet, |
| E | eine direkte Bindung, Sauerstoff oder Methylenoxy bedeutet, |
| $R^9$ und $R^{10}$ | gleich oder verschieden sind und jeweils Wasserstoff, Halogen, ($C_1$-$C_4$)Alkyl, ($C_3$-$C_6$)-Cycloalkyl, ($C_1$-$C_4$)Halogenalkyl, $C_3$- oder $C_4$-Alkenyl, ($C_1$-$C_4$)Alkoxy, ($C_1$-$C_4$)Alkylthio oder Nitro bedeuten, |
| $R^{11}$ | die für $R^9$ und $R^{10}$ angegebenen Bedeutungen hat, sowie für den Fall, daß E Sauerstoff bedeutet, zusätzlich eine Gruppe der Formel X-Y-$(GO)_n$-$R^{12}$ bedeutet, worin |
| X | eine ($C_1$-$C_8$)Alkylengruppe oder eine ($C_1$-$C_8$)Alkylengruppe mit einem ($C_1$-$C_4$)-Alkoxysubsituenten bedeutet, |
| Y | Sauerstoff, Schwefel oder eine Iminogruppe bedeutet, |
| G | eine ($C_1$-$C_8$)Alkylengruppe oder eine ($C_1$-$C_4$)Alkylenoxy-($C_1$-$C_4$)alkylengruppe bedeutet, |
| n | Null oder 1 ist, |
| $R^{12}$ | ($C_1$-$C_4$)Alkyl, $C_3$- oder $C_4$-Alkenyl, ($C_4$-$C_6$)Alkadienyl, $C_3$- oder $C_4$-Alkinyl, Phenyl-($C_1$-$C_3$)alkyl oder eine Gruppe der Formel $CH_2$-W bedeutet, worin |
| W | eine Gruppe der Formel CH=N-$OR^{13}$ bedeutet, worin |
| $R^{13}$ | Wasserstoff, ($C_1$-$C_4$)Alkyl, $C_3$- oder $C_4$-Alkenyl oder Phenyl-($C_1$-$C_3$)alkyl bedeutet, |
| W | weiterhin eine Morpholinomethylgruppe oder eine heterocyclische Gruppe mit 5 bis 8 Ringatomen bedeutet, von denen 2 oder 3 Sauerstoff- oder Schwefelatome sind, und der besagte Heterocyclus gesättigt ist oder eine Doppelbildung im Ring besitzt und der besagte Heterocyclus unsubstituiert ist oder einen oder zwei Alkyl-, ($C_1$-$C_4$)Halogenalkyl- oder Phenylsubstituenten trägt, oder |
| Q | die Bedeutung $Q^3$ hat und |
| $Q^3$ | eine Gruppe der allgemeinen Formel III bedeutet, worin $R^9$ und $R^{10}$ die oben angegebenen Bedeutungen haben, |
| U | eine direkte Bindung, Sauerstoff, Schwefel, ($C_1$-$C_3$)Alkylen oder ($C_1$-$C_3$)-Alkylenoxy bedeutet, und |
| $R^{14}$ | Phenyl oder einen Heterocyclus bedeutet, wobei jeder der beiden vorgenannten Reste unsubstituiert oder mit einem oder zwei Substituenten versehen sein kann und diese Substituenten gleich oder verschieden sind und jeweils Halogen, ($C_1$-$C_4$)Alkyl, ($C_1$-$C_4$)-Halogenalkyl, Nitro,<br>($C_1$-$C_4$)Alkoxy, ($C_1$-$C_4$)Alkylthio, ($C_1$-$C_4$)Alkoxy-($C_1$-$C_4$)alkyl, Phenyl, Phenoxy, Halogenphenoxy oder ($C_1$-$C_4$)Alkylphenoxy bedeuten, |
| $R^{14}$ | weiterhin, für den Fall, daß U Sauerstoff bedeutet, ($C_5$-$C_{10}$)Alkyl, Allyl, Geranyl, Farnesyl, ($C_1$-$C_4$)Halogenalkyl, ($C_3$-$C_6$)Cycloalkylmethyl, eine Ethylgruppe, die in 2-Stellung durch ($C_1$-$C_4$)Alkoxy, ($C_1$-$C_4$)Alkoxy-($C_1$-$C_4$)alkyl, ($C_1$-$C_4$)-Alkylthio, ($C_1$-$C_4$)Alkylsulfonyl oder eine Phenoxygruppe, die gegebenenfalls mit einer oder zwei ($C_1$-$C_4$)Alkylgruppen substituiert sein kann, Glycidyl, Acetonyl, Halogenphenoxymethyldioxolanyl, 2,2-Diethoxyethyl, 1-Ethoxycarbonylmethyl, Trimethylsilylmethyl, 1-Pyridylethyl oder eine ($C_1$-$C_4$)-Alkylgruppe, die mit einer ($C_1$-$C_4$)Alkoxyimino- oder einer Benzyloxyiminogruppe substituiert ist, substituiert ist, bedeutet, |
| $R^{14}$ | weiterhin, für den Fall daß U Sauerstoff ist, eine Gruppe der allgemeinen Formel IV bedeutet, worin |
| X' | Stickstoff oder eine Gruppe $CR^{15}$ bedeutet, worin |
| $R^{15}$ | Wasserstoff, Halogen, ($C_1$-$C_4$)Alkyl, ($C_1$-$C_4$)Halogenalkyl, ($C_1$-$C_4$)Alkanoyl, Nitro, Cyano oder 1,3-Dioxolan-2-yl bedeutet, sowie ihre Salze und ihre Stereoisomeren. |

Stärker bervorzugt sind weiterhin Verbindungen der Formel I, worin

| | |
|---|---|
| $R^1$ | Wasserstoff, Methyl oder Halogen bedeutet, |
| $R^2$ | Wasserstoff, Methyl, Ethyl, Halogen, Trifluormethyl, ($C_1$-$C_4$)Alkoxy, ($C_1$-$C_4$)Halogenalkoxy, Methoxymethyl, Dimethylamino oder Diethylamino bedeutet, |
| $R^3$ | Wasserstoff, ($C_1$-$C_4$)Alkyl, ($C_1$-$C_4$)Alkoxy, ($C_1$-$C_4$)Halogenalkoxy oder, falls $R^3$ nicht Ethyl ist, auch Halogen bedeutet, |
| $R^4$ | Wasserstoff bedeutet, |

$R^5$ Wasserstoff, $(C_1-C_4)$Alkyl oder Cyclopropyl bedeutet,
Q die Bedeutung $Q^1$ hat und
$Q^1$ $(C_1-C_{15})$Alkyl bedeutet, gegebenenfalls substituiert mit einem, zwei oder drei Halogenatomen, einer $(C_1-C_{15})$Alkoxygruppe, einer $(C_4-C_8)$Cycloalkylalkoxygruppe, einer Dioxolanylgruppe, einer $(C_1-C_4)$Alkoxy-$(C_1-C_4)$-alkoxygruppe, einer Hydroxygruppe, einer Methoxycarbonylgruppe, einer $(C_3-C_6)$-Cycloalkylgruppe, einer 2-[2-($(C_1-C_4)$Alkoxy)ethoxy]ethoxygruppe, einer Pyranyloxygruppe, einer Imidazol-1-ylgruppe, einer Triazol-1-ylgruppe, einer Pyrazol-1-ylgruppe oder einer Gruppe -A-B-$R^8$, worin A Sauerstoff, Schwefel oder Imino, B Carbonyl, Thiocarbonyl oder Sulfonyl bedeutet und $R^8$ $(C_1-C_4)$Alkyl, $(C_3-C_6)$Cycloalkyl, $(C_1-C_4)$Halogenalkyl, $(C_1-C_4)$Alkoxy, eine substituierte oder unsubstituierte Phenoxygruppe, eine substituierte oder unsubstituierte Phenylgruppe, eine substituierte oder unsubstituierte Anilinogruppe, eine 2,6-Dimethylmorpholin-4-yl-, 4-Methylpiperazin-1-yl-, Imidazol-1-yl-, Triazol-1-yl-, Pyrazol-1-ylgruppe oder eine Mono- oder Di-$(C_1-C_4)$alkylaminogruppe bedeutet, sowie ihre Salze und ihre Stereoisomeren.

Stärker bevorzugt sind weiterhin Verbindungen der Formel I, worin
$R^1$ Wasserstoff, Methyl oder Halogen bedeutet,
$R^2$ Wasserstoff, Methyl, Ethyl, Halogen, Trifluormethyl, $(C_1-C_4)$Alkoxy, $(C_1-C_4)$-Halogenalkoxy, Methoxymethyl, Dimethylamino oder Diethylamino bedeutet,
$R^3$ Wasserstoff, $(C_1-C_4)$Alkyl, $(C_1-C_4)$Alkoxy, $(C_1-C_4)$Halogenalkoxy oder Halogen bedeutet, mit der Maßgabe, daß wenn Q keine der später aufgeführten Gruppen der allgemeinen Formeln II', II'', II''' oder II'''' ist und $R^3$ eine $(C_1-C_4)$Alkylgruppe oder ein Halogenatom bedeutet, $R^2$ nicht gleichzeitig Methyl, Ethyl, Halogen oder Methoxymethyl bedeutet,
$R^4$ Wasserstoff bedeutet,
$R^5$ Wasserstoff, $(C_1-C_4)$Alkyl oder Cyclopropyl bedeutet,
Q die Bedeutung $Q^2$ hat und
$Q^2$ eine Gruppe der allgemeinen Formeln II, II', II'', II''' oder II'''' bedeutet, worin
D eine $(C_1-C_6)$Alkylengruppe bedeutet,
E eine direkte Bindung, Sauerstoff oder Methylenoxy bedeutet,
$R^9$ und $R^{10}$ gleich oder verschieden sind und jeweils Wasserstoff, Halogen, $(C_1-C_4)$Alkyl, $(C_3-C_6)$-Cycloalkyl, $(C_1-C_4)$Halogenalkyl, $C_3$- oder $C_4$-Alkenyl, $(C_1-C_4)$Alkoxy, $(C_1-C_4)$Alkylthio oder Nitro bedeuten,
$R^{11}$ die für $R^9$ und $R^{10}$ angegebenen Bedeutungen hat, sowie für den Fall, daß E Sauerstoff bedeutet, zusätzlich eine Gruppe der Formel X-Y-$(GO)_n$-$R^{12}$ bedeutet, worin X eine $(C_1-C_8)$Alkylengruppe oder eine $(C_1-C_8)$Alkylengruppe mit einem $(C_1-C_4)$Alkoxysubsituenten bedeutet,
Y Sauerstoff, Schwefel oder eine Iminogruppe bedeutet,
G eine $(C_1-C_8)$Alkylengruppe oder eine $(C_1-C_4)$Alkylenoxy-$(C_1-C_4)$alkylengruppe bedeutet,
n Null oder 1 ist,
$R^{12}$ $(C_1-C_4)$Alkyl, $C_3$- oder $C_4$-Alkenyl, $(C_4-C_6)$Alkadienyl, $C_3$- oder $C_4$-Alkinyl, Phenyl-$(C_1-C_3)$alkyl oder eine Gruppe der Formel $CH_2$-W bedeutet, worin
W eine Gruppe der Formel CH = N-$OR^{13}$ bedeutet, worin
$R^{13}$ Wasserstoff, $(C_1-C_4)$Alkyl, $C_3$- oder $C_4$-Alkenyl oder Phenyl-$(C_1-C_3)$alkyl bedeutet, und
W weiterhin eine Morpholinomethylgruppe oder eine heterocyclische Gruppe mit 5 bis 8 Ringatomen bedeutet, von denen 2 oder 3 Sauerstoff- oder Schwefelatome sind, und der besagte Heterocyclus gesättigt ist oder eine Doppelbindung im Ring besitzt und der besagte Heterocyclus unsubstituiert ist oder einen oder zwei Alkyl-, $(C_1-C_4)$Halogenalkyl- oder Phenylsubstituenten trägt, sowie ihre Salze und ihre Stereoisomeren.

Stärker bevorzugt sind weiterhin Verbindungen der Formel I, worin
$R^1$ Wasserstoff, Methyl oder Halogen bedeutet
$R^2$ Wasserstoff, Methyl, Ethyl, Halogen, Trifluormethyl, $(C_1-C_4)$Alkoxy, $(C_1-C_4)$-Halogenalkoxy, Methoxymethyl, Dimethylamino oder Diethylamino bedeutet,
$R^3$ Wasserstoff, $(C_1-C_4)$Alkyl, $(C_1-C_4)$Alkoxy, $(C_1-C_4)$Halogenalkoxy oder Halogen bedeutet, mit der Maßgabe, daß wenn $R^3$ $(C_1-C_4)$Alkyl oder Halogen bedeutet, $R^2$ nicht gleichzeitig Halogen, Methyl, Ethyl oder Methoxymethyl bedeutet,
$R^4$ Wasserstoff bedeutet,
$R^5$ Wasserstoff, $(C_1-C_4)$Alkyl oder Cyclopropyl bedeutet,
Q die Bedeutung $Q^3$ hat und

| | |
|---|---|
| $Q^3$ | eine Gruppe der allgemeinen Formel III bedeutet, worin |
| $R^9$ und $R^{10}$ | die genannten Bedeutungen haben, |
| U | eine direkte Bindung, Sauerstoff, Schwefel, $(C_1-C_3)$Alkylen oder $(C_1-C_3)$Alkylenoxy bedeutet, und |
| $R^{14}$ | Phenyl oder einen Hetercyclus bedeutet, wobei jeder der beiden vorgenannten Reste unsubstituiert oder mit einem oder zwei Substituenten versehen sein kann und diese Substituenten gleich oder verschieden sind und jeweils Halogen, $(C_1-C_4)$Alkyl, $(C_1-C_4)$-Halogenalkyl, Nitro, $(C_1-C_4)$Alkoxy, $(C_1-C_4)$Alkylthio, $(C_1-C_4)$Alkoxy-$(C_1-C_4)$alkyl, Phenyl, Phenoxy, Halogenphenoxy oder $(C_1-C_4)$Alkylphenoxy bedeuten, |
| $R^{14}$ | weiterhin, für den Fall, daß U Sauerstoff ist, $(C_5-C_{10})$Alkyl, Allyl, Geranyl, Farnesyl, $(C_1-C_4)$Halogenalkyl, $(C_3-C_6)$Cycloalkylmethyl, eine Ethylgruppe, die in 2-Stellung durch $(C_1-C_4)$Alkoxy, $(C_1-C_4)$Alkoxy-$(C_1-C_4)$alkyl, $(C_1-C_4)$-Alkylthio, einer $(C_1-C_4)$Alkylsulfonyl- oder eine Phenoxygruppe, die gegebenenfalls mit einer oder zwei $(C_1-C_4)$Alkylgruppen substituiert sein kann, Glycidyl, Acetonyl, Halogenphenoxymethyldioxolanyl, 2,2-Diethoxyethyl, 1-Ethoxycarbonylmethyl, Trimethylsilylmethyl, 1-Pyridylethyl oder eine $(C_1-C_4)$-Alkylgruppe, die mit einer $(C_1-C_4)$Alkoxyimino- oder einer Benzyloxyiminogruppe substituiert ist, substituiert ist, bedeutet, |
| $R^{14}$ | weiterhin, für den Fall, daß U Sauerstoff ist, eine Gruppe der allgemeinen Formel IV bedeutet, worin |
| X' | Stickstoff oder eine Gruppe $CR^{15}$ bedeutet, worin |
| $R^{15}$ | Wasserstoff, Halogen, $(C_1-C_4)$Alkyl, $(C_1-C_4)$Halogenalkyl, $(C_1-C_4)$Alkanoyl, Nitro, Cyano oder 1,3-Dioxolan-2-yl bedeutet, sowie ihre Salze und ihre Stereoisomeren. |

Noch stärker bevorzugt sind Verbindungen der Formel I, worin

| | |
|---|---|
| $R^1$ | Wasserstoff, Methyl oder Halogen bedeutet, |
| $R^2$ | Wasserstoff, Halogen, Methoxy, Ethoxy, Trifluormethyl, Methoxymethyl, Dimethylamino oder Diethylamino bedeutet, |
| $R^3$ | Wasserstoff, $(C_1-C_3)$Alkyl, Methoxy, Ethoxy, $(C_1-C_2)$-Halogenalkoxy oder Halogen bedeutet, mit der Maßgabe, daß wenn Q keine der später aufgeführten Gruppen der Bedeutung $Q^1$ oder der allgemeinen Formeln II', II'', II''' oder II'''' ist und $R^3$ eine $(C_1-C_3)$Alkylgruppe oder ein Halogenatom bedeutet, $R^2$ nicht gleichzeitig Halogen oder Methoxymethyl bedeutet, |
| $R^4$ | Wasserstoff bedeutet, |
| $R^5$ | Wasserstoff, $(C_1-C_4)$Alkyl oder Cyclopropyl bedeutet, |
| Q | die Bedeutung $Q^1$ hat und |
| $Q^1$ | $(C_1-C_{15})$Alkyl bedeutet, gegebenenfalls substituiert mit einem, zwei oder drei Halogenatomen, einer $(C_1-C_{15})$Alkoxygruppe, einer $(C_4-C_8)$Cycloalkylalkoxygruppe, einer Dioxolanylgruppe, einer $(C_1-C_4)$Alkoxy-$(C_1-C_4)$alkoxygruppe, einer Hydroxygruppe, einer Methoxycarbonylgruppe, einer $(C_3-C_6)$-Cycloalkylgruppe, einer 2-[2-($(C_1-C_4)$Alkoxy)ethoxy]ethoxygruppe, einer Pyranyloxygruppe, einer Imidazol-1-ylgruppe, einer Triazol-1-ylgruppe, einer Pyrazol-1-ylgruppe oder einer Gruppe $-A-B-R^8$, worin A Sauerstoff, Schwefel oder Imino, B Carbonyl, Thiocarbonyl oder Sulfonyl bedeutet und $R^8$ $(C_1-C_4)$Alkyl, $(C_3-C_6)$Cycloalkyl, $(C_1-C_4)$Halogenalkyl, $(C_1-C_4)$Alkoxy, eine substituierte oder unsubstituierte Phenoxygruppe, eine substituierte oder unsubstituierte Phenylgruppe, eine substituierte oder unsubstituierte Anilinogruppe, eine 2,6-Dimethylmorpholin-4-yl-, 4-Methylpiperazin-1-yl-, Imidazol-1-yl-, Triazol-1-yl-, Pyrazol-1-ylgruppe oder eine Mono- oder Di-$(C_1-C_4)$alkylaminogruppe bedeutet, oder |
| Q | die Bedeutung $Q^2$ hat und |
| $Q^2$ | eine Gruppe der allgemeinen Formeln II, II', II'', II''' oder II'''' bedeutet, worin |
| D | eine $(C_1-C_6)$Alkylengruppe bedeutet, |
| E | eine direkte Bindung, Sauerstoff oder Methylenoxy bedeutet, |
| $R^9$ und $R^{10}$ | gleich oder verschieden sind und jeweils Wasserstoff, Halogen, $(C_1-C_4)$Alkyl, $(C_3-C_6)$-Cycloalkyl, $(C_1-C_4)$Halogenalkyl, $C_3$- oder $C_4$-Alkenyl, $(C_1-C_4)$Alkoxy,$(C_1-C_4)$Alkylthio oder Nitro bedeuten, |
| $R^{11}$ | die für $R^9$ und $R^{10}$ angegebenen Bedeutungen hat, sowie für den Fall, daß E Sauerstoff bedeutet zusätzlich eine Gruppe der Formel $X-Y-(GO)_n-R^{12}$ bedeutet, worin |
| X | eine $(C_1-C_8)$Alkylengruppe oder eine $(C_1-C_8)$Alkylengruppe mit einem $(C_1-C_4)$- |

Alkoxysubsituenten bedeutet,

Y Sauerstoff, Schwefel oder eine Iminogruppe bedeutet,

G eine (($C_1$-$C_8$)Alkylengruppe oder eine ($C_1$-$C_4$)Alkylenoxy-($C_1$-$C_4$)alkylengruppe bedeutet,

n Null oder 1 ist,

$R^{12}$ ($C_1$-$C_4$)Alkyl, $C_3$- oder $C_4$-Alkenyl, ($C_4$-$C_6$)Alkadienyl, $C_3$- oder $C_4$-Alkinyl, Phenyl-($C_1$-$C_3$)alkyl oder eine Gruppe der Formel $CH_2$-W bedeutet, worin

W eine Gruppe der Formel $CH=N-OR^{13}$ bedeutet, worin

$R^{13}$ Wasserstoff, ($C_1$-$C_4$)Alkyl, $C_3$- oder $C_4$-Alkenyl oder Phenyl-($C_1$-$C_3$)alkyl bedeutet,

W weiterhin eine Morpholinomethylgruppe oder eine heterocyclische Gruppe mit 5 bis 8 Ringatomen bedeutet, von denen 2 oder 3 Sauerstoff oder Schwefelatome sind, und der besagte Heterocyclus gesättigt ist oder eine Doppelbindung im Ring besitzt und der besagte Heterocyclus unsubstituiert ist oder einen oder zwei Alkyl-, ($C_1$-$C_4$)Halogenalkyl- oder Phenylsubstituenten trägt, oder

Q die Bedeutung $Q^3$ hat und

$Q^3$ eine Gruppe der allgemeinen Formel III bedeutet, worin $R^9$ und $R^{10}$ die oben angegebenen Bedeutungen haben,

U eine direkte Bindung, Sauerstoff, Schwefel, ($C_1$-$C_3$)Alkylen oder ($C_1$-$C_3$)Alkylenoxy bedeutet, und

$R^{14}$ Phenyl oder einen Heterocyclus bedeutet, wobei jeder der beiden vorgenannten Reste unsubstituiert oder mit einem oder zwei Substituenten versehen sein kann und diese Substituenten gleich oder verschieden sind und jeweils Halogen, ($C_1$-$C_4$)Alkyl, ($C_1$-$C_4$)-Halogenalkyl, Nitro

($C_1$-$C_4$)Alkoxy, ($C_1$-$C_4$)Alkylthio, ($C_1$-$C_4$)Alkoxy-($C_1$-$C_4$)alkyl, Phenyl, Phenoxy, Halogenphenoxy oder ($C_1$-$C_4$)Alkylphenoxy bedeuten,

$R^{14}$ weiterhin, für den Fall, daß U Sauerstoff ist, ($C_5$-$C_{10}$)Alkyl, Allyl, Geranyl, Farnesyl, ($C_1$-$C_4$)Halogenalkyl, ($C_3$-$C_6$)Cycloalkylmethyl, eine Ethylgruppe, die in 2-Stellung durch ($C_1$-$C_4$)Alkoxy, ($C_1$-$C_4$)Alkoxy-($C_1$-$C_4$)alkyl, ($C_1$-$C_4$)Alkylthio, ($C_1$-$C_4$)Alkylsulfonyl oder eine Phenoxygruppe, die gegebenenfalls mit einer oder zwei ($C_1$-$C_4$)Alkylgruppen substituiert sein kann, Glycidyl, Acetonyl, Halogenphenoxymethyldioxolanyl, 2,2-Diethoxyethyl, 1-Ethoxycarbonylmethyl, Trimethylsilylmethyl, 1-Pyridylethyl oder eine ($C_1$-$C_4$)Alkylgruppe, die mit einer ($C_1$-$C_4$)Alkoxyimino- oder einer Benzyloxyiminogruppe substituiert ist, substituiert ist, bedeutet,

$R^{14}$ weiterhin, für den Fall, daß U Sauerstoff ist, eine Gruppe der allgemeinen Formel IV bedeutet, worin

X' Stickstoff oder eine Gruppe $CR^{15}$ bedeutet, worin

$R^{15}$ Wasserstoff, Halogen, ($C_1$-$C_4$)Alkyl, ($C_1$-$C_4$)Halogenalkyl, ($C_1$-$C_4$)Alkanoyl, Nitro, Cyano oder 1,3-Dioxolan-2-yl bedeutet, sowie ihre Salze und ihre Stereoisomeren.

Noch stärker bevorzugt sind weiterhin Verbindungen der Formel I, worin

$R^1$ Wasserstoff, Methyl oder Halogen bedeutet,

$R^2$ Wasserstoff, Halogen, Methoxy, Ethoxy, Trifluormethyl, Methoxymethyl, Dimethylamino oder Diethylamino bedeutet,

$R^3$ Wasserstoff, ($C_1$-$C_3$)Alkyl, Methoxy, Ethoxy, ($C_1$-$C_2$)Halogenalkoxy oder Halogen bedeutet,

$R^4$ Wasserstoff bedeutet,

$R^5$ Wasserstoff, ($C_1$-$C_4$)Alkyl oder Cyclopropyl bedeutet,

Q die Bedeutung $Q^1$ hat und

$Q^1$ ($C_1$-$C_{15}$)Alkyl bedeutet, gegebenenfalls substituiert mit einem, zwei oder drei Halogenatomen, einer ($C_1$-$C_{15}$)Alkoxygruppe, einer ($C_4$-$C_8$)Cycloalkylalkoxygruppe, einer Dioxolanylgruppe, einer ($C_1$-$C_4$)Alkoxy-($C_1$-$C_4$)alkoxygruppe, einer Hydroxygruppe, einer Methoxycarbonylgruppe, einer ($C_3$-$C_6$)Cycloalkylgruppe, einer 2-[2-(($C_1$-$C_4$)Alkoxy)ethoxy]ethoxygruppe, einer Pyranyloxygruppe, einer Imidazol-1-ylgruppe, einer Triazol-1-ylgruppe, einer Pyrazol-1-ylgruppe oder einer Gruppe-A-B-$R^8$, worin A Sauerstoff, Schwefel oder Imino, B Carbonyl, Thiocarbonyl oder Sulfonyl bedeutet und $R^8$ ($C_1$-$C_4$)Alkyl, ($C_3$-$C_6$)Cycloalkyl, ($C_1$-$C_4$)Halogenalkyl, ($C_1$-$C_4$)Alkoxy, eine substituierte oder unsubstituierte Phenoxygruppe, eine substituierte oder unsubstituierte Phenylgruppe, eine substituierte oder unsubstituierte Anilinogruppe, eine 2,6-Dimethylmorpholin-4-yl-, 4-Methylpiperazin-1-yl-, Imidazol-1-yl-, Triazol-1-yl-, Pyrazol-1-ylgruppe oder Mono- oder Di-($C_1$-$C_4$)alkylaminogruppe bedeutet, sowie ihre Salze und ihre Stereoisomeren.

Noch stärker bevorzugt sind weiterhin Verbindungen der Formel I, worin

$R^1$ Wasserstoff, Methyl oder Halogen bedeutet,
$R^2$ Wasserstoff, Halogen, Methoxy, Ethoxy, Trifluormethyl, Methoxymethyl, Dimethylamino oder Diethylamino bedeutet,
$R^3$ Wasserstoff, $(C_1\text{-}C_3)$Alkyl, Methoxy, Ethoxy, $(C_1\text{-}C_2)$Halogenalkoxy oder Halogen bedeutet, mit der Maßgabe daß wenn Q keine der später aufgeführten Gruppen der allgemeinen Formeln II', II'', II''' oder II'''' ist und $R^3$ eine $(C_1\text{-}C_3)$Alkylgruppe oder ein Halogenatom bedeutet, $R^2$ nicht gleichzeitig Halogen oder Methoxymethyl bedeutet,
$R^4$ Wasserstoff bedeutet,
$R^5$ Wasserstoff, $(C_1\text{-}C_4)$Alkyl oder Cyclopropyl bedeutet,
Q die Bedeutung $Q^2$ hat und
$Q^2$ eine Gruppe der allgemeinen Formeln II, II', II'', II''' oder II'''' bedeutet, worin
D eine $(C_1\text{-}C_6)$Alkylengruppe bedeutet,
E eine direkte Bindung, Sauerstoff oder Methylenoxy bedeutet,
$R^9$ und $R^{10}$ gleich oder verschieden sind und jeweils Wasserstoff, Halogen, $(C_1\text{-}C_4)$Alkyl, $(C_3\text{-}C_6)$-Cycloalkyl, $(C_1\text{-}C_4)$Halogenalkyl, $C_3$- oder $C_4$-Alkenyl, $(C_1\text{-}C_4)$Alkoxy, $(C_1\text{-}C_4)$Alkylthio oder Nitro bedeuten,
$R^{11}$ die für $R^9$ und $R^{10}$ angegebenen Bedeutungen hat, sowie für den Fall, daß E Sauerstoff bedeutet, zusätzlich eine Gruppe der Formel $X\text{-}Y\text{-}(GO)_n\text{-}R^{12}$ bedeutet, worin
X eine $(C_1\text{-}C_8)$Alkylengruppe oder eine $(C_1\text{-}C_8)$Alkylengruppe mit einem $(C_1\text{-}C_4)$-Alkoxysubstituenten bedeutet,
Y Sauerstoff, Schwefel oder eine Iminogruppe bedeutet,
G eine $(C_1\text{-}C_8)$Alkylengruppe oder eine $(C_1\text{-}C_8)$Alkylenoxy-$(C_1\text{-}C_4)$alkylengruppe bedeutet,
n Null oder 1 ist,
$R^{12}$ $(C_1\text{-}C_4)$Alkyl, $C_3$- oder $C_4$-Alkenyl, $(C_4\text{-}C_6)$Alkadienyl, $C_3$- oder $C_4$-Alkinyl, Phenyl-$(C_1\text{-}C_3)$alkyl oder eine Gruppe der Formel $CH_2$-W bedeutet, worin
W eine Gruppe der Formel $CH=N\text{-}OR^{13}$ bedeutet, worin
$R^{13}$ Wasserstoff, $(C_1\text{-}C_4)$Alkyl, $C_3$- oder $C_4$-Alkenyl oder Phenyl-$(C_1\text{-}C_3)$alkyl bedeutet, und
W weiterhin eine Morpholinomethylgruppe oder eine heterocyclische Gruppe mit 5 bis 8 Ringatomen bedeutet, von denen 2 oder 3 Sauerstoff- oder Schwefelatome sind, und der besagte Heterocyclus gesättigt ist oder eine Doppelbindung im Ring besitzt und der besagte Heterocyclus unsubstituiert ist oder einen oder zwei Alkyl-, $(C_1\text{-}C_4)$-Halogenalkyl- oder Phenylsubstituenten trägt, sowie ihre Salze und ihre Stereoisomeren.

Noch stärker bevorzugt sind weiterhin Verbindungen der Formel I, worin
$R^1$ Wasserstoff, Methyl oder Halogen bedeutet,
$R^2$ Wasserstoff, Halogen, Methoxy, Ethoxy, Trifluormethyl, Methoxymethyl, Dimethylamino oder Diethylamino bedeutet,
$R^3$ Wasserstoff, $(C_1\text{-}C_3)$Alkyl, Methoxy, Ethoxy, $(C_1\text{-}C_2)$Halogenalkoxy oder Halogen bedeutet, mit der Maßgabe, daß wenn $R^3$ $(C_1\text{-}C_3)$Alkyl oder Halogen bedeutet, $R^2$ nicht gleichzeitig Halogen oder Methoxymethyl bedeutet,
$R^4$ Wasserstoff bedeutet,
$R^5$ Wasserstoff, $(C_1\text{-}C_4)$Alkyl oder Cyclopropyl bedeutet,
Q die Bedeutung $Q^3$ hat und
$Q^3$ eine Gruppe der allgemeinen Formel III bedeutet, worin $R^9$ und $R^{10}$ die oben angegebenen Bedeutungen haben,
U eine direkte Bindung, Sauerstoff, Schwefel, $(C_1\text{-}C_3)$Alkylen oder $(C_1\text{-}C_3)$Alkylenoxy bedeutet, und
$R^{14}$ Phenyl oder einen Heterocyclus bedeutet, wobei jeder der beiden vorgenannten Reste unsubstituiert oder mit einem oder zwei Substituenten versehen sein kann und diese Substituenten gleich oder verschieden sind und jeweils Halogen, $(C_1\text{-}C_4)$Alkyl, $(C_1\text{-}C_4)$Halogenalkyl, $(C_1\text{-}C_4)$Alkoxy, Nitro $(C_1\text{-}C_4)$Alkylthio, $(C_1\text{-}C_4)$Alkoxy-$(C_1\text{-}C_4)$alkyl, Phenyl, Phenoxy, Halogenphenoxy oder $(C_1\text{-}C_4)$Alkylphenoxy bedeuten,
$R^{14}$ weiterhin, für den Fall, daß U Sauerstoff ist, $(C_5\text{-}C_{10})$Alkyl, Allyl, Geranyl, Farnesyl, $(C_1\text{-}C_4)$-Halogenalkyl, $(C_3\text{-}C_6)$Cycloalkylmethyl, eine Ethylgruppe, die in 2-Stellung durch $(C_1\text{-}C_4)$Alkoxy, einer $(C_1\text{-}C_4)$Alkoxy-$(C_1\text{-}C_4)$Alkyl-, $(C_1\text{-}C_4)$Alkylthio-, $(C_1\text{-}C_4)$Alkylsulfonyl- oder eine Phenoxygruppe, die gegebenenfalls mit einer oder zwei $(C_1\text{-}C_4)$Alkylgruppen substituiert sein kann, Glycidyl, Acetonyl, Halogenphenoxymethyldioxolanyl, 2,2-Diethoxyethyl, 1-Ethoxycabonylmethyl, Trimethylsilylmethyl, 1-Pyridylethyl oder eine $(C_1\text{-}C_4)$Alkylgruppe, die mit einer $(C_1\text{-}C_4)$-Alkoxyimino- oder eine Benzyloxyiminogruppe substituiert ist, substituiert ist, bedeutet,
$R^{14}$ weiterhin, für den Fall, daß U Sauerstoff ist, eine Gruppe der allgemeinen Formel IV bedeutet,

worin
X' Stickstoff oder eine Gruppe $CR^{15}$ bedeutet, worin
$R^{15}$ Wasserstoff, Halogen, $(C_1-C_4)$Alkyl, $(C_1-C_4)$Halogenalkyl, $(C_1-C_4)$Alkanoyl, Nitro, Cyano oder 1,3-Dioxolan-2-yl bedeutet, sowie ihre Salze und ihre Stereoisomeren.

Am stärksten bevorzugt sind Verbindungen der allgemeinen Formel I, worin

$R^1$ Wasserstoff bedeutet,
$R^2$ Methoxy oder Methoxymethyl bedeutet,
$R^3$ Methoxy bedeutet,
$R^4$ Wasserstoff bedeutet,
$R^5$ Wasserstoff, Methyl, Ethyl oder Cyclopropyl bedeutet,
Q die Bedeutung $Q^1$ hat und
$Q^1$ $(C_1-C_{15})$Alkyl bedeutet, gegebenenfalls substituiert mit einem, zwei oder drei Halogenatomen, einer $(C_1-C_{15})$Alkoxygruppe, einer $(C_4-C_8)$Cycloalkylalkoxygruppe, einer Dioxolanylgruppe, einer $(C_1-C_4)$Alkoxy-$(C_1-C_4)$-alkoxygruppe, einer Hydroxygruppe, einer Methoxycarbonylgruppe, einer $(C_3-C_6)$-Cycloalkylgruppe, einer 2[2-($(C_1-C_4)$Alkoxy)ethoxy]ethoxygruppe, einer Pyranyloxygruppe, einer Imidazol-1-ylgruppe, einer Triazol-1-ylgruppe, einer Pyrazol-1-ylgruppe oder einer Gruppe -A-B-$R^8$, worin A Sauerstoff, Schwefel oder Imino, B Carbonyl, Thiocarbonyl oder Sulfonyl bedeutet und $R^8$ $(C_1-C_4)$Alkyl, $(C_3-C_6)$Cycloalkyl, $(C_1-C_4)$Halogenalkyl, $(C_1-C_4)$Alkoxy, eine substituierte oder unsubstituierte Phenoxygruppe, eine substituierte oder unsubstituierte Phenylgruppe, eine substituierte oder unsubstituierte Anilinogruppe, eine 2,6-Dimethylmorpholin-4-yl-, 4-Methylpiperazin-1-yl-, Imidazol-1-yl-, Triazol-1-yl-, Pyrazol-1-ylgruppe oder eine Mono- oder Di-$(C_1-C_4)$alkylaminogruppe bedeutet oder
Q die Bedeutung $Q^2$ hat und
$Q^2$ eine Gruppe der allgemeinen Formeln II, II', II'', II''' oder II'''' bedeutet, worin
D eine $(C_1-C_6)$Alkylengruppe bedeutet,
E eine direkte Bindung, Sauerstoff oder Methylenoxy bedeutet,
$R^9$ und $R^{10}$ gleich oder verschieden sind und jeweils Wasserstoff, Halogen, $(C_1-C_4)$Alkyl, $(C_3-C_6)$-Cycloalkyl, $(C_1-C_4)$Halogenalkyl, $C_3$- oder $C_4$-Alkenyl, $(C_1-C_4)$Alkoxy, $(C_1-C_4)$Alkylthio oder Nitro bedeuten,
$R^{11}$ die für $R^9$ und $R^{10}$ angegebene Bedeutung hat, sowie für den Fall, daß E Sauerstoff bedeutet, zusätzlich eine Gruppe der Formel X-Y-$(GO)_n$-$R^{12}$ bedeutet, worin
X eine $(C_1-C_8)$Alkylengruppe oder eine $(C_1-C_8)$Alkylengruppe mit einem $(C_1-C_4)$-Alkoxysubstituenten bedeutet,
Y Sauerstoff, Schwefel oder eine Iminogruppe bedeutet,
G eine $(C_1-C_8)$Alkylengruppe oder eine $(C_1-C_4)$Alkylenoxy-$(C_1-C_4)$alkylengruppe bedeutet,
n Null oder 1 ist,
$R^{12}$ $(C_1-C_4)$Alkyl, $C_3$- oder $C_4$-Alkenyl, $(C_4-C_6)$Alkadienyl, $C_3$- oder $C_4$-Alkinyl, Phenyl-$(C_1-C_3)$alkyl oder eine Gruppe der Formel $CH_2$-W bedeutet, worin
W eine Gruppe der Formel $CH=N-OR^{13}$ bedeutet, worin
$R^{13}$ Wasserstoff, $(C_1-C_4)$Alkyl, $C_3$- oder $C_4$-Alkenyl oder Phenyl-$(C_1-C_3)$alkyl bedeutet,
W weiterhin eine Morpholinomethylgruppe oder eine heterocyclische Gruppe mit 5 bis 8 Ringatomen bedeutet, von denen 2 oder 3 Sauerstoff- oder Schwefelatome sind, und der besagte Heterocyclus gesättigt ist oder eine Doppelbindung im Ring besitzt und der besagte Heterocyclus unsubstituiert ist oder einen oder zwei Alkyl-, $(C_1-C_4)$-Halogenalkyl- oder Phenylsubstituenten trägt, oder
Q die Bedeutung $Q^3$ hat und
$Q^3$ eine Gruppe der allgemeinen Formel III bedeutet, worin $R^9$ und $R^{10}$ die oben angegebenen Bedeutungen haben,
U eine direkte Bindung, Sauerstoff, Schwefel, $(C_1-C_3)$Alkylen oder $(C_1-C_3)$Alkylenoxy bedeutet, und
$R^{14}$ Phenyl oder einen Heterocyclus bedeutet, wobei jeder der beiden vorgenannten Reste unsubstituiert oder mit einem oder zwei Substituenten versehen sein kann und diese Substituenten gleich oder verschieden sind und jeweils Halogen, $(C_1-C_4)$Alkyl, $(C_1-C_4)$-Halogenalkyl, $(C_1-C_4)$Alkoxy, Nitro $(C_1-C_4)$Alkylthio, $(C_1-C_4)$Alkoxy-$(C_1-C_4)$alkyl, Phenyl, Phenoxy, Halogenphenoxy oder $(C_1-C_4)$Alkylphenoxy bedeuten,
$R^{14}$ weiterhin, für den Fall, daß U Sauerstoff ist, $(C_5-C_{10})$Alkyl, Allyl, Geranyl, Farnesyl, $(C_1$-

$C_4$)Halogenalkyl, ($C_3$-$C_6$)Cycloalkylmethyl, eine Ethylgruppe, die in 2-Stellung durch ($C_1$-$C_4$)Alkylthio, ($C_1$-$C_4$)Alkylsulfonyl oder eine Phenoxygruppe, die gegebenenfalls mit einer oder zwei ($C_1$-$C_4$)Alkylgruppen substituiert sein kann, Glycidyl, Acetonyl, Halogenphenoxymethyldioxolanyl, 2,2-Diethoxyethyl, 1-Ethoxycarbonylmethyl, Trimethylsilylmethyl, 1-Pyridylethyl oder eine ($C_1$-$C_4$)Alkylgruppe, die mit einer ($C_1$-$C_4$)Alkoxyimino- oder eine Benzyloxyiminogruppe substituiert ist, substituiert ist, bedeutet,

$R^{14}$ weiterhin, für den Fall, daß U Sauerstoff ist, eine Gruppe der allgemeinen Formel IV bedeutet, worin

X' Stickstoff oder eine Gruppe $CR^{15}$ bedeutet, worin

$R^{15}$ Wasserstoff, Halogen, ($C_1$-$C_4$)Alkyl, ($C_1$-$C_4$)Alkyl, ($C_1$-$C_4$)Halogenalkyl, ($C_1$-$C_4$)-Alkanoyl, Nitro, Cyano oder 1,3-Dioxolan-2-yl bedeutet, sowie ihre Salze und ihre Stereoisomeren.

Am stärksten bevorzugt sind weiterhin Verbindungen der allgemeinen Formel I, worin

$R^1$ Wasserstoff bedeutet,

$R^2$ Methoxymethyl bedeutet,

$R^3$ Methoxy bedeutet,

$R^4$ Wasserstoff bedeutet,

$R^5$ Wasserstoff, Methyl, Ethyl oder Cyclopropyl bedeutet,

Q die Bedeutung $Q^1$ hat und

$Q^1$ ($C_1$-$C_{15}$)Alkyl bedeutet, gegebenenfalls substituiert mit einem, zwei oder drei Halogenatomen, einer ($C_1$-$C_{15}$)Alkoxygruppe, einer ($C_4$-$C_8$)Cycloalkylalkoxygruppe, einer Dioxolanylgruppe, einer ($C_1$-$C_4$)Alkoxy-($C_1$-$C_4$)-alkoxygruppe, einer Hydroxygruppe, einer Methoxycarbonylgruppe, einer ($C_3$-$C_6$)-Cycloalkylgruppe, einer 2-[2-(($C_1$-$C_4$)Alkoxy)ethoxy]ethoxygruppe, einer Pyranyloxygruppe, einer Imidazol-1-ylgruppe, einer Triazol-1-ylgruppe, einer Pyrazol-1-ylgruppe oder einer Gruppe -A-B-$R^8$, worin A Sauerstoff, Schwefel oder Imino, B Carbonyl, Thiocarbonyl oder Sulfonyl bedeutet und $R^8$ ($C_1$-$C_4$)Alkyl, ($C_3$-$C_6$)Cycloalkyl, ($C_1$-$C_4$)Halogenalkyl, ($C_1$-$C_4$)Alkoxy, eine substituierte oder unsubstituierte Phenoxygruppe, eine substituierte oder unsubstituierte Phenylgruppe, eine substituierte oder unsubstituierte Anilinogruppe, eine 2,6-Dimethylmorpholin-4-yl-, 4-Methylpiperazin-1-yl-, Imidazol-1-yl-, Triazol-1-yl-, Pyrazol-1-ylgruppe oder eine Mono- oder Di-($C_1$-$C_4$)-alkylaminogruppe bedeutet, vorzugsweise $Q^1$ ($C_3$-$C_{13}$)Alkyl, oder

Q die Bedeutung $Q^2$ hat und

$Q^2$ eine Gruppe der allgemeinen Formeln II, II', II'', II''' oder II'''' bedeutet, worin

D eine ($C_1$-$C_2$)Alkylengruppe bedeutet,

E eine direkte Bindung oder Sauerstoff bedeutet,

$R^9$ und $R^{10}$ gleich oder verschieden sind und jeweils Wasserstoff, Halogen, ($C_1$-$C_4$)Alkyl, ($C_3$-$C_6$)-Cycloalkyl, ($C_1$-$C_4$)Halogenalkyl, oder ($C_1$-$C_4$)Alkoxy bedeuten,

$R^{11}$ die für $R^9$ und $R^{10}$ angegebenen Bedeutungen hat, sowie für den Fall, daß E Sauerstoff bedeutet, zusätzlich eine Gruppe der Formel X-Y-$(GO)_n$-$R^{12}$ bedeutet, worin

X eine ($C_1$-$C_2$)Alkylengruppe bedeutet,

Y Sauerstoff bedeutet,

G eine Ethylgruppe bedeutet,

n Null oder 1 ist, vorzugsweise Null und

$R^{12}$ ($C_1$-$C_4$)Alkyl bedeutet, oder

Q die Bedeutung $Q^3$ hat und

$Q^3$ eine Gruppe der allgemeinen Formel III worin $R^9$ und $R^{10}$ die oben angegebenen Bedeutungen haben, vorzugsweise H, Methyl oder Ethyl bedeuten,

U Sauerstoff bedeutet und

$R^{14}$ Phenyl oder einen Heterocyclus bedeutet, wobei jeder der beiden vorgenannten Reste unsubstituiert oder mit einem oder zwei Substituenten versehen sein kann und diese Substituenten gleich oder verschieden sind und jeweils Halogen, ($C_1$-$C_4$)Alkyl, ($C_1$-$C_4$)-Halogenalkyl, vorzugsweise $CF_3$, ($C_1$-$C_4$)Alkoxy, Nitro($C_1$-$C_4$)Alkylthio, ($C_1$-$C_4$)Alkoxy-($C_1$-$C_4$)alkyl, Phenyl, Phenoxy, Halogenphenoxy oder ($C_1$-$C_4$)Alkylphenoxy bedeuten,

$R^{14}$ weiterhin, für den Fall, daß U Sauerstoff ist, ($C_5$-$C_{10}$)Alkyl, Allyl, Geranyl, Farnesyl, ($C_1$-$C_4$)Halogenalkyl oder ($C_3$-$C_6$)Cycloalkylmethyl bedeutet, oder

$R^{14}$ weiterhin, für den Fall, daß U Sauerstoff ist, eine Gruppe der allgemeinen Formel IV bedeutet, worin

X' Stickstoff oder eine Gruppe der Formel CF bedeutet, sowie ihre Salze und ihre Stereoisomeren.

Am stärksten bevorzugt sind weiterhin Verbindungen der allgemeinen Formel I, worin

$R^1$ Wasserstoff bedeutet,
$R^2$ Methoxy oder Methoxymethyl, vorzugsweise Methoxymethyl bedeutet,
$R^3$ Methoxy bedeutet,
$R^4$ Wasserstoff bedeutet,
$R^5$ Wasserstoff, Methyl oder Cyclopropyl bedeutet,
Q die Bedeutung $Q^1$ hat und
$Q^1$ $(C_1-C_{15})$Alkyl bedeutet, gegebenenfalls substituiert mit einem, zwei oder drei Halogenatomen, einer $(C_1-C_{15})$Alkoxygruppe, einer $(C_4-C_8)$Cycloalkylalkoxygruppe, einer Dioxolanylgruppe, einer $(C_1-C_4)$Alkoxy-$(C_1-C_4)$alkoxygruppe, einer Hydroxygruppe, einer Methoxycarbonylgruppe, einer $(C_3-C_6)$-Cycloalkylgruppe, einer 2-[2-($(C_1-C_4)$Alkoxy)ethoxy]ethoxygruppe, einer Pyranyloxygruppe, einer Imidazol-1-ylgruppe, einer Triazol-1-ylgruppe, einer Pyrazol-1-ylgruppe oder einer Gruppe -A-B-$R^8$, worin A Sauerstoff, Schwefel oder Imino, B Carbonyl, Thiocarbonyl oder Sulfonyl bedeutet und $R^8$ $(C_1-C_4)$Alkyl, $(C_3-C_6)$Cycloalkyl, $(C_1-C_4)$Halogenalkyl, $(C_1-C_4)$Alkoxy, eine substituierte oder unsubstituierte Phenoxygruppe, eine substituierte oder unsubstituierte Phenylgruppe, eine substituierte oder unsubstituierte Anilinogruppe, eine 2,6-Dimethylmorpholin-4-yl-, 4-Methylpiperazin-1-yl-, Imidazol-1-yl-, Triazol-1-yl-, Pyrazol-1-ylgruppe oder eine Mono- oder Di-$(C_1-C_4)$-alkylaminogruppe bedeutet, sowie ihre Salze und ihre Stereoisomeren.

Am stärksten bevorzugt sind weiterhin Verbindungen der allgemeinen Formel I, worin

$R^1$ Wasserstoff bedeutet,
$R^2$ Methoxy oder Methoxymethyl, vorzugsweise Methoxymethyl bedeutet,
$R^3$ Methoxy bedeutet,
$R^4$ Wasserstoff bedeutet,
$R^5$ Wasserstoff oder Methyl bedeutet,
Q die Bedeutung $Q^2$ hat und
$Q^2$ eine Gruppe der allgemeinen Formeln II, II', II'', II''' oder II'''' bedeutet, worin
D eine ($C_1$ oder $C_2$)Alkylengruppe bedeutet,
E eine direkte Bindung oder Sauerstoff bedeutet,
$R^9$ und $R^{10}$ gleich oder verschieden sind und jeweils Wasserstoff, Halogen, $(C_1-C_4)$Alkyl, $(C_1-C_4)$-Halogenalkyl, $C_3$- oder $C_4$-Alkenyl, $(C_1-C_4)$Alkoxy, $(C_1-C_4)$Alkylthio oder Nitro bedeuten,
$R^{11}$ die für $R^9$ und $R^{10}$ angegebenen Bedeutungen hat, sowie für den Fall, daß E Sauerstoff bedeutet, zusätzlich eine Gruppe der Formel X-Y-$(GO)_n$-$R^{12}$ bedeutet, worin
X eine $(C_1-C_8)$Alkylengruppe oder eine $(C_1-C_8)$Alkylengruppe mit einem $(C_1-C_4)$-Alkoxysubstituenten bedeutet,
Y Sauerstoff, Schwefel oder eine Iminogruppe bedeutet,
G eine $(C_1-C_8)$Alkylengruppe oder eine $(C_1-C_4)$Alkylenoxy-$(C_1-C_4)$alkylengruppe bedeutet,
n Null oder 1 ist,
$R^{12}$ $(C_1-C_4)$Alkyl, $C_3$- oder $C_4$-Alkenyl, $(C_4-C_6)$Alkadienyl, $C_3$- oder $C_4$-Alkinyl, Phenyl-$(C_1-C_3)$alkyl oder eine Gruppe der Formel $CH_2$-W bedeutet, worin
W eine Gruppe der Formel CH = N-$OR^{13}$ bedeutet, worin
$R^{13}$ Wasserstoff, $(C_1-C_4)$Alkyl, $C_3$- oder $C_4$-Alkenyl oder Phenyl-$(C_1-C_3)$alkyl bedeutet,
W weiterhin eine Morpholinomethylgruppe oder eine heterocyclische Gruppe mit 5 bis 8 Ringatomen bedeutet, von denen 2 oder 3 Sauerstoff- oder Schwefelatome sind, und der besagte Heterocyclus gesättigt ist oder eine Doppelbindung im Ring besitzt und der besagte Heterocyclus unsubstituiert oder einen oder zwei Alkyl-, $(C_1-C_4)$Halogenlakyl- oder Phenylsubstituenten trägt, sowie ihre Salze und ihre Stereoisomeren.

Am stärksten bevorzugt sind weiterhin Verbindungen der allgemeinen Formel I, worin

$R^1$ Wasserstoff bedeutet,
$R^2$ Methoxy oder Methoxymethyl, vorzugsweise Methoxymethyl bedeutet,
$R^3$ Methoxy bedeutet,
$R^4$ Wasserstoff bedeutet,
$R^5$ Wasserstoff, Methyl, Ethyl oder Cyclopropyl bedeutet,
Q die Bedeutung $Q^3$ hat und
$Q^3$ eine Gruppe der allgemeinen Formel III bedeutet, worin $R^9$ und $R^{10}$ die oben angegebenen Bedeutungen haben,

U eine direkte Bindung, Sauerstoff, Schwefel, $(C_1-C_3)$Alkylen oder $(C_1-C_3)$Alkylenoxy bedeutet, und

$R^{14}$ Phenyl oder einen Heterocyclus bedeutet, wobei jeder der beiden vorgenannten Reste unsubstituiert oder mit einem oder zwei Substituenten versehen sein kann und diese Substituenten gleich oder verschieden sind und jeweils Halogen, $(C_1-C_4)$Alkyl, $(C_1-C_4)$Halogenalkyl, $(C_1-C_4)$Alkoxy, Nitro, $(C_1-C_4)$Alkylthio, $(C_1-C_4)$Alkoxy-$(C_1-C_4)$alkyl, Phenyl, Phenoxy, Halogenphenoxy oder $(C_1-C_4)$Alkylphenoxy bedeuten,

$R^{14}$ weiterhin, für den Fall, daß U Sauerstoff ist, $(C_5-C_{10})$Alkyl, Allyl, Geranyl, Farnesyl, $(C_1-C_4)$-Halogenalkyl, $(C_3-C_6)$Cycloalkylmethyl, eine Ethylgruppe, die in 2-Stellung durch $(C_1-C_4)$Alkoxy, $(C_1-C_4)$Alkoxy-$(C_1-C_4)$alkyl, $(C_1-C_4)$Alkylthio, $(C_1-C_4)$Alkylsulfonyl oder eine Phenoxygruppe, die gegebenenfalls mit einer oder zwei $(C_1-C_4)$Alkylgruppen substituiert sein kann, Glycidyl, Acetonyl, Halogenphenoxymethyldioxolanyl, 2,2-Diethoxyethyl, 1-Ethoxycabonylmethyl, Trimethylsilylmethyl, 1-Pyridylethyl oder eine $(C_1-C_4)$Alkylgruppe, die mit einer $(C_1-C_4)$Alkoxyimino- oder eine Benzyloxyiminogruppe substituiert ist, substituiert ist, bedeutet,

$R^{14}$ weiterhin, für den Fall, daß U Sauerstoff ist, eine Gruppe der allgemeinen Formel IV bedeutet, worin

X' Stickstoff oder eine Gruppe $CR^{15}$ bedeutet, worin

$R^{15}$ Wasserstoff, Halogen, $(C_1-C_4)$Alkyl, $(C_1-C_4)$Halogenalkyl, $(C_1-C_4)$Alkanoyl, Nitro, Cyano oder 1,3-Dioxolan-2-yl bedeutet, sowie ihre Salze und ihre Stereoisomeren.

Stärker bevorzugt sind auch Verbindungen der allgemeinen Formel I, worin

$R^1$ Wasserstoff, Methyl oder Halogen bedeutet,

$R^2$ und $R^3$ zusammen mit dem Kohlenstoffatom an das sie gebunden sind einen ungesättigten 5-gliedrigen Ring bilden, der ein an den Pyrimidin-Ring gebundenes Sauerstoff- oder Schwefelatom enthält und der gegebenenfalls durch $(C_1-C_4)$Alkyl oder Halogen substituiert ist,

$R^4$ Wasserstoff bedeutet,

$R^5$ Wasserstoff, $(C_1-C_4)$Alkyl oder Cyclopropyl bedeutet,

Q die Bedeutung $Q^1$ hat und

$Q^1$ $(C_1-C_{15})$Alkyl, gegebenenfalls substituiert mit einem, zwei oder drei Halogenatomen, einer $(C_1-C_{15})$Alkoxygruppe, einer $(C_4-C_8)$Cycloalkylalkoxygruppe, einer Dioxolanylgruppe, einer $(C_1-C_4)$Alkoxy-$(C_1-C_4)$-alkoxygruppe, einer Hydroxygruppe, einer Methoxycarbonylgruppe, einer $(C_3-C_6)$-Cycloalkylgruppe, einer 2-[2($(C_1-C_4)$Alkoxy)ethoxy]ethoxygruppe, einer Pyranyloxygruppe, einer Imidazol-1-ylgruppe, einer Triazol-1-ylgruppe, einer Pyrazol-1-ylgruppe oder einer Gruppe -A-B-$R^8$, worin A Sauerstoff, Schwefel oder Imino, B Carbonyl, Thiocarbonyl oder Sulfonyl bedeutet und $R^8$ $(C_1-C_4)$Alkyl, $(C_3-C_6)$Cycloalkyl, $(C_1-C_4)$Halogenalkyl, $(C_1-C_4)$Alkoxy, eine substituierte oder unsubstituierte Phenoxygruppe, eine substituierte oder unsubstituierte Phenylgruppe, eine substituierte oder unsubstituierte Anilinogruppe, eine 2,6-Dimethylmorpholin-4-yl-, 4-Methylpiperazin-1-yl-, Imidazol-1-yl-, Triazol-1-yl-, Pyrazol-1-ylgruppe oder eine Mono- oder Di-$(C_1-C_4)$-alkylaminogruppe bedeutet, sowie ihre Salze und ihre Stereoisomeren.

Stärker bevorzugt sind auch Verbindungen der allgemeinen Formel I für die

$R^1$ Wasserstoff, Methyl oder Halogen bedeutet,

$R^2$ und $R^3$ zusammen mit dem Kohlenstoffatom an das sie gebunden sind, einen ungesättigten 5-gliedrigen Ring bilden, der ein an den Pyrimidin-Ring gebundenes Sauerstoff- oder Schwefelatom enthält und der gegebenenfalls durch $(C_1-C_4)$Alkyl oder Halogen substituiert ist,

$R^4$ Wasserstoff bedeutet,

$R^5$ Wasserstoff, $(C_1-C_4)$Alkyl oder Cyclopropyl bedeutet,

Q die Bedeutung $Q^2$ hat und

$Q^2$ eine Gruppe der allgemeinen Formeln II, II', II'', II''' oder II'''' bedeutet, worin

D eine $(C_1-C_6)$Alkylengruppe bedeutet,

E eine direkte Bindung oder Methylenoxy bedeutet, und für den Fall, daß Q eine Gruppe der Formeln II', II'', II''' oder II'''' ist, E zusätzlich Sauerstoff bedeutet,

$R^9$ und $R^{10}$ gleich oder verschieden sind und jeweils Wasserstoff, Halogen, $(C_1-C_4)$Alkyl, $(C_3-C_6)$-Cycloalkyl, $(C_1-C_4)$Halogenalkyl, $C_3$- oder $C_4$-Alkenyl, $(C_1-C_4)$Alkoxy, $(C_1-C_4)$Alkylthio oder Nitro bedeuten,

$R^{11}$ die für $R^9$ und $R^{10}$ angegebenen Bedeutungen hat, sowie ihre Salze und ihre Stereoisomeren.

Stärker bevorzugt sind auch Verbindungen der allgemeinen Formel I, worin

$R^1$ Wasserstoff, Methyl oder Halogen bedeutet,

$R^2$ und $R^3$ zusammmen mit dem Kohlenstoffatom an das sie gebunden sind, einen ungesättigten 5-gliedrigen Ring bilden, der ein an den Pyrimidin-Ring gebundenes Schwefel- oder Sauerstoffatom enthält und gegebenenfalls durch $(C_1-C_4)$Alkyl oder Halogen substituiert ist,

$R^4$ Wasserstoff bedeutet,

$R^5$ Wasserstoff, $(C_1-C_4)$Alkyl, Alkyl oder Cyclopropyl bedeutet,

Q die Bedeutung $Q^3$ hat und

$Q^3$ eine Gruppe der allgemeinen Formel III bedeutet, worin $R^9$ und $R^{10}$ die oben angegebenen Bedeutungen haben,

U eine direkte Bindung, Sauerstoff, Schwefel, $(C_1-C_3)$Alkylen oder $(C_1-C_3)$Alkylenoxy bedeutet, und

$R^{14}$ Phenyl oder einen Heterocyclus bedeutet, wobei jeder der beiden vorgenannten Reste unsubstituiert oder mit einem oder zwei Substituenten versehen sein kann und diese Substituenten gleich oder verschieden sind und jeweils Halogen, $(C_1-C_4)$Alkyl, $(C_1-C_4)$-Halogenalkyl, $(C_1-C_4)$Alkoxy, Nitro, $(C_1-C_4)$Alkylthio, $(C_1-C_4)$Alkoxy-$(C_1-C_4)$alkyl, Phenyl, Phenoxy, Halogenphenoxy oder $(C_1-C_4)$Alkylphenoxy bedeuten,

$R^{14}$ weiterhin, für den Fall, daß U Sauerstoff ist, $(C_5-C_{10})$Alkyl, Allyl, Geranyl, Farnesyl, $(C_1-C_4)$Halogenalkyl, $(C_3-C_6)$Cycloalkylmethyl, eine Ethylgruppe, die in 2-Stellung durch $(C_1-C_4)$Alkoxy-, einer $(C_1-C_4)$Alkoxy-$(C_1-C_4)$alkyl-, $(C_1-C_4)$Alkylthio-, $(C_1-C_4)$-Alkylsulfonyl- oder eine Phenoxygruppe, die gegebenenfalls mit einer oder zwei $(C_1-C_4)$Alkylgruppen substituiert sein kann, Glycidyl, Acetonyl, Halogenphenoxymethyldioxolanyl, 2,2-Diethoxyethyl, 1-Ethoxycabonylmethyl, Trimethylsilylmethyl, 1-Pyridylethyl oder eine $(C_1-C_4)$Alkylgruppe, die mit einer $(C_1-C_4)$Alkoxyimino- oder eine Benzyloxyiminogruppe substituiert ist, substituiert ist, bedeutet, sowie ihre Salze und ihre Stereoisomeren.

Ganz besonders bevorzugt sind auch Verbindungen der allgemeinen Formel I, worin

$R^1$ Wasserstoff bedeutet,

$R^2$ und $R^3$ zusammen mit dem Kohlenstoffatom an das sie gebunden sind einen ungesättigten 5-gliedrigen Ring bilden, der ein an den Pyrimidin-Ring gebundenes Schwefelatom enthält,

$R^4$ Wasserstoff bedeutet,

$R^5$ Wasserstoff, Methyl oder Cyclopropyl bedeutet,

Q die Bedeutung $Q^1$ hat und

$Q^1$ $(C_1-C_{15})$Alkyl, gegebenenfalls substituiert mit einem, zwei oder drei Halogenatomen, einer $(C_1-C_{15})$Alkoxygruppe, einer $(C_4-C_8)$Cycloalkylalkoxygruppe, einer Dioxolanylgruppe, einer $(C_1-C_4)$Alkoxy-$(C_1-C_4)$alkoxygruppe, einer Hydroxygruppe, einer Methoxycarbonylgruppe, einer $(C_3-C_6)$Cycloalkylgruppe, einer 2-[2-($(C_1-C_4)$Alkoxy)ethoxy]ethoxygruppe, einer Pyranyloxygruppe, einer Imidazol-1-ylgruppe, einer Triazol-1-ylgruppe, einer Pyrazol-1-ylgruppe oder einer Gruppe -A-B-$R^8$, worin A Sauerstoff, Schwefel oder Imino, B Carbonyl, Thiocarbonyl oder Sulfonyl bedeutet und $R^8$ $(C_1-C_4)$Alkyl, $(C_3-C_6)$Cycloalkyl, $(C_1-C_4)$-Halogenalkyl, $(C_1-C_4)$Alkoxy, einer substituierte oder unsubstituierte Phenoxygruppe, eine substituierte oder unsubstituierte Phenylgruppe, eine substituierte oder unsubstituierte Anilinogruppe, einer 2,6-Dimethylmorpholin-4-yl-, 4-Methylpiperazin-1-yl-, Imidazol-1-yl-, Triazol-1-yl-, Pyrazol-1-ylgruppe oder ene Mono- oder Di-$(C_1-C_4)$-alkylaminogruppe bedeutet, sowie ihre Salze und ihre Stereoisomeren.

Ganz besonders bevorzugt sind auch Verbindungen der allgemeinen Formel I, worin

$R^1$ Wasserstoff bedeutet,

$R^2$ und $R^3$ zusammen mit dem Kohlenstoffatomen an das sie gebunden sind, einen ungesättigten 5-gliedrigen Ring bilden, der ein an den Pyrimidin-Ring gebundenes Schwefelatom enthält,

$R^4$ Wasserstoff bedeutet,

$R^5$ Wasserstoff oder Methyl bedeutet,

Q die Bedeutung $Q^2$ hat und

$Q^2$ eine Gruppe der allgemeinen Formeln II', II'', II''' oder II'''' bedeutet, worin

D eine $(C_1-C_6)$Alkylengruppe bedeutet,

E eine direkte Bindung oder Methylenoxy bedeutet, und für den Fall, daß $Q^2$ eine Gruppe der Formeln II', II'', II''' oder II'''' ist, E zusätzlich Sauerstoff bedeutet,

$R^9$ und $R^{10}$ gleich oder verschieden sind und jeweils Wasserstoff, Halogen, $(C_1-C_4)$Alkyl, $(C_3-C_6)$-Cycloalkyl, $(C_1-C_4)$Halogenalkyl, $C_3$- oder $C_3$-Alkenyl, $(C_1-C_4)$Alkoxy, $(C_1-C_4)$Alkylthio oder Nitro bedeuten, und

$R^{11}$ die für $R^9$ und $R^{10}$ angegebenen Bedeutungen hat, sowie ihre Salze und ihre Stereoisomeren.

Ganz besonders bevorzugt sind auch Verbindungen der allgemeinen Formel I, worin

$R^1$ Wasserstoff bedeutet,

$R^2$ und $R^3$ auch zusammen mit dem Kohlenstoffatom an das sie gebunden sind, einen ungesättigten 5-gliedrigen Ring bilden, der ein an den Pyrimidin-Ring gebundenes Schwefelatom enthält,

$R^4$ Wasserstoff bedeutet,

$R^5$ Wasserstoff, Methyl, Ethyl oder Cyclopropyl bedeutet,

Q die Bedeutung $Q^3$ hat und

$Q^3$ eine Gruppe der allgemeinen Formel III bedeutet, worin $R^9$ und $R^{10}$ die oben angegebenen Bedeutungen hat,

U eine direkte Bindung, Sauerstoff, Schwefel, $(C_1\text{-}C_3)$Alkylen oder $(C_1\text{-}C_3)$Alkylenoxy bedeutet, und

$R^{14}$ Phenyl oder einen Heterocyclus bedeutet, wobei jeder der beiden vorgenannten Reste unsubstituiert oder mit einem oder zwei Substituenten versehen sein kann und diese Substituenten gleich oder verschieden sind und jeweils Halogen, $(C_1\text{-}C_4)$Alkyl, $(C_1\text{-}C_4)$-Halogenalkyl, $(C_1\text{-}C_4)$Alkoxy, Nitro $(C_1\text{-}C_4)$Alkylthio, $(C_1\text{-}C_4)$Alkoxy-$(C_1\text{-}C_4)$alkyl, Phenyl, Phenoxy, Halogenphenoxy oder $(C_1\text{-}C_4)$Alkylphenoxy bedeuten,

$R^{14}$ weiterhin, für den Fall, daß U Sauerstoff ist, $(C_5\text{-}C_{10})$Alkyl, Allyl, Geranyl, Farnesyl, $(C_1\text{-}C_4)$Halogenalkyl, $(C_3\text{-}C_6)$Cycloalkylmethyl, eine Ethylgruppe, die in 2-Stellung durch $(C_1\text{-}C_4)$Alkoxy, $(C_1\text{-}C_4)$Alkoxy-$(C_1\text{-}C_4)$alkyl, $(C_1\text{-}C_4)$Alkylthio, $(C_1\text{-}C_4)$-Alkylsulfonyl oder eine Phenoxygruppe, die gegebenenfalls mit einer oder zwei $(C_1\text{-}C_4)$Alkylgruppen substituiert sein kann, Glycidyl, Acetonyl, Halogenphenoxymethyldioxolanyl, 2,2-Diethoxyethyl, 1-Ethoxycarbonylmethyl, Trimethylsilylmethyl, 1-Pyridylethyl oder eine $(C_1\text{-}C_4)$Alkylgruppe, die mit einer $(C_1\text{-}C_4)$Alkoxyimino- oder eine Benzyloxyiminogruppe substituiert ist, substituiert ist, bedeutet, sowie ihre Salze und ihre Stereoisomeren.

In der obigen Formel I ist unter "Halogen" ein Fluor-, Chlor-, Brom- oder Jodatom, vorzugsweise ein Chlor- oder Bromatom zu verstehen,

unter dem Ausdruck "$(C_1\text{-}C_4)$Alkyl" ein unverzweigter oder verzweigter Kohlenwasserstoffrest mit 1-4 Kohlenstoffatomen, wie z.B. der Methyl-, Ethyl-, Propyl-, 1-Methylethyl-, 2-Methylpropyl- oder 1,1-Dimethylethylrest,

unter dem Ausdruck "$(C_3\text{-}C_6)$Cycloalkyl" die Cyclopropyl-, Cyclobutyl-, Cyclopentyl- oder Cyclohexyl-Gruppe,

unter dem Ausdruck "$(C_1\text{-}C_4)$Alkoxy" eine Alkoxygruppe, deren Kohlenwasserstoffrest die unter dem Ausdruck "$(C_1\text{-}C_4)$Alkyl" angegebene Bedeutung hat,

unter dem Ausdruck "$(C_1\text{-}C_4)$Alkylthio" eine Alkylthiogruppe, deren Kohlenwasserstoffrest die unter dem Ausdruck "$(C_1\text{-}C_4)$Alkyl" angegebene Bedeutung hat,

unter dem Ausdruck "$(C_1\text{-}C_4)$-Halogenalkyl" eine unter dem Ausdruck "$(C_1\text{-}C_4)$Alkyl" genannten Alkylgruppen in der eines oder mehrere Wasserstoffatome durch die obengenannten Halogenatome, bevorzugt Chlor oder Fluor, ersetzt sind, wie beispielsweise die Trifluormethylgruppe, die 2,2,2-Trifluorethylgruppe, die Chlormethyl- oder Fluormethylgruppe,

unter dem Ausdruck "$(C_1\text{-}C_4)$Alkoxy-$(C_1\text{-}C_4)$alkyl" beispielsweise eine 1-Methoxyethylgruppe, eine 2-Methoxyethylgruppe, eine 2-Ethoxyethylgruppe, eine Methoxymethyl- oder Ethoxymethylgruppe, eine 3-Methoxypropylgruppe oder eine 4-Butoxybutylgruppe,

unter dem Ausdruck "$(C_1\text{-}C_4)$Alkylthio-$(C_1\text{-}C_4)$alkyl" beispielsweise Methylthiomethyl, Ethylthiomethyl, Propylthiomethyl, 2-Methylthiomethyl, 2-Ethylthioethyl oder 3-Methylthiopropyl,

unter dem Ausdruck "$(C_1\text{-}C_4)$Alkylamino" eine Alkylaminogruppe, deren Kohlenwasserstoffrest die unter dem Ausdruck "$(C_1\text{-}C_4)$Alkyl" angegebene Bedeutung hat, bevorzugt die Ethyl- und Methylaminogruppe,

unter dem Ausdruck "$(C_1\text{-}C_4)$Dialkylamino" eine Dialkylaminogruppe, deren Kohlenwasserstoffreste die unter dem Ausdruck "$(C_1\text{-}C_4)$Alkyl" angegebene Bedeutung hat, bevorzugt die Dimethyl- und Diethylaminogruppe.

Beispiele für den gesättigten oder ungesättigten 5- oder 6-gliedrigen Ring, den $R^6$ und $R^7$ zusammen mit dem Stickstoffatom an das sie gebunden sind bilden und der zusätzlich weitere Stickstoffatome oder ein Sauerstoff- oder Schwefelatom enthalten kann, sind die Imidazol-1-yl-, die Pyrazol-1-yl-, 1,2,4-Triazol-1-yl-, die Thiazol-1-yl-, Piperazin-1-yl-, die Morpholin-4-yl- oder Thiomorpholin-4-ylgruppe.

Unter dem Ausdruck "$(C_1\text{-}C_{15})$Alkyl" ist zu verstehen ein verzweigter oder unverzweigter Kohlenwasserstoffrest mit 1-15 Kohlenstoffatomen wie z.B. die vorstehend unter "$(C_1\text{-}C_4)$Alkyl" genannten Reste oder der

Pentyl-, Hexyl-, Heptyl-, Octyl-, 1-Nonyl-, 2-Nonyl- 1-Decyl-, 2-Decyl-, 1-Undecyl-, 2-Undecyl-, Dodecyl-, Tridecyl-, 4-Methylpentyl-, der Tetradecyl- oder der Pentadecylrest,
unter dem Ausdruck "($C_1$-$C_{15}$)Alkoxy" eine Alkoxygruppe deren Alkylgruppen die vorstehend unter "($C_1$-$C_{15}$)Alkyl" vorstehend genannte Bedeutung haben,
unter dem Ausdruck "($C_4$-$C_8$)-Cycloalkylalkoxy" z.B. die Cyclopropylmethoxygruppe, die Cyclopropylethoxygruppe, die Cyclobutylmethoxygruppe, die Cyclopentylmethoxygruppe, die Cyclohexylmethoxygruppe oder die Cyclohexylethoxygruppe,
unter dem Ausdruck "($C_1$-$C_4$)Alkoxy-($C_1$-$C_4$)alkoxy" z.B. eine 2-Ethoxyethoxygruppe, eine 2-Methoxyethoxygruppe, eine 2-Propoxyethoxygruppe, eine 2-Butoxyethoxygruppe, eine 3-Methoxypropoxygruppe oder eine 4-Methoxybutoxygruppe,
unter dem Ausdruck "($C_1$-$C_6$)Alkylen" eine geradkettige oder verzweigte Alkylenkette mit 1-6 Kohlenstoffatomen wie z.B. die Methylengruppe, die Ethylengruppe, die Trimethylengruppe, die Tetramethylengruppe, $-CH(CH_3)-$,

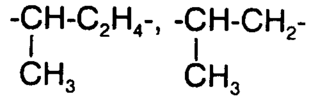

oder

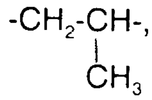

unter dem Ausdruck "($C_1$-$C_8$)Alkylen" eine geradkettige oder verzweigte Alkylenkette mit 1-8 Kohlenstoffatomen wie z.B. die vorstehend unter "($C_1$-$C_3$)Alkylen" genannten Reste oder die Tetramethylengruppe, die Pentamethylengruppe, die Hexamethylengruppe, die Heptamethylengruppe oder die Octamethylengruppe,
unter dem Ausdruck "$C_3$- oder $C_4$-Alkenyl" Allyl, 1-Methylallyl, 2-Methylallyl, 1-Butenyl- oder 2-Butenyl,
unter dem Ausdruck "($C_4$-$C_6$)Alkadienyl" 1,3-Butadienyl oder 1,4-Hexadienyl,
unter dem Ausdruck "$C_3$- oder $C_4$-Alkinyl" 1-Propinyl, 2-Propinyl oder 2-Butinyl,
unter dem Ausdruck "Phenyl-($C_1$-$C_3$)alkyl" z.B. Benzyl, Phenylethyl oder $\alpha$-Methylbenzyl.

Die oben gegebene Erläuterung gilt entsprechend for Homologe.

Die vorliegende Erfindung betrifft die Verbindungen der Formel I in Form der freien Base oder eines Säureadditionssalzes. Säuren, die zur Salzbildung herangezogen werden können, sind anorganische Säuren, wie Salzsäure, Bromwasserstoffsäure, Salpetersäure, Schwefelsäure, Phosphorsäure oder organische Säuren wie Ameisensäure, Essigsäure, Propionsäure, Malonsäure, Oxalsäure, Fumarsäure, Adipinsäure, Stearinsäure, Ölsäure, Methansulfonsäure, Benzolsulfonsäure oder Toluolsulfonsäure.

Die Verbindungen der Formel I weisen zum Teil ein oder mehrere asymmetrische Kohlenstoffatome auf. Es können daher Racemate und Diastereomere auftreten. Die Erfindung umfaßt sowohl die reinen Isomeren als auch deren Gemische. Die Gemische von Diastereomeren können nach gebräuchlichen Methoden, z.B. durch selektive Kristallisation aus geeigneten Lösungsmitteln oder durch Chromatographie in die Komponenten aufgetrennt werden. Racemate können nach üblichen Methoden in die Enantiomeren aufgetrennt werden, so z.B. durch Salzbildung mit einer optisch aktiven Säure, Trennung der diasteromeren Salze und Freisetzung der reinen Enantiomeren mittels einer Base.

Gegenstand der Erfindung ist weiterhin ein Verfahren zur Herstellung von Verbindungen der Formel I, dadurch gekennzeichnet, daß man eine Verbindung der allgemeinen Formel V

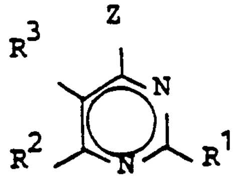

(V),

worin $R^1$, $R^2$ und $R^3$ die unter Formel I angegebenen Bedeutungen haben, und Z die Abgangsgruppe Halogen, Alkylthio, Alkansulfonyloxy oder Arylsulfonyloxy, Alkylsulfonyl oder Arylsulfonyl bedeutet, mit einem Amin der allgemeinen Formel VI,

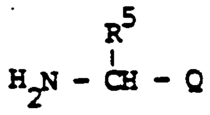

(VI),

worin $R^5$ und Q die unter Formel I angegebenen Bedeutungen haben, umsetzt und die so erhaltenen Verbindungen der Formel I gegebenenfalls am Stickstoff carbamoyliert oder am $C_5$-Atom des Pyrimidins chloriert oder bromiert.

Die oben beschriebene Substitutionsreaktion ist im Prinzip bekannt. Die Abgangsgruppe Z ist in weiteren Grenzen variierbar und kann beispielsweise ein Halogenatom wie Fluor, Chlor, Brom oder Jod bedeuten oder Alkylthio wie Methyl- oder Ethylthio, oder Alkansulfonyloxy wie Methan-, Trifluormethan- oder Ethansulfonyloxy oder Arylsulfonyloxy, wie Benzolsulfonyloxy oder Toluolsulfonyloxy oder Alkylsulfonyl wie Methyl- oder Ethylsulfonyl oder Arylsulfonyl wie Phenyl- oder Toluolsulfonyl.

Die vorgenannte Reaktion wird in einem Temperaturbereich von 20-150 °C, zweckmäßig in Anwesenheit einer Base und gegebenenfalls in einem inerten organischen Lösungsmittel wie N,N-Dimethylformamid, N,N-Dimethylacetamid, Dimethylsulfoxid, N-Methylpyrrolidin-2-on, Dioxan, Tetrahydrofuran, 4-Methyl-2-pentanon, Methanol, Ethanol, Butanol, Ethylenglykol, Ethylenglykoldimethylether, Toluol, Chlorbenzol oder Xylol durchgeführt. Es können auch Gemische der genannten Lösungsmittel verwendet werden.

Geeignete Basen sind beispielsweise Alkali- oder Erdalkalimetallcarbonate, -hydrogencarbonate, -hydroxide, -alkoholate oder -hydride wie Natriumcarbonat, Natriumhydrogencarbonat, Kaliumcarbonat, Natriumhydroxid, Natriummethylat oder Natriumhydrid oder organische Basen wie Triethylamin oder Pyridin. Auch ein zweites Äquivalent des Amins VI kann als Hilfsbase eingesetzt werden.

Gegenstand der Erfindung ist weiterhin ein Verfahren zur Herstellung von Verbindungen der allgemeinen Formel I'

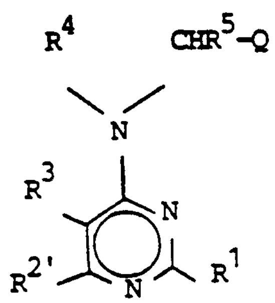

(I'),

worin $R^1$, $R^3$, $R^4$, $R^5$ und Q die unter Formel I angegebenen Bedeutungen hat und $R^{2'}$ $(C_1-C_4)$Alkoxy, $(C_1-C_4)$Halogenalkoxy, $(C_1-C_4)$Alkylthio, $(C_1-C_4)$Alkylamino oder $(C_1-C_4)$Dialkylamino bedeutet, dadurch gekennzeichnet, daß man eine Verbindung der allgemeinen Formel VII

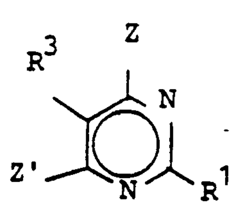

(VII)

worin Z und Z' gleich oder verschieden sein können und die Abgangsgruppe Halogen, Alkylthio, Alkansulfonyloxy, Arylsulfonyloxy, Alkylsulfonyl oder Arylsulfonyl bedeuten, und $R^1$ und $R^3$ die unter Formel I angegebene Bedeutung haben, mit einer Verbindung der allgemeinen Formel VI

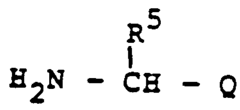
(VI),

worin $R^5$ und Q die unter Formel I angegebenen Bedeutungen haben, zu einer Verbindung der allgemeinen Formel VIII

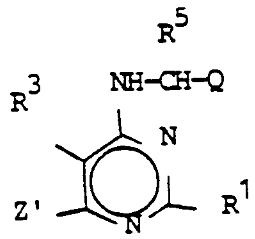
(VIII),

worin $R^1$, $R^3$, $R^5$, Q und Z' die oben angegebenen Bedeutungen haben, umsetzt und in einem zweiten Reaktionsschritt die Verbindung der allgemeinen Formel VIII mit einer Verbindung der Formel IX umsetzt

$HR^{2'}$ (IX),

worin $R^{2'}$ die oben angegebenen Bedeutungen hat, und die so erhaltene Verbindung der Formel I' gegebenenfalls am Stickstoff carbamoyliert oder gegebenenfalls falls $R^3$ Wasserstoff bedeutet, chloriert oder bromiert.

Zur Herstellung der Verbindungen der Formel I' wird dabei völlig analog der oben beschriebenen Herstellung der Verbindungen der Formel I durch Umsetzung der Verbindungen V mit den Verbindungen VI verfahren.

Die im zweiten Reaktionsschritt benötigte Abgangsgruppe Z' kann die gleiche Bedeutung wie die oben beschriebene Abgangsgruppe Z haben. Was die Reaktionsbedingungen betrifft, so können im zweiten Reaktionsschritt die gleichen Lösungsmittel, Hilfsbasen und Reaktionstemperaturen zur Anwendung kommen wie bei der oben beschriebenen Herstellung der Verbindungen der Formel I aus den Verbindungen der Formeln V und VI. Beide Reaktionsschritte können ohne Aufarbeitung nach der ersten Reaktionsstufe als Eintopfreaktion durchgeführt werden.

Gegenstand der Erfindung ist weiterhin ein Verfahren zur Herstellung von Verbindungen der Formel I', dadurch gekennzeichnet, daß man eine Verbindung der obengenannten Formel VII mit einer Verbindung der Formel IX zu einer Verbindung der Formel X

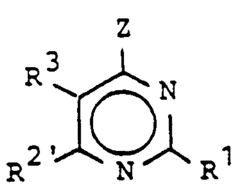
(X),

worin $R^1$ und $R^3$ die unter Formel I angegebene Bedeutung haben,
$R^{2'}$ $(C_1\text{-}C_4)$Alkoxy, $(C_1\text{-}C_4)$Halogenalkoxy, $(C_1\text{-}C_4)$Alkylthio, $(C_1\text{-}C_4)$Alkylamino oder $(C_1\text{-}C_4)$Dialkylamino bedeutet und Z die unter Formel (V) angegebenen Bedeutungen hat, umsetzt, die Verbindung der allgemeinen Formel X mit einem Amin der Formel VI umsetzt und die so erhaltene Verbindung der Formel

I' gegebenenfalls am Stickstoff carbamoyliert, oder, falls $R^3$ Wasserstoff bedeutet, chloriert oder bromiert.

Was die Reaktionsbedingungen betrifft, so können für beide Reaktionsschritte die gleichen Lösungsmittel, Hilfsbasen und Reaktionstemperaturen zur Anwendung kommen wie bei der oben beschriebenen Herstellung der Verbindungen der Formel I aus den Verbindungen der Formeln V und VI.

Beide Reaktionsschritte können auch ohne Aufarbeitung nach der ersten Reaktionsstufe als Eintopfreaktion durchgeführt werden.

Die Ausgangsverbindungen der Formel V können in Analogie zu bekannten Verfahren hergestellt werden. Als Ausgangsprodukte dienen Acetessigester-Derivate, die über die entsprechenden Hydroxypyrimidine in die Chlorpyrimidine überführt werden:

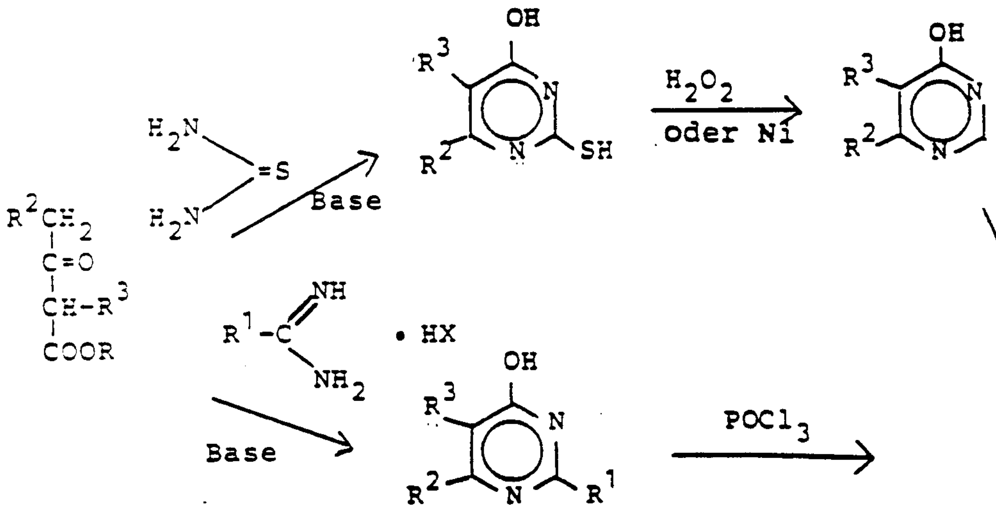


(V)
(Z=Cl)

Die Ausgangsverbindungen der Formel VII können in Analogie zu bekannten Verfahren aus Malonester-Derivate erhalten werden:

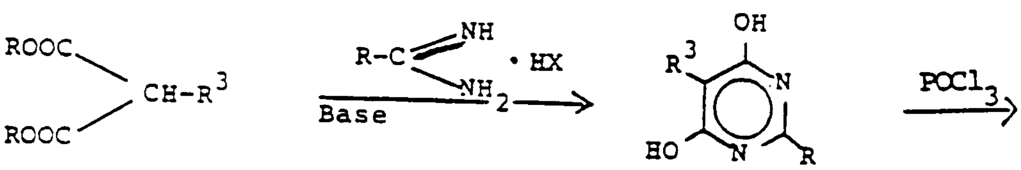


(VII)
(Z=Z'=Cl)

Die als Ausgangsprodukte benötigten Amine der Formel VI können nach bekannten Verfahren hergestellt werden.

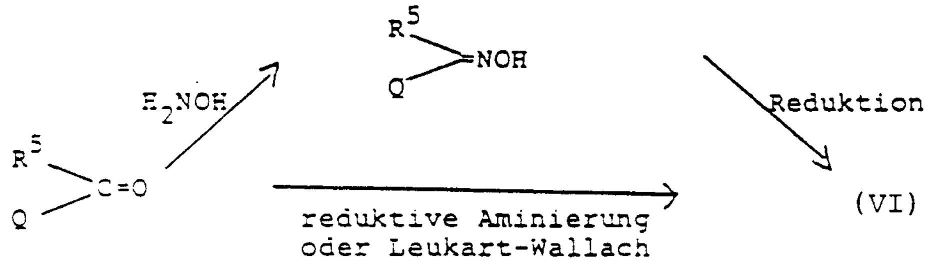


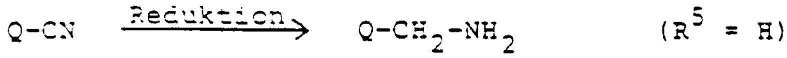


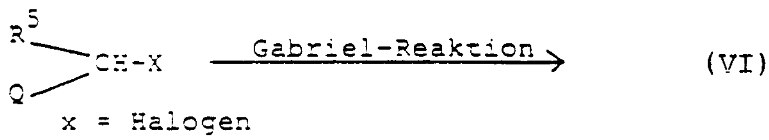


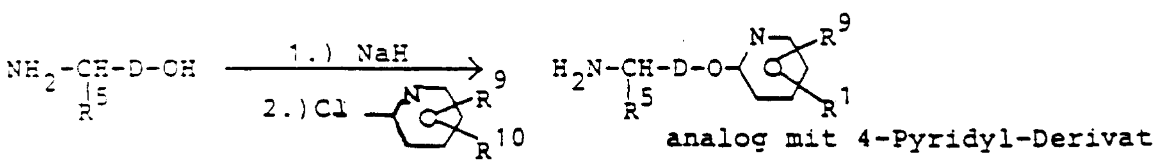


Die Verbindungen der Formel I, für die $R^3$ Wasserstoff bedeutet, können gegebenenfalls nach bekannten Verfahren halogeniert werden.

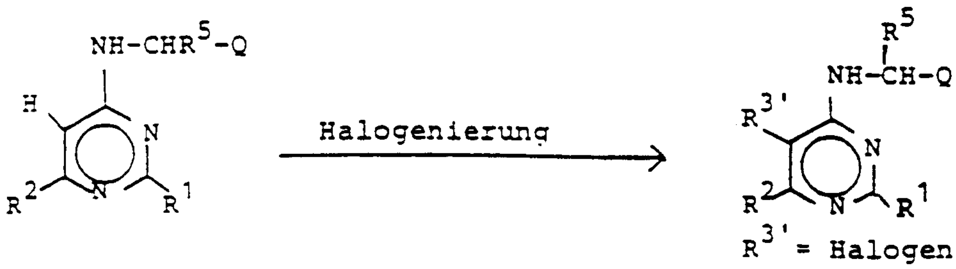


Die Wirkstoffe eignen sich bei guter Pflanzenverträglichkeit zur Bekämpfung von tierischen Schädlingen, insbesondere Insekten, Spinnentieren, Helminthen und Mollusken, ganz besonders bevorzugtzur Bekämpfung von Insekten und Spinnentieren, die in der Landwirtschaft, bei der Tierzucht, in Forsten, im Vorrats- und Materialschutz sowie auf dem Hygienesektor vorkommen.

Hervorzuheben ist die Eigenschaft der Wirkstoffe von der Pflanze über Stengel und Blätter aufgenommen zu werden und durch basipetalen Transport bis in die Wurzeln transportiert zu werden und damit eine effektive Kontrolle von Nematoden zu ermöglichen.

Die Wirkstoffe sind gegen normal sensible und resistente Arten sowie alle oder einzelne Entwicklungsstadien wirksam. Zu den oben erwähnten Schädlingen gehören:

Aus der Ordnung der Acarina z.B. Acarus siro, Agras spp., Ornithrodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Eotetranychus spp., Oligonychus spp., Eutetranychus spp.

Aus der Ordnung der Isopoda, z.B. Oniscus asselus, Armadium vulgare, Porcellio scaber.

Aus der Ordnung der Diplopoda z.B. Blaniulus guttulatus. Aus der Ordnung der Chilopoda z.B. Geophilus carpophagus, Scutigera spp.

Aus der Ordnung der Symphyla z.B. Scutigerella immaculata.

Aus der Ordung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blatella germanica, Acheta domesticus, Gryllotalpa spp., Locusta migratoria migratorioides, Melanoplus differentialis, Schistocerca gregarai.

Aus der Ordnung der Dermaptera z.B. Forficula auricularia.

Aus der Ordnung des Isoptera z.B. Reticulitermes spp.

Aus der Ordnung der Anoplura z.B. Phylloera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera a.B. Aleurodes brassicae, Bemisia tabaci, Trialeurodes vaporarium, Aphis gossypii, Bravicornyne brassicae, Cryptomyzus ribis, Doralis fabae, Doralis pomi, Eriosoma lanigerum, Hyalopterus arundinis, Macrosiphon avenae, Myzus spp., Phorodon humili, Rhopalosiphon padi, Empoasca spp., Euscelus bilobatus, Naphotettix cincticeps, Lecanium corni, Saissetia oleae, Laodelphax striatellus, Nilaparvata lugens, Aonidiella aurantii, Apidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podanana, Capua reticulana, Choristoneura fumiferana, Clysia ambiguella, Homona magnanima, Tortris viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylloides chrysocephala, Epilachna varivestis, Atomaria spp., Oryzaephilus surinamensis, Anthonumus spp., Sitophilus spp., Otiorrhynchus sulcatus, Cosmopolites sordidus, Ceuthorrhynchus assimilis, Hypera postica, Dermestes spp., Trogoderma, Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliophora erythrocephala, Lucilia spp., Chrysomyia spp., Cuterebra spp., Gastrophilus spp., Hypobosca spp., Stomoxys spp., Oestrus spp. Hypoderma spp., Tabanus spp., Tannia spp., Bibio hortulanus, Oscinella frit, Phorbia spp., Pegomia hyoscyami, Ceratitis capitata, Dacus oleae, Tipula paludosa.

Aus der Ordnung der Siphonaptera z.B. Xeonopsylla cheopsis, Ceratophyllus spp.

Aus der Ordnung der Arachnida z.B. Scorpio maurus, Latrodectus mactans.

Aus der Klasse der Helminthen z.B. Haemonchus, Trichostrongulus, Ostertragia. Cooperia, Chabertia, Strongyloides, Oesophagostomum, Hyostrongulus, Ancylostoma, Ascaris und Heterakis sowie Fasciola und pflanzenschädigende Nematoden z.B. solche der Gattungen Meloidogyne, Heterodera, Ditylenchus, Aphelenchoides, Radopholus, Globodera, Pratylenchus, Longidorus und Xiphenema.

Aus der Klasse der Gastropoda z.B. Deroceras spp., Arion spp., Lymnaea spp., Galba spp., Succinea spp., Biophalaria spp., Bulinus spp., Oncomelania spp. Aus der Klasse der Bivalva z.B. Dreissena spp.

Gegenstand der Erfindung sich auch insektizide und akarizide Mittel, die die Verbindungen der Formel I neben geeigneten Formulierungshilfsmitteln enthalten.

Die erfindungsgemäßen Mittel enthalten die Wirkstoffe der Fomel I im allgemeinen zu 1 bis 95 Gew.-%.

Sie können auf verschiedene Art formuliert werden, je nachdem wie es durch die biologischen und/oder chemischphysikalischen Parameter vorgegeben ist. Als Formulierungsmöglichkeiten kommen daher infrage: Spritzpulver (WP), emulgierbare Konzentrate (EC), wäßrige Lösungen (SC), Emulsionen, versprühbare Lösungen, Dispersionen auf Öl- oder Wasserbasis (SC), Suspoemulsionen (SC), Stäubemittel (DP), Beizmittel, Granulate in Form von Mikro-, Sprüh-, Aufzugs- und Adsorptionsgranulaten, wasserdispergierbare Granulate (WG), ULV-Formulierungen, Mikrokapseln, Wachse oder Köder.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of

Insecticide Dust Diluents and Cariers", 2nd Ed., Darland Books, Caldwell N.J.; H.v.Olphen, "Introduction to Clay Colloid Chemistry", 2nd Ed., J. Wiley & Sons, N.Y.; Marsden, "Solvents Guide", 2nd Ed., Interscience, N.Y. 1950; McCutcheon's, "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl- oder Alkylphenol-sulfonate und Dispergiermittel, z.B. ligninsulfonsaures Natrium, 2,2'-dinapthylmethan-6,6'-disulfonsaures Natrium, dibutylnaphthalin-sulfonsaures Natrium oder auch oleylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch hörersiedenden Aromaten oder Kohlenwasserstoffen unter Zusatz von einem oder mehreren Emulgatoren hergestellt. Als Emulgatoren können beispielsweise verwendet werden:

Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit oder Pyrophillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Die erfindungsgemäßen Wirkstoffe können in ihren handelsüblichen Formulierungen sowie in den aus diesen Formulierungen bereiteten Anwendungsformen in Mischungen mit anderen Wirkstoffen, wie Insektiziden, Lockstoffen, Sterilantien, Akariziden, Nematiziden, Fungiziden, wachstumsregulierenden Stoffen oder Herbiziden vorliegen.

Zu den Schädlingsbekämpfungsmitteln zählen beispielsweise Phosphorsäureester, Carbamate, Carbonsäureester, Formamidine, Zinnverbindungen, durch Mikroorganismen hergestellte Stoffe u.a. Bevorzugte Mischungspartner sind

1. aus der Gruppe der Phosphorverbindungen Acephate, Azamethiphos, Azinphos-ethyl, Azinphos-methyl, Bromophos, Bromophos-ethyl, Chlorfenvinphos, Chlormephos, Chlorpyrifos, Chlorpyrifos-methyl, Demeton, Demeton-S-methyl, Demeton-S-methyl sulphone, Dialifos, Diazinon, Dichlorvos, Dicrotophos, O,O-1,2,2,2-tetrachlorethylphosphorthioate (SD 208 304), Dimethoate, Disulfoton, EPN, Ethion, Ethoprophos, Etrimfos, Famphur, Fenamiphos, Fenitriothion, Fensulfothion, Fenthion, Fonofos, Formothion,

Heptenophos, Isozophos, Isothioate, Isoxathion, Malathion, Methacrifos, Methamidophos, Methidation, Salithion, Mevinphos, Monocrotophos, Naled, Omethoate, Oxydemeton-methyl, Parathion, Parathion-methyl, Phenthoate, Phorate, Phosalone, Phosfolan, Phosmet, Phosphamidon, Phoxim, Pirimiphos-ethyl, Pirimiphos-methyl, Profenofos, Propaphos, Proetamphos, Prothiofos, Pyraclofos, Pyridapenthion, Quinalphos, Sulprofos, Temephos, Terbufos, Tetrachlorvinphos, Thiometon, Triazophos, Trichlorphon, Vamidothion;

2. aus der Gruppe der Carbamate

Aldicarb, 2-sec.-Butylphenylmethylcarbamate (BPMC), Carbaryl, Carbofuran, Carbosulfan, Cloethocarb, Benfuracarb, Ethiofencarb, Furathiocarb, Isoprocarb, Methomyl, 5-Methyl-m-cu-menylbutyryl(methyl)-carbamate, Oxamyl, Pirimicarb, Propoxur, Thiodicarb, Thiofanox, Ethyl 4,6,9-triaza-4-benzyl-6,10-dimethyl-8-oxa-7-oxo-5,11-dithia-9-dodecenoate (OK 135), 1-methylthio(ethylideneamino)-N-methyl-N-(morpholinothio)carbamate (UC 51717);

3. aus der Gruppe der Carbonsäureester

Allethrin, Alphamethrin, 5-Benzyl-3-furylmethyl-(E)-(1R)-cis-2,2-di-methyl-3-(2-oxothiolan-3-ylidenemethyl)cyclopropanecarboxylate, Bioallethrin, Bioallethrin((S)-cyclopentylisomer), Bioresmethrin, Biphenate, (RS)-1-Cyano-1-(6-phenoxy-2-pyridyl)methyl-(1RS)-trans-3-(4-tert.butylphenyl)-2,2-dimethylcyclopropanecarboxylate (NCI 85193), Cycloprothrin, Cyfluthrin, Cyhalothrin, Cypermethrin, Cyphenothrin, Deltamethrin, Emphenthrin, Esfenvalerate, Fenfluthrin, Fenpropathrin, Fenvalerate, Flucythrinate, Flumethrin, Fluvalinate (D-isomer), Permethrin, Pheothrin ((R)-Isomer), d-Pralethrin, Pyrethrine (natürliche Produkte), Resmethrin, Tefluthrin, Tetramethrin, Tralomethrin;

4. aus der Gruppe der Amidine

Amitraz, Chlordimeform;

5. aus der Gruppe der Zinnverbindungen

Cyhexatin, Fenbutatinoxide;

6. Sonstige

Abamectin, Bacillus thuringiensis, Bensultap, Binapacryl, Bromopropylate, Buprofezin, Camphechlor, Cartap, Chlorobenzilate, Chlorfluazuron, 2-(4-(Chlorphenyl)-4,5-di-phenylthiophen (UBI-T 930), Chlorfentezine,

Cyclopropancarbonsäure-(2-naphthylmethyl)ester (Ro 12-0470), Cyromazin, N-(3,5-Dichlor-4-(1,1,2,3,3,3-hexafluor-1-propyloxy)phenyl)carbamoyl)-2-chlorbenzcarboximidsäureethylester, DDT, Dicofol, N-(N-(3,5-Di-chlor-4-(1,1,2,2-tetrafluorethoxy)phenylamino)carbonyl)-2,6-difluorbenzamid (XRD 473), Diflubenzuron, N-(2,3-Dihydro-3-methyl-1,3-thiazol-2-ylidene)-2,4-xylidine, Dinobuton, Dinocap, Endosulfan, Ethofenprox, (4-Ethoxyphenyl) (dimethyl)(3-(3-phenoxyphenyl)propyl)silan, (4-Ethoxyphenyl) (3-(4-fluoro-3-phenoxyphenyl)propyl)dimethylsilan, Fenoxycarb, 2-Fluoro-5-(4-(4-ethoxyphenyl)-4-methyl-1-pentyl)-diphenylether (MTI 800), Granulose- und Kernpolyederviren, Fenthiocarb, Flubenzimine, Flucycloxuron, Flufenoxuron, Gamma-HCH, Hexythiazox, Hydramethylnon (AC 217300), Ivermectin, 2-Nitromethyl-4,5-dihydro-6H-thiazin (SD 52618), 2-Nitromethyl-3,4-dihydrothiazol (SD 35651), 2-Nitromethylene-1,2-thiazinan-3-ylcarbamaldehyde (WL 108477), Propargite, Teflubenzuron, Tetradifon, Tetrasul, Thiocyclam, Triflumuron.

Der Wirkstoffgehalt der aus den handelsüblichen Formulierungen bereiteten Anwendungsformen kann von 0,00000001 bis zu 100 Gew.-% Wirkstoff, vorzugsweise zwischen 0,00001 und 1 Gew.-% liegen.

Die Anwendung geschieht in einer den Anwendungsformen angepaßten üblichen Weise.

Die erfindungsgemäßen Wirkstoffe eignen sich auch zur Bekämpfung von Endo- und Ektoparasiten auf dem veterinärmedizinischen Gebiet bzw. auf dem Gebiet der Tierhaltung.

Die Anwendung der erfindungsgemäßen Wirkstoffe geschieht hier in bekannter Weise wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießen (pour-on and spot-on) und des Einpuderns sowie durch parenterale Anwendung in Form beispielsweise der Injektion.

Die erfindungsgemäßen neuen Verbindungen der Formel I können demgemäß auch besonders vorteilhaft in der Viehhaltung (z.B. Rinder, Schafe, Schweine und Geflügel wie Hühner, Gänse usw.) eingesetzt werden. In einer bevorzugten Ausführungsform der Erfindung werden den Tieren die neuen Verbindungen, gegebenenfalls in geeigneten Formulierungen (vgl. oben) und gegebenenfalls mit dem Trinkwasser oder Futter oral verabreicht. Da eine Ausscheidung im Kot in wirksamer Weise erfolgt, läßt sich auf diese Weise sehr einfach die Entwicklung von Insekten im Kot der Tiere verhindern. Die jeweils geeigneten Dosierungen und Formulierungen sind insbesondere von der Art und dem Entwicklungsstadium der Nutztiere und auch vom Befallsdruck abhängig und lassen sich nach den üblichen Methoden leicht ermitteln und festlegen. Die neuen Verbindungen können bei Rindern z.B. in Dosierungen von 0,01 bis 1 mg/kg Körpergewicht eingesetzt werden.

Die erfindungsgemäßen Verbindungen der Formel I zeichnen sich durch eine hervorragende fungizide Wirkung aus. Bereits in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der beanspruchten Verbindungen erfaßt eine Vielzahl verschiedener wirtschaftlich bedeutender, phytopathogener Pilze, wie z. B. Pyricularia oryzae, Venturia inaequalis, Cercospora beticola, echte Mehltauarten, Fusariumarten, Plasmopara viticola, verschiedene Rostpilze und Pseudocercosporella herpotrichoides.

Die erfindungsgemäßen Verbindungen eignen sich daneben auch für den Einsatz in technischen Bereichen, beispielsweise als Holzschutzmittel, als Konservierungsmittel in Anstrichfarben, in Kühlschmiermittel für die Metallbearbeitung oder als Konservierungsmittel in Bohr- und Schneidölen.

Gegenstand der Erfindung sind auch fungizide Mittel, die die Verbindungen der Formel I neben geeigneten Formulierungshilfsmitteln enthalten. Die erfindungsgemäßen Mittel enthalten die Wirkstoffe der Formel I im allgemeinen zu 1 bis 95 Gew.-%.

Sie können auf verschiedene Art formuliert werden, je nachdem die es durch die biologischen und/oder chemisch-physikalischen Parameter vorgegeben ist. Als Formulierungsmöglichkeiten kommen daher in Frage:
Spritzpulver (WP), emulgierbare Konzentrate (EC), wäßrige Dispersionen auf öl- oder Wasserbasis (SC), Suspoemulsionen (SC), Stäubemittel (DP), Beizmittel, Granulate in Form von wasserdispergierbare Granulate (WG), ULV-Formulierungen, Mikrokapseln, Wachse oder Köder.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in:
Watkins, "Handbook of Insecticide Dust Diluents and Carrier", 2nd Ed., Darland Books, Caldwell N.J.; H.v.Olphen, "Introduction to Clay Colloid Chemistry, 2nd Ed., J. Wiley & Sons, N.Y.; Marsden, "Solvents Guide", 2nd Ed., Interscience, N.Y. 1950; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Bund 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z. B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Netzmittel, z. B. polyoxethylierte Alkylphenole, polyoxethylierte Fettalkohole, Alkyl- oder Alkylphenol-sulfonate und Dispergiermittel, z. B. ligninsulfonsaures Natrium, 2,2'-dinaphthylmethan-6,6`-disulfonsaures Natrium, dibutylnaphthalinsulfonsaures Natrium oder auch oleylmethyltaurinsaures Natrium enthalten. Emulgierbare Konzentrate werden durch Auflösen des Wirkstoffes in einem organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden:
Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z. B. Talkum, natürlichen Tonen wie Kaolin, Bentonit oder Pyrophyllit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z. B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-%. Bei Granulaten hängt der Wirkstoffgehalt zum Teil davon ab, ob die wirksame Verbindung flüssig oder

fest vorliegt und welche Granulierhilfsmittel, Füllstoffe usw. verwendet werden.

Daneben enthalten die genannten Wirkstofformulierungen gegebenenfallS die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser.

Staubförmige und granulierte Zübereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z. B. zwischen 0,005 und 10,0 kg/ha oder mehr Aktivsubstanz, vorzugsweise liegt sie jedoch zwischen 0,01 und 5 kg/ha.

Die erfindungsgemäßen Wirkstoffe können in ihren handelsüblichen Formulierungen entweder allein oder in Kombination mit weiteren, literaturbekannten Fungiziden angewendet werden.

Als literaturbekannte Fungizide, die erfindungsgemäß mit den Verbindungen der Formel I kombiniert werden können, kommen Anilazine, Benalaxyl, Benodanil, Benomyl, Binapacryl, Bitertanol, Buthiobat, Captafol, Captan, Carbendazim, Carboxin, CGD-94240 F, Chlobenzthiazone, Chlorthalonil, Cymoxanil, Cyproconazole, Cyprofuram, Dichlofluanid, Dichlomezin, Diclobutrazol, Diethofencarb, Difluconazole, Dimethirimol, Dimethomorph, Diniconazole, Dinocap, Dithianon, Dodemorph, Dodine, Edifenfos, Ethirimol, Etridiazol, Fenarimol, Fenfuram, Fenpiclonil, Fenpropidin, Fenpropomorph, Fentinacetate, Fentinhydroxide, Fluaziram, Fluobenzimine, Fluorimide, Flusilazole, Flutolanil, Flutriafol, Folpet, Fosetylaluminium, Fuberidazole, Furalaxyl, Furmecyclox, Guazatine, Hexaconazole, Imazalil, Iprobenfos, Iprodione, Isoprothiolane, Kupferverbindungen wie Cu-oxychlorid, Oxine-Cu, Cu-oxide, Mancozeb, Maneb, Mepronil, Metalaxyl, Methasulfocarb, Methfuroxam, Myclobutanil, Nabam, Nitrothalidopropyl, Nuarimol, Ofurace, Oxadixyl, Oxycarboxin, Penconazol, Pencycuron, PP 969, Probenazole, Probineb, Prochloraz, Procymidon, Propamocarb, Propiconazol, Prothiocarb, Pyracarbolid, Pyrifenox, Pyroquilon, Rabenzazole, Schwefel, Tebuconazole, Thiabendazole, Thiofanatemethyl, Thiram, Tolclofos-methyl, Tolylfluanid, Triadimefon, Triadimenol, Tricyclazole, Tridemorph, Triflumizol, Triforine, Vinchlozolin, Zineb, Natrium-dodecylsulfonat, Natrium-dodecylsulfat, Natrium-C13/C15-alkoholethersulfonat, Natrium-cetostearylphosphatester, Dioctylnatriumsulfosuccinat, Natrium-isopropylnaphthalinsulfonat, Natrium-methylenbisnaphthalinsulfonat, Cetyl-trimethyl-ammoniumchlorid, Salze von langkettigen primären, sekundären oder tertiären Aminen, Alkyl-propylenamine, Lauryl-pyrimidiniumbromid, ethoxilierte quarternierte Fettamine, Alkyl-dimethyl-benzyl-ammoniumchlorid und 1-Hydroxyethyl-2-alkyl-imidazolin in Frage.

Die oben genannten Kombinationspartner stellen bekannte Wirkstoffe dar, die zum großen Teil in C. R. Worthing, S. B. Walker, The Pesticide Manual, 7. Auflage (1983), British Crop Protection Council,beschrieben sind.

Darüber hinaus können die erfindungsgemäßen Wirkstoffe, insbesondere die der aufgeführten Beispiele, in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen.

Der Wirkstoffgehalt der aus den handelsüblichen Formulierungen bereiteten Anwendungsformen kann in weiten Bereichen variieren, die Wirkstoffkonzentration der Anwendungsformen kann von 0,0001 bis zu 100 Gew.-%

Wirkstoff, vorzugsweise zwischen 0,001 und 1 Gew.-% liegen. Die Anwendung geschieht in einer den Anwendungsformen angepaßten üblichen Weisen.

Nachfolgende Beispiele dienen zur Erläuterung der Erfindung.

A) Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.

b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat stellt man her, indem man 40 Gew.-Teile Wirkstoff mit 7 Gew.-Teilen eines sulfobernsteinsäurehalbesters, 2 Gew.-Teilen eines Ligninsulfonsäure-Natriumsalzes und 51 Gew.-Teilen Wasser mischt und in einer Reibkugelmühle auf eine Feinheit von unter 5 Mikron vermahlt.

d) Ein emulgierbares Konzentrat läßt sich herstellen aus 15 Gew.-Teilen Wirkstoff, 75 Gew.-Teilen Cyclohexanon als Lösungsmittel und 10 Gew.-Teilen oxethyliertem Nonylphenol (10 AeO) als Emulgator.

e) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten
Trägermaterials ca.
95 % des fertigen Granulats.

B) Chemische Beispiele

Beispiel A

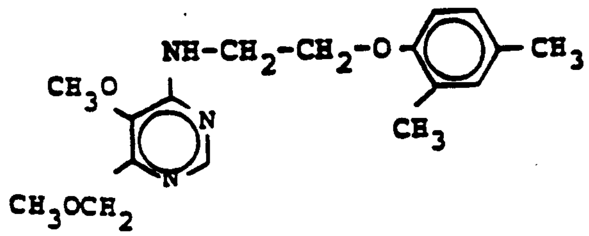

Eine Mischung aus 3,8 g (0,02 mol) 4-Chlor-5-methoxy-6-methoxymethylpyrimidin und 6,6 g (0,04 Mol) 2-(2,4-Dimethyl-phenoxy)ethylamin wurde 2 Stunden auf 100° erwärmt. Nach Abkühlen wurde mit einem Wasser/Methylenchlorid-Gemisch aufgenommen, die organische Phase mehrere Male mit Wasser gewaschen, getrocknet und eingeengt. Zur Reinigung wurde das Rohprodukt mit Ethylacetat an Kieselgel chromatographiert.
Ausbeute: 4,1 g (64,6 % d. Th.) 5-Methoxy-6-methoxymethyl-4-[2-(2,4-dimethylphenoxy)-ethylamino]-pyrimidin vom Schmelzpunkt 49-50° C.

Herstellung der Ausgangsverbindung 4-Chlor-5-methoxy-6-methoxymethylpyrimidin

85,0 g (0,5 Mol) 4-Hydroxy-5-methoxy-6-methoxymethyl-pyrimidin wurden in 600 ml Dichlorethan aufgeschlämmt und nach Zugabe von 50,5 g Triethylamin 260 g (1,7 Mol) Phosphoroxychlorid ohne Kühlung zugetropft. Man rührte 3 Stunden bei 80° C, goß auf Eiswasser, neutralisierte durch Zugabe von festem Soda, trennte die organische Phase ab und rührte die Wasserphase mehrmals mit Methylenchlorid aus. Die vereinigten organischen Phasen wurden getrocknet und eingeengt. Der Rückstand wurde im Wasserstrahlvakuum destilliert. Man erhielt 66,1 g (70% d. Th.) eines gelben Öls. Kp 93-96° C/0,1 mm.

Herstellung von 4-Hydroxy-5-methoxy-6-methoxymethyl-pyrimidin

Zu 750 ml auf 80° C erwärmtes 12%iges Wasserstoffperoxid wurden portionsweise 101 g (0,5 Mol) 4-Hydroxy-2-mercapto-5-methoxy-6-methoxymethyl-pyrimidin so gegeben, daß sich die Temperatur bei 80° C hielt. Anschließend wurde 2 Stunden bei 80° C nachgerührt. Nach Abkühlen wurde festes Natriumdisulfit zugegeben bis Kaliumjodid-Stärkepapier keine Reaktion auf Peroxid mehr ergab, dann durch Zugabe von Kaliumcarbonat neutralisiert. Nach Abdestillieren des Wassers wurde der feste Rückstand mit Methylenchlorid, dem etwas Ethanol zugesetzt war, aufgeschlämmt und abgesaugt. Nach Einengen des Filtrats wurden 63 g (70% d. Th.) Produkt vom Schmelzpunkt 141-143° C erhalten.

Herstellung von 4-Hydroxy-2-mercapto-5-methoxy-6-methoxymethyl-pyrimidin

Man legte 352,0 g (2,0 Mol) 2,4-Dimethoxyacetessigsäuredimethylester und 152,2 g Thioharnstoff in 1,5 l Ethanol vor, gab 360,0 g (2,0 Mol) 30%ige Natriummethylatlösung zu und erhitzte 6 Stunden unter Rückfluß. Nach Abdestillieren des Lösungsmittels wurde der Rückstand in Wasser aufgenommen und mit konzentrierter Salzsäure auf pH 5 gebracht. Das ausgefallene Produkt wurde abfiltriert. Man erhielt 300,0 g (74,2% d. Th.) gelbe Kristalle vom Schmelzpunkt 187-189° C.

Beispiel B

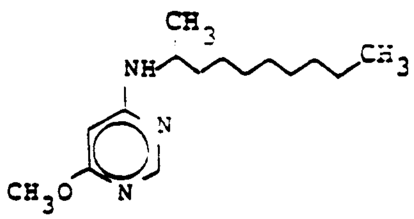

7,2 g (0,05 Mol) 4-Chlor-6-methoxy-pyrimidin und 15,7 g (0,1 Mol) 2-Decylamin wurden 2 Stunden auf 100° C erwärmt. Man arbeitete mit einem Wasser/Methylenchlorid-Gemisch auf und erhielt nach chromatographischer Reinigung (Kieselgel/Essigester) 8,0 g (58,8 % d. Th.) 4-(2-Decylamino)-6-methoxypyrimidin als gelbes Öl.

Herstellung von 4-Chlor-6-methoxypyrimidin

22,4 g (0,15 Mol) 4,6-Dichlorpyrimidin wurden in 250 ml Methanol vorgelegt. Bei 0°C wurden 27 g 30%ige Natriummethylat-Lösung zugetropft. Nach Einengen wurde mit einem Wasser/Methylenchlorid-Gemisch aufgearbeitet. Man erhielt 15,0 g (69,2% d. Th.) gelbe Kristalle; Fp 32-34°C.

Beispiel C

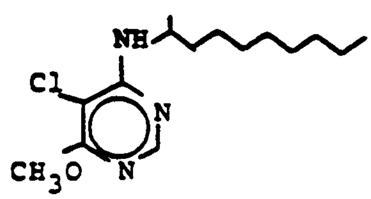

4,0 g (0,015 Mol) 4-(2-Decylamino)-6-methoxy-pyrimidin wurden mit 2,2 g (0,016 Mol) N-Chlorsuccinimid in 25 ml Chloroform 1 Stunde unter Rückfluß erhitzt. Nach Abkühlen wurde mit 2N Natronlauge ausgerührt und die organische Phase getrocknet und eingeengt. Man erhielt 3,8 g (84,3% d. Th.) 5-Chlor-4-(2-decylamino)-6-methoxypyrimidin als gelbes Öl.

Beispiel D

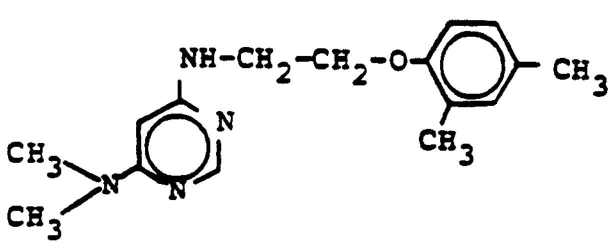

Zu einer lösung von 7,45 g (0,05 Mol) 4,6-Dichlorpyrimidin in 100 ml Ethanol wurde bei 20°C eine Lösung von 8,3 g (0,05 Mol) 2-(2,4-Dimethylphenoxy)ethylamin und 5,1 g (0,05 Mol) Triethylamin in 50 ml Ethanol zugetropft. Das Gemisch wurde 6 h unter Rückfluß erhitzt und nach Abkühlen auf Raumtemperatur das Dimethylamin bis zur Sättigung eingeengt. Nach nochmalige Stehenlassen über Nacht wurde das Lösungsmittel abdestilliert, der Rückstand in einem Wasser/Methylenchlorid-Gemisch aufgenommen, die

organische Phase getrocknet und eingeengt. Nach chromatographischer Reinigung des Rohproduktes (Kieselgel/Ethylacetat) erhielt man 6,0 g (41,9% d. Th.) 6-Dimethylamino-4-[2-(2,4-dimethylphenoxy)-ethylamino]-pyrimidin vom Schmelzpunkt 115-117 ° C.

Beispiel E

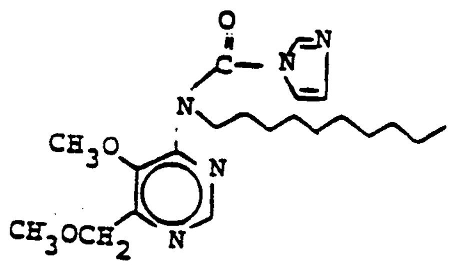

9,3 g (0,03 Mol) 4-(Decylamino)-5-methoxy-6-methoxymethyl (hergestellt analog Beispiel A aus 1-Decylamin und 4-Chlor-5-methoxy-6-methoxymethyl-pyrimidin) und 3,6 g (0,036 Mol) Triethylamin in 20 ml Toluol wurden bei 5 ° C zu einer Lösung von 4,0 g (0,02 Mol) Diphosgen in 60 ml Toluol getropft. Man rührte 6 Stunden bei Raumtemperatur. Nach Zugabe von 50 ml Wasser wurde weitere 2 Stunden bei Raumtemperatur gerührt. Die organische Phase wurde getrocknet und eingeengt. Man erhielt 11,8 g braunes Öl (Carbamoylchlorid). Dieses Öl wurde in Toluol gelöst und bei Raumtemperatur zu einer Lösung von 3,5 g Imidazol und 5 g Triethylamin in 50 ml Toluol getropft. Man rührte 2 Stunden bei Raumtemperatur nach, filtrierte vom Triethylamin-Hydrochlorid ab und engte das Filtrat ein. Nach chromatographischer Reinigung (Kieselgel/Ethylacetat) wurden 10,4 g (85,9% d. Th.) N-Decyl-N-(imidazol-1-ylcarbonyl)-5-methoxy-6-methoxymethyl-pyrimidin-4-amin als gelbes Öl erhalten.

Beispiel F

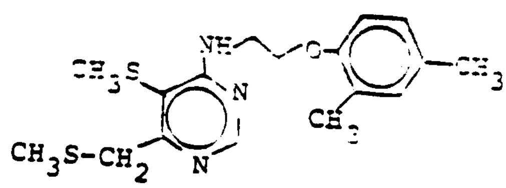

4,4g (0,02 Mol) 4-Chlor-5-methylthio-6-methylthiomethyl-pyrimidin und 7,4 g (0,045 Mol) 2-(2,4-Dimethylphenoxy)-ethylamin wurden 2 Stunden auf 100 ° C erwärmt. Man arbeitete mit einem Wasser/Methylenchlorid-Gemisch auf, trocknete die organische Phase und engte ein. Das Rohprodukt wurde durch Chromatographie an Kieselgel (Laufmittel: Ethylacetat/Methanol (19:1)) gereinigt. Man erhielt 3,3 g (47,2 % d. Th.) 4-[2-(2,4-Dimethylphenoxy)-ethylamino)-5-methylthio-6-methylthiomethyl-pyrimidin als gelbes Öl.

Herstellung der Ausgangsverbindung 4-Chlor-5-methylthio-6-methylthiomethyl-pyrimidin

Die Herstellung erfolgte analog der Darstellung der Ausgangsverbindung 4-Chlor-5-methoxy-6-methoxymethyl-pyrimidin aus 42 g 4-Hydroxy-5-methylthio-6-methylthiomethyl-pyrimidin.
Ausbeute: 29,5 g (63,6% d. Th.) Rohprodukt (wurde ohne weitere Reinigung eingesetzt).

Herstellung von 4-Hydroxy-5-methylthio-6-methylthiomethyl-pyrimidin

Zu einer Lösung von 52 g (0,5 Mol) Formamidinacetat und 104 g (0,5 Mol) 2,4-Bis-(mercaptomethyl)-acetessigsäuremethylester in 400 ml Methanol tropfte man bei -10 ° C 180 g 30%ige Natriummethanolat-Lösung. Es wurde 10 Stunden bei Raumtemperatur nachgerührt, abfiltriert und eingeengt. Der harzige

Rückstand wurde in ca. 150 ml eines 4:1 Gemischs aus Ethylacetat und Methanol aufgenommen. Beim Stehenlassen kristallisierte 42 g farbloses Produkt (41,6 g d. Th.) aus.

Beispiel G

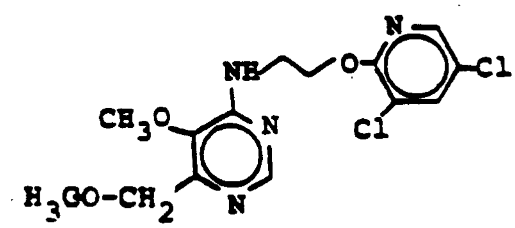

Analog Beispiel A wurden 3,8 g (0,02 Mol) 4-Chlor-5-methoxy-6-methoxymethyl-pyrimidin und 9,3 g (0,045 Mol) 2-(3,5-Dichlor-pyridyl-2-oxy)-ethylamin umgesetzt. Nach chromatographischer Reinigung (Kieseigel/Ethylacetat) verblieben 4,5 g (62,6 % d. Th.) 4-[2-(3,5-Dichlor-pyridyl-2-oxy)ethylamino]-5-methoxy-6-methoxymethyl-pyrimidin, als braunes Öl, das allmählich erstarrte. Fp: 65-66 ° C.

Herstellung von 2-(3,5-Dichlor-pyridyl-2-oxy)-ethylamin

20,0 g (0,5 Mol) 60%iges Natriumhydrid (Dispersion in Mineralöl) wurden in 500 ml trockenen Dimethylacetamid auf 50 ° C erwärmt. Hierzu tropfte man 30,6 g (0,5 Mol) 2-Aminoethanol und rührte bei 50 ° C bis zum Ende der Gasentwicklung. Nach Abkühlen auf Raumtemperatur gab man unter gelegentlicher Kühlung 91,2 g (0,5 Mol) 2,3,5-Trichlorpyridin portionsweise zu. Anschließend wurde 4 Stunden bei 80 ° C nachgerührt. Nach Aufarbeitung mit einem Wasser/Methylenchlorid-Gemisch und chromatographischer Reinigung des Rohproduktes (Kieseigel/Ethylacetat/Methanol 3:1) erhielt man 40,0 g (38,6%) eines gelben Öls.

Weitere Beispiele finden sich in der folgenden Tabelle A. Die in der Tabelle aufgeführten Reste $R^1$ bis $R^5$ und Q entsprechen den Symbolen in der Formel I

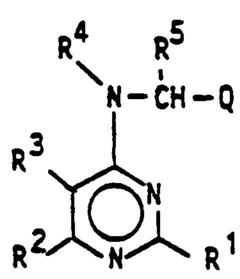

(I).

Tabelle B beinhaltet Verbindungen der Formel

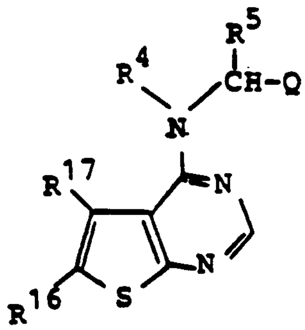

und Tabelle C beinhaltet Verbindungen der Formel

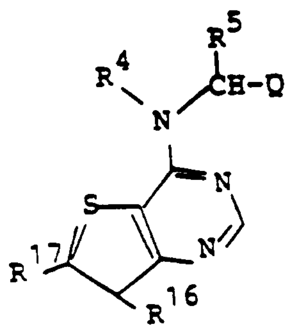

,

die sich beide von Formel I herleiten.

Tabelle A

| Beisp. Nr. | $R^1$ | $R^2$ | $R^3$ | $R^4$ | $R^5$ | Q | Fp [°C] |
|---|---|---|---|---|---|---|---|
| 1 | H | $CH_2OCH_3$ | $OCH_3$ | H | H | 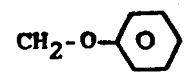 | Öl |
| 2 | H | $CH_2OCH_3$ | $OCH_3$ | H | H | 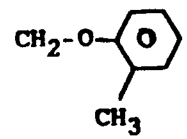 | Öl |
| | | | | | | | Öl |
| 3 | H | $CH_2OCH_3$ | $OCH_3$ | H | H | 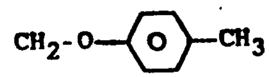 | Öl |
| 4 | H | $CH_2OCH_3$ | $OCH_3$ | H | H | 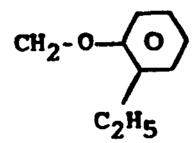 | Öl |
| 5 | H | $CH_2OCH_3$ | $OCH_3$ | H | H | 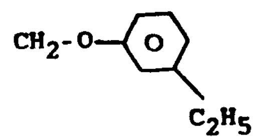 | 61-63 |

| Beisp. Nr. | $R^1$ | $R^2$ | $R^3$ | $R^4$ | $R^5$ | Q | Fp [°C] |
|---|---|---|---|---|---|---|---|
| 6 | H | $CH_2OCH_3$ | $OCH_3$ | H | H | 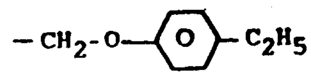 | 64-66 |
| 7 | H | $CH_2OCH_3$ | $OCH_3$ | H | H | 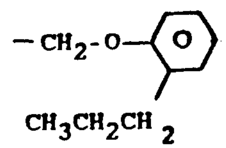 | Öl |
| 8 | H | $CH_2OCH_3$ | $OCH_3$ | H | H | 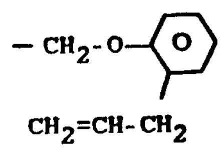 | 65-66 |
| 9 | H | $CH_2OCH_3$ | $OCH_3$ | H | H | 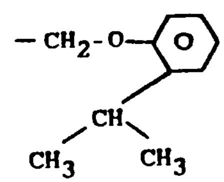 | Öl |

| Beisp. Nr. | $R^1$ | $R^2$ | $R^3$ | $R^4$ | $R^5$ | Q | Fp [°C] |
|---|---|---|---|---|---|---|---|
| 10 | H | $CH_2OCH_3$ | $OCH_3$ | H | H | 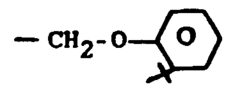 | Öl |
| 11 | H | $CH_2OCH_3$ | $OCH_3$ | H | H | 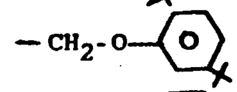 | Öl |
| 12 | H | $CH_2OCH_3$ | $OCH_3$ | H | H | 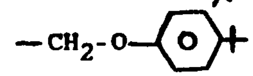 | 77-79 |
| 13 | H | $CH_2OCH_3$ | $OCH_3$ | H | H | 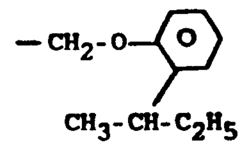 | Öl |
| 14 | H | $CH_2OCH_3$ | $OCH_3$ | H | H | 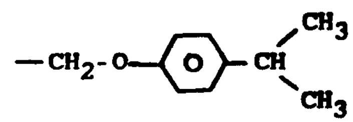 | Öl |
| 15 | H | $CH_2OCH_3$ | $OCH_3$ | H | H | 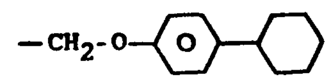 | Öl |

| Beisp. Nr. | $R^1$ | $R^2$ | $R^3$ | $R^4$ | $R^5$ | Q | Fp [°C] |
|---|---|---|---|---|---|---|---|
| 16 | H | $CH_2OCH_3$ | $OCH_3$ | H | H | 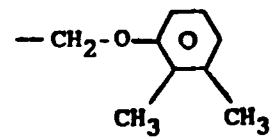 | Öl |
| 17 | H | $CH_2OCH_3$ | H | H | H | 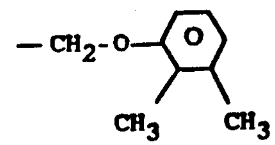 | 100-102 |
| 18 | H | $CH_2OCH_3$ | $C_2H_5$ | H | H | 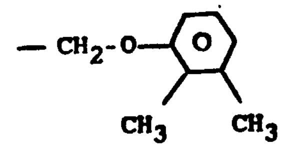 | 90-92 |
| 19 | $SCH_3$ | $CH_2OCH_3$ | $OCH_3$ | H | H | 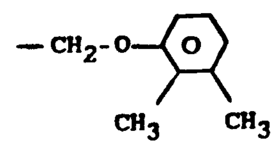 | Öl |

| Beisp. Nr. | $R^1$ | $R^2$ | $R^3$ | $R^4$ | $R^5$ | Q | Fp [°C] |
|---|---|---|---|---|---|---|---|
| 20 | H | $CH_2OC_2H_5$ | $OC_2H_5$ | H | H | 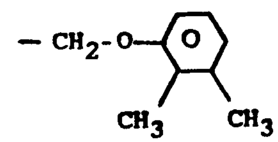 | Öl |
| 21 | H | $CH_2OCH_3$ | $OCH_3$ | H | H | 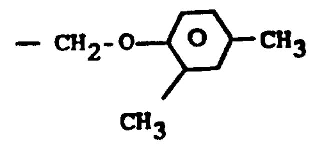 | 49-50 |
| 22 | H | $CH_2SCH_3$ | $SCH_3$ | H | H | 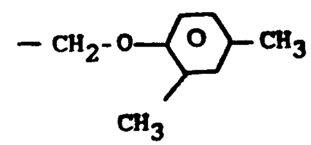 | Öl |
| 23 | $CH_3$ | $CH_2OCH_3$ | $OCH_3$ | H | H | 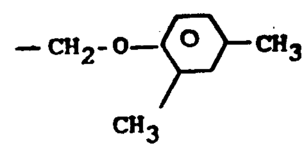 | 87-89 |

| Beisp. Nr. | $R^1$ | $R^2$ | $R^3$ | $R^4$ | $R^5$ | Q | Fp [°C] |
|---|---|---|---|---|---|---|---|
| 24 | Cl | $CH_2OCH_3$ | $OCH_3$ | H | H | 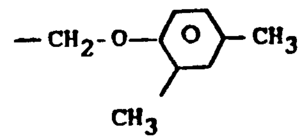 | |
| 25 | H | $CH_2OCH_3$ | $OCH_3$ | H | $CH_3$ | 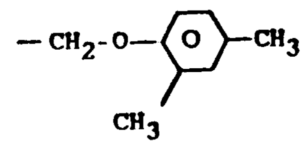 | Öl |
| 26 | H | $CH_2OCH_3$ | $OCH_3$ | H | H | 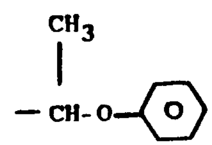 | 88-90 |
| 27 | H | $CH_2OCH_3$ | $OCH_3$ | H | H | 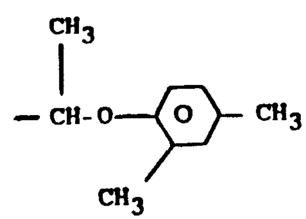 | flüssig |

| Beisp. Nr. | $R^1$ | $R^2$ | $R^3$ | $R^4$ | $R^5$ | Q | Fp [°C] |
|---|---|---|---|---|---|---|---|
| 28 | H | $CH_2OCH_3$ | $OCH_3$ | H | $CH_3$ | 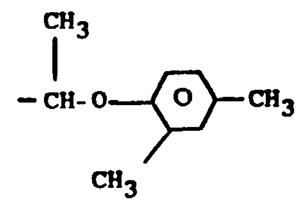 | Öl |
| 29 | H | $CH_2OCH_3$ | $OCH_3$ | H | H | 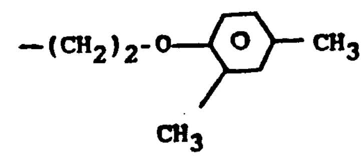 | Öl |
| 30 | H | $CH_2OCH_3$ | $OCH_3$ | H | H | 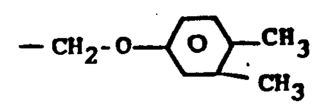 | 60-61 |

| Beisp. Nr. | $R^1$ | $R^2$ | $R^3$ | $R^4$ | $R^5$ | Q | Fp [°C] |
|---|---|---|---|---|---|---|---|
| 31 | H | $CH_2OCH_3$ | $OCH_3$ | H | H | 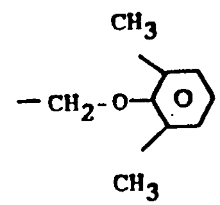 | Öl |
| 32 | H | $CH_2OCH_3$ | $OCH_3$ | H | H | 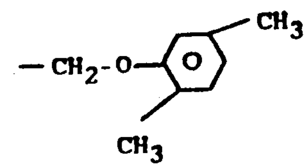 | Öl |
| 33 | H | $CH_2OCH_3$ | $OCH_3$ | H | H | 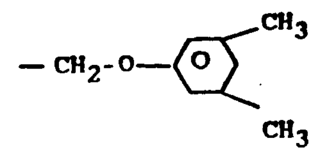 | 88-89 |

| Beisp. Nr. | $R^1$ | $R^2$ | $R^3$ | $R^4$ | $R^5$ | Q | Fp [°C] |
|---|---|---|---|---|---|---|---|
| 34 | H | $CH_2OCH_3$ | $OCH_3$ | H | H | 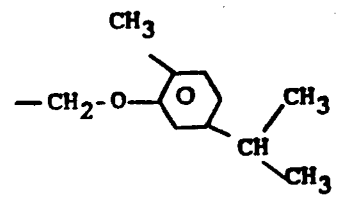 | Öl |
| 35 | H | $CH_2OCH_3$ | $OCH_3$ | H | H | 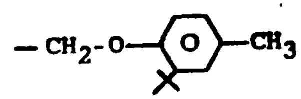 | Öl |
| 36 | H | $CH_2OCH_3$ | $OCH_3$ | H | H | 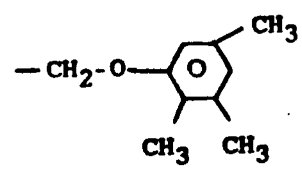 | 82-83 |
| 37 | H | $CH_2OCH_3$ | $OCH_3$ | H | H | 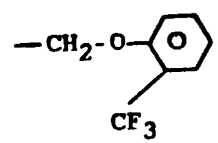 | 79-80 |

| Beisp. Nr. | $R^1$ | $R^2$ | $R^3$ | $R^4$ | $R^5$ | Q | Fp [°C] |
|---|---|---|---|---|---|---|---|
| 38 | H | $CH_2OCH_3$ | $OCH_3$ | H | H | 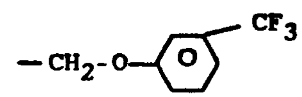 | 80-82 |
| 39 | H | $CH_2OCH_3$ | $OCH_3$ | H | H | 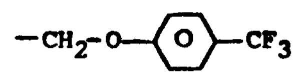 | 96-97 |
| 40 | H | $CH_2OCH_3$ | $OCH_3$ | H | H | 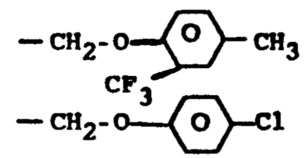 | Öl |
| 41 | H | $CH_2OCH_3$ | $OCH_3$ | H | H | | Öl |
| 42 | H | $CH_2OCH_3$ | $OCH_3$ | H | H | 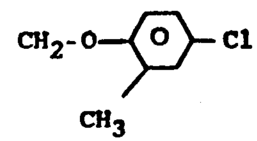 | 76-78 |
| 43 | H | $CH_2OCH_3$ | $OCH_3$ | H | H | 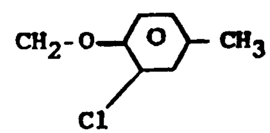 | Öl |

| Beisp. Nr. | $R^1$ | $R^2$ | $R^3$ | $R^4$ | $R^5$ | Q | Fp [°C] |
|---|---|---|---|---|---|---|---|
| 44 | H | $CH_2OCH_3$ | $OCH_3$ | H | H | 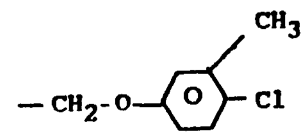 | 79-81 |
| 45 | H | $CH_2OCH_3$ | $OCH_3$ | H | H | 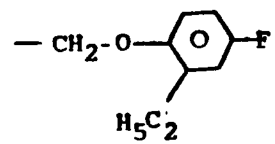 | öl |
| 46 | H | $CH_2OCH_3$ | $OCH_3$ | H | H | 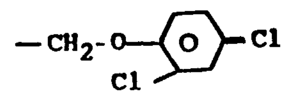 | öl |
| 47 | H | $CH_2OCH_3$ | $OCH_3$ | H | H | 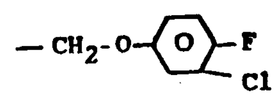 | 100-101 |
| 48 | H | $CH_2OCH_3$ | $OCH_3$ | H | H | 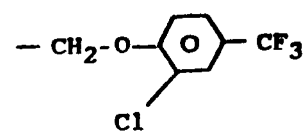 | öl |
| 49 | H | $CH_2OCH_3$ | $OCH_3$ | H | H | 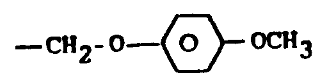 | |

| Beisp. Nr. | $R^1$ | $R^2$ | $R^3$ | $R^4$ | $R^5$ | Q | Fp [°C] |
|---|---|---|---|---|---|---|---|
| 50 | H | $CH_2OCH_3$ | $OCH_3$ | H | H | 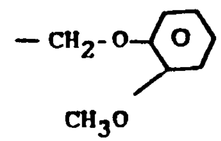 | |
| 51 | H | $CH_2OCH_3$ | $OCH_3$ | H | H | 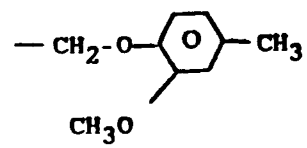 | |
| 52 | H | $CH_2OCH_3$ | $OCH_3$ | H | H | 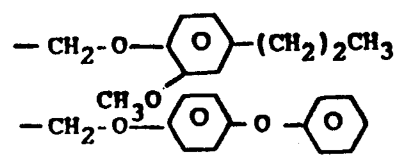 | 53-55 |
| 53 | H | $CH_2OCH_3$ | $OCH_3$ | H | H | | Harz |
| 54 | H | $CH_2OCH_3$ | $OCH_3$ | H | H | 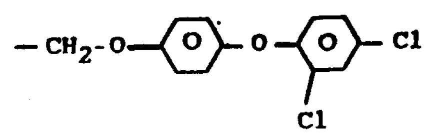 | |

| Beisp. Nr. | $R^1$ | $R^2$ | $R^3$ | $R^4$ | $R^5$ | Q | Fp [°C] |
|---|---|---|---|---|---|---|---|
| 55 | H | $CH_2OCH_3$ | $OCH_3$ | H | H | 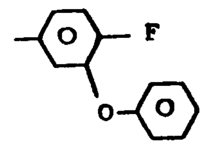 | Harz |
| 56 | H | $CH_2OCH_3$ | $OCH_3$ | H | $C_2H_5$ | 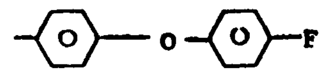 | Harz |
| 57 | H | $CH_2SCH_3$ | $SCH_3$ | H | $C_2H_5$ | 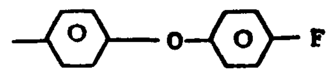 | |
| 58 | H | $CH_2OCH_3$ | $OCH_3$ | H | 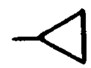 | 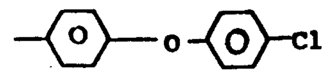 | Harz |
| 59 | H | $CH_2OCH_3$ | $OCH_3$ | H | H | $(CH_2)_8CH_3$ | Öl |
| 60 | H | $CH_2OCH_3$ | $OCH_3$ | 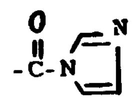 | H | $(CH_2)_8CH_3$ | Öl |
| 61 | H | $CH_2OCH_3$ | $OCH_3$ | H | $CH_3$ | $(CH_2)_8CH_3$ | Öl |

| Beisp. Nr. | $R^1$ | $R^2$ | $R^3$ | $R^4$ | $R^5$ | Q | Fp [°C] |
|---|---|---|---|---|---|---|---|
| 62 | H | $CH_2OCH_3$ | $OCH_3$ | H | $CH_3$ | $(CH_2)_7CH_3$ | Öl |
| 63 | H | $CH_2OCH_3$ | $OCH_3$ | 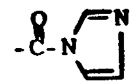 | $CH_3$ | $(CH_2)_7CH_3$ | Öl |
| 64 | H | $CH_2SCH_3$ | $SCH_3$ | H | $CH_3$ | $(CH_2)_7CH_3$ | Öl |
| 65 | H | $CH_3$ | $SCH_3$ | H | $CH_3$ | $(CH_2)_7CH_3$ | Öl |
| 66 | H | $OCH_3$ | H | H | $CH_3$ | $(CH_2)_7CH_3$ | Öl |
| 67 | H | $OCH_3$ | Cl | H | $CH_3$ | $(CH_2)_7CH_3$ | Öl |
| 68 | H | $OCH_3$ | Br | H | $CH_3$ | $(CH_2)_7CH_3$ | Öl |
| 69 | H | $OCH_3$ | $C_2H_5$ | H | $CH_3$ | $(CH_2)_7CH_3$ | |

| Beisp. Nr. | $R^1$ | $R^2$ | $R^3$ | $R^4$ | $R^5$ | Q | Fp [°C] |
|---|---|---|---|---|---|---|---|
| 70 | H | $OCH_3$ | $OCH_3$ | H | $CH_3$ | $(CH_2)_7CH_3$ | Öl |
| 71 | H | $OC_2H_5$ | H | H | $CH_3$ | $(CH_2)_7CH_3$ | Öl |
| 72 | H | $OC_2H_5$ | H | H | $CH_3$ | $(CH_2)_8CH_3$ | Öl |
| 73 | H | $OCH_2CF_3$ | H | H | $CH_3$ | $(CH_2)_8CH_3$ | Öl |
| 74 | H | $OCH_2CF_3$ | H | H | $CH_3$ | $(CH_2)_7CH_3$ | Öl |
| 75 | H | $OCH_2CF_3$ | Cl | H | $CH_3$ | $(CH_2)_7CH_3$ | Öl |
| 76 | H | $N(CH_3)_2$ | H | H | $CH_3$ | $(CH_2)_7CH_3$ | Öl |
| 77 | H | $N(CH_3)_2$ | Cl | H | $CH_3$ | $(CH_2)_7CH_3$ | |
| 78 | H | $N(CH_3)_2$ | Br | H | $CH_3$ | $(CH_2)_7CH_3$ | |

| Beisp. Nr. | $R^1$ | $R^2$ | $R^3$ | $R^4$ | $R^5$ | Q | Fp [°C] |
|---|---|---|---|---|---|---|---|
| 79 | H | $N(CH_3)_2$ | $OCH_3$ | H | $CH_3$ | $(CH_2)_7CH_3$ | |
| 80 | H | $N(CH_3)_2$ | H | H | H | 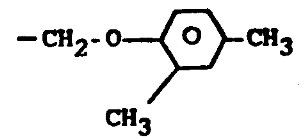 | 115-117 |
| 81 | H | $N(CH_3)_2$ | Cl | H | H | 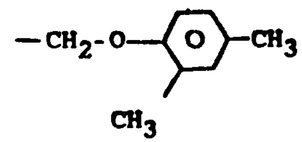 | 82-83 |
| 82 | H | $N(CH_3)_2$ | Br | H | H | 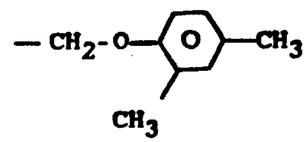 | Harz |
| 83 | H | Cl | H | H | H | 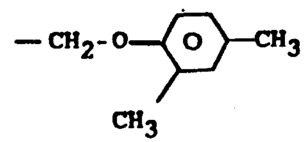 | 99-101 |

| Beisp. Nr. | $R^1$ | $R^2$ | $R^3$ | $R^4$ | $R^5$ | Q | Fp [°C] |
|---|---|---|---|---|---|---|---|
| 84 | H | $OCH_3$ | $OCH_3$ | H | H | 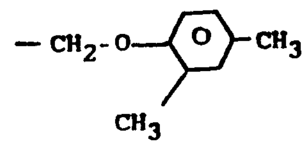 | Harz |
| 85 | H | $CH_2OCH_3$ | $OCH_3$ | H | H | 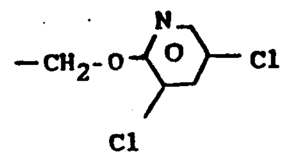 | 69-70 |
| 86 | H | $CH_2OCH_3$ | $OCH_3$ | H | H | 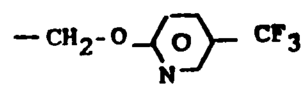 | 111-113 |
| 87 | H | $CH_2OCH_3$ | $OCH_3$ | H | H | 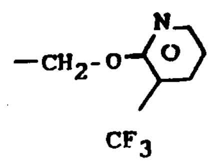 | Öl |

| Beisp. Nr. | $R^1$ | $R^2$ | $R^3$ | $R^4$ | $R^5$ | Q | Fp [°C] |
|---|---|---|---|---|---|---|---|
| 88 | H | $CH_2OCH_3$ | $OCH_3$ | H | H | 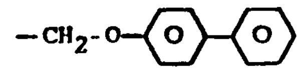 | |
| 89 | H | $CH_2OCH_3$ | $OCH_3$ | H | H | 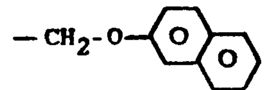 | |
| 90 | H | $CH_2OCH_3$ | $OCH_3$ | H | H | 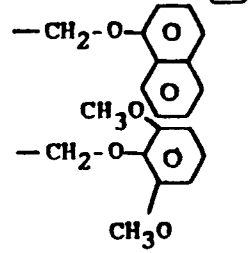 | |
| 91 | H | $CH_2OCH_3$ | $OCH_3$ | H | H | | |
| 92 | H | $OCH_3$ | $NO_2$ | H | H | 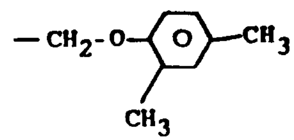 | 101-103 |

| Beisp. Nr. | $R^1$ | $R^2$ | $R^3$ | $R^4$ | $R^5$ | Q | Fp [°C] |
|---|---|---|---|---|---|---|---|
| 93 | H | $OCH_3$ | $NO_2$ | H | $CH_3$ | $(CH_2)_7CH_3$ | Öl |
| 94 | H | $CH_2OCH_3$ | $OCH_3$ | H | H | $(CH_2)_{10}CH_3$ | Öl |
| 95 | H | $CH_2OCH_3$ | $OCH_3$ | H | H | $(CH_2)_9CH_3$ | Öl |
| 96 | H | $CH_2OCH_3$ | $OCH_3$ | H | H | $(CH_2)_7CH_3$ | Öl |
| 97 | H | $CH_2OCH_3$ | $OCH_3$ | H | H | $(CH_2)_6CH_3$ | Öl |
| 98 | H | $CH_2OCH_3$ | $OCH_3$ | H | H | $(CH_2)_5CH_3$ | Öl |
| 99 | H | $CH_2OCH_3$ | $OCH_3$ | H | H | $(CH_2)_4CH_3$ | |
| 100 | H | $CH_2OCH_3$ | $OCH_3$ | H | H | $(CH_2)_3CH_3$ | |

| Beisp. Nr. | $R^1$ | $R^2$ | $R^3$ | $R^4$ | $R^5$ | Q | Fp [°C] |
|---|---|---|---|---|---|---|---|
| 101 | H | $CH_2OCH_3$ | $OCH_3$ | H | $CH_3$ | $(CH_2)_9CH_3$ | Öl |
| 102 | H | $CH_2OCH_3$ | $OCH_3$ | H | $CH_3$ | $(CH_2)_6CH_3$ | Öl |
| 103 | H | $CH_2OCH_3$ | $OCH_3$ | H | $CH_3$ | $(CH_2)_5CH_3$ | Öl |
| 104 | H | $CH_2OCH_3$ | $OCH_3$ | H | $CH_3$ | $(CH_2)_4CH_3$ | |
| 105 | H | $CH_2OCH_3$ | $OCH_3$ | H | $CH_3$ | $(CH_2)_3CH_3$ | |
| 106 | H | $CH_2OCH_3$ | $OCH_3$ | H | $CH_3$ | $(CH_2)_2CH_3$ | |
| 107 | H | $CH_2OCH_3$ | $OCH_3$ | H | $CH_3$ | $(CH_2)_3-CH(CH_3)_2$ | Öl |
| 108 | H | $CH_2OCH_3$ | $OCH_3$ | H | $C_2H_5$ | $(CH_2)_3CH_3$ | Öl |

| Beisp. Nr. | $R^1$ | $R^2$ | $R^3$ | $R^4$ | $R^5$ | Q | Fp [°C] |
|---|---|---|---|---|---|---|---|
| 109 | H | $CH_2OCH_3$ | $OCH_3$ | H | $C_2H_5$ | $(CH_2)_6CH_3$ | Öl |
| 110 | H | $CH_2OCH_3$ | $OCH_3$ | H | n-$C_3H_7$ | $(CH_2)_5CH_3$ | Öl |
| 111 | H | $CH_2OCH_3$ | $OCH_3$ | H | i-$C_3H_7$ | $(CH_2)_7CH_3$ | Öl |
| 112 | H | $CH_2OHC_3$ | $OCH_3$ | H | 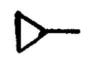 | $(CH_2)_7CH_3$ | Öl |
| 113 | H | $CH_2OCH_3$ | $OCH_3$ | H | $CH_3$ | $(CH_2)_2$ 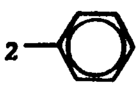 | Öl |
| 114 | H | $CH_2OCH_3$ | $OCH_3$ | H | $CH_3$ | $(CH_2)_2-OCH_2$ 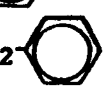 | Öl |
| 115 | H | $CH_2OCH_3$ | $OCH_3$ | H | 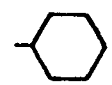 | $CH_3$ R-Form | Öl |
| 116 | H | $CH_2OCH_3$ | $OCH_3$ | H | 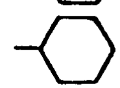 | $CH_3$ S-Form | Öl |

| Beisp. Nr. | $R^1$ | $R^2$ | $R^3$ | $R^4$ | $R^5$ | Q | Fp [°C] |
|---|---|---|---|---|---|---|---|
| 117 | H | $CH_2OCH_3$ | $OCH_3$ | H | H | 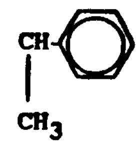 | Öl |
| 118 | H | $CH_2OCH_3$ | $OCH_3$ | H | H | 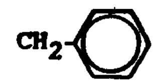 | Öl |
| 119 | H | $CH_2OCH_3$ | $OCH_3$ | H | H | 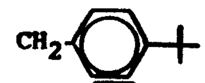 | 87-88 |
| 120 | H | $CH_2OCH_3$ | $OCH_3$ | H | $CH_3$ | 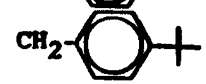 | |
| 121 | H | $CH_2OCH_3$ | $OCH_3$ | H | H | 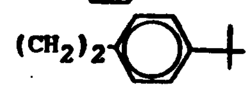 | Öl |
| 122 | H | $CH_2OCH_3$ | $OCH_3$ | H | $CH_3$ | 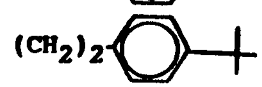 | Öl |
| 123 | H | $OCH_3$ | $OCH_3$ | H | H | $(CH_2)_{10}CH_3$ | |
| 124 | H | $OCH_3$ | $OCH_3$ | H | H | $(CH_2)_9CH_3$ | |

| Beisp. Nr. | $R^1$ | $R^2$ | $R^3$ | $R^4$ | $R^5$ | Q | Fp [°C] |
|---|---|---|---|---|---|---|---|
| 125 | H | $OCH_3$ | $OCH_3$ | H | H | $(CH_2)_7CH_3$ | |
| 126 | H | $OCH_3$ | $OCH_3$ | H | H | $(CH_2)_6CH_3$ | |
| 127 | H | $OCH_3$ | $OCH_3$ | H | H | $(CH_2)_7CH_3$ | |
| 128 | H | $OCH_3$ | $OCH_3$ | H | H | $(CH_2)_6CH_3$ | |
| 129 | H | $OCH_3$ | $OCH_3$ | H | H | $(CH_2)_5CH_3$ | |
| 130 | H | $OCH_3$ | $OCH_3$ | H | H | $(CH_2)_4CH_3$ | |
| 131 | H | $OCH_3$ | $OCH_3$ | H | H | $(CH_2)_3CH_3$ | |
| 132 | H | $OCH_3$ | $OCH_3$ | H | $CH_3$ | $(CH_2)_9CH_3$ | |
| 133 | H | $OCH_3$ | $OCH_3$ | H | $CH_3$ | $(CH_2)_8CH_3$ | Öl |

| Beisp. Nr. | $R^1$ | $R^2$ | $R^3$ | $R^4$ | $R^5$ | Q | Fp [°C] |
|---|---|---|---|---|---|---|---|
| 134 | H | $OCH_3$ | $OCH_3$ | H | $CH_3$ | $(CH_2)_6CH_3$ | |
| 135 | H | $OCH_3$ | $OCH_3$ | H | $CH_3$ | $(CH_2)_5CH_3$ | |
| 136 | H | $OCH_3$ | $OCH_3$ | H | $CH_3$ | $(CH_2)_4CH_3$ | |
| 137 | H | $OCH_3$ | $OCH_3$ | H | $CH_3$ | $(CH_2)_3CH_3$ | |
| 138 | H | $OCH_3$ | $OCH_3$ | H | $CH_3$ | $(CH_2)_2CH_3$ | |
| 139 | H | $OCH_3$ | $OCH_3$ | H | 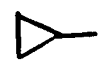 | $(CH_2)_7CH_3$ | |
| 140 | H | $CH_2OCH_3$ | $OCH_3$ | H | H | 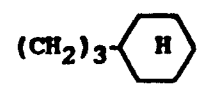 | Öl |
| 141 | H | $CH_2OCH_3$ | $OCH_3$ | H | $C_2H_5$ | 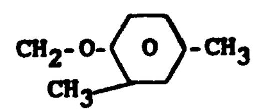 | |

| Beisp. Nr. | $R^1$ | $R^2$ | $R^3$ | $R^4$ | $R^5$ | Q | Fp [°C] |
|---|---|---|---|---|---|---|---|
| 142 | H | $CH_2OCH_3$ | $OCH_3$ | H | 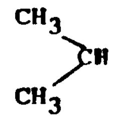 | 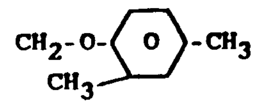 | |
| 143 | $CH_3$ | $CH_2OCH_3$ | $OCH_3$ | H | $CH_3$ | $(CH_2)_7CH_3$ | Öl |
| 144 | H | $CH_2OCH_3$ | $OCH_3$ | H | H | 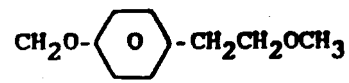 | Öl |
| 145 | H | $CH_2OCH_3$ | $OCH_3$ | H | H | 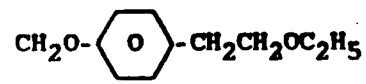 | Öl |
| 146 | H | $CH_2OCH_3$ | $OCH_3$ | H | H | 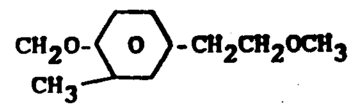 | Öl |
| 147 | H | $CH_2OCH_3$ | $OCH_3$ | H | H | 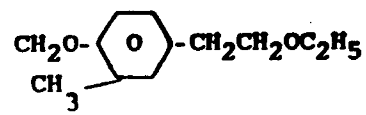 | Öl |

| Beisp. Nr. | $R^1$ | $R^2$ | $R^3$ | $R^4$ | $R^5$ | Q | Fp [°C] |
|---|---|---|---|---|---|---|---|
| 148 | H | $CH_2OCH_3$ | $OCH_3$ | H | H | 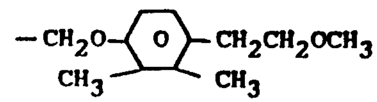 | Öl |
| 149 | H | $CH_2OCH_3$ | $OCH_3$ | H | H | 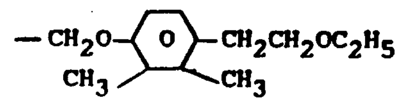 | Öl |
| 150 | H | $CH_2OCH_3$ | $OCH_3$ | H | $C_2H_5$ | 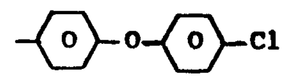 | Öl |
| 151 | H | $CH_2OCH_3$ | $OCH_3$ | H | $C_2H_5$ | 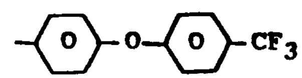 | |
| 152 | H | $CH_2OCH_3$ | $OCH_3$ | H | $C_2H_5$ | 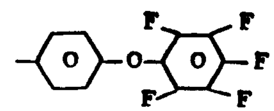 | |
| 153 | H | $CH_2OCH_3$ | $OCH_3$ | H | $C_2H_5$ | 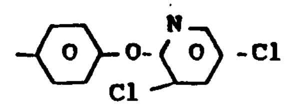 | |

| Beisp. Nr. | $R^1$ | $R^2$ | $R^3$ | $R^4$ | $R^5$ | Q | Fp [°C] |
|---|---|---|---|---|---|---|---|
| 154 | H | $CH_2OCH_3$ | $OCH_3$ | H | $C_2H_5$ | 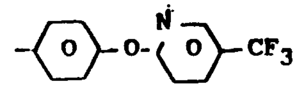 | Öl |
| 155 | H | $CH_2OCH_3$ | $OCH_3$ | H | $C_2H_5$ | 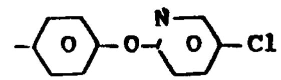 | |
| 156 | H | $CH_2OCH_3$ | $OCH_3$ | H | $C_2H_5$ | 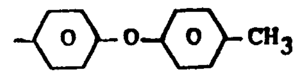 | Öl |
| 157 | H | $CH_2OCH_3$ | $OCH_3$ | H | $C_2H_5$ | 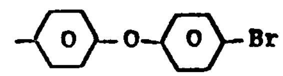 | |
| 158 | H | $CH_2OCH_3$ | $OCH_3$ | H | $C_2H_5$ | 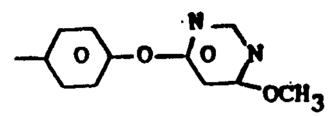 | |
| 159 | H | $CH_2OCH_3$ | H | H | $C_2H_5$ | 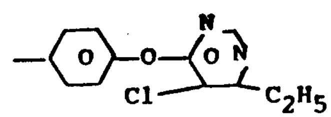 | |

| Beisp. Nr. | $R^1$ | $R^2$ | $R^3$ | $R^4$ | $R^5$ | Q | Fp [°C] |
|---|---|---|---|---|---|---|---|
| 160 | H | $CH_2OCH_3$ | H | H | $C_2H_5$ | 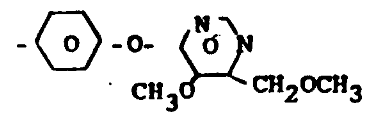 | Öl |
| 161 | H | $CH_2OCH_3$ | H | H | $C_2H_5$ | 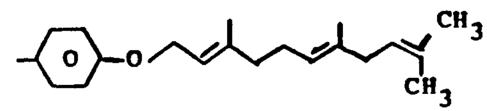 | |
| 162 | H | $CH_2OCH_3$ | H | H | $C_2H_5$ | 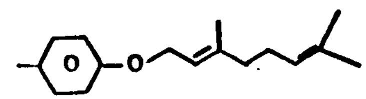 | |
| 163 | H | $CH_2OCH_3$ | H | H | $C_2H_5$ | 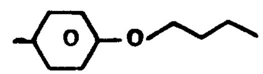 | Öl |
| 164 | H | $CH_2OCH_3$ | H | H | $C_2H_5$ | 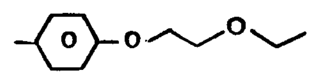 | |

| Beisp. Nr. | $R^1$ | $R^2$ | $R^3$ | $R^4$ | $R^5$ | Q | Fp [°C] |
|---|---|---|---|---|---|---|---|
| 165 | H | $SCH_3$ | $OCH_3$ | H | $CH_3$ | $(CH_2)_7CH_3$ | Öl |
| 166 | H | $C_2H_5$ | $C_2H_5$ | H | $CH_3$ | $(CH_2)_7CH_3$ | Öl |
| 167 | H | H | $OCH_3$ | H | $CH_3$ | $(CH_2)_7CH_3$ | Öl |
| 168 | H | Cl | $OCH_3$ | H | $CH_3$ | $(CH_2)_7CH_3$ | Öl |
| 169 | H | $OCH_3$ | $C_2H_5$ | H | $CH_3$ | $(CH_2)_7CH_3$ | Öl |
| 170 | H | $SCH_3$ | $C_2H_5$ | H | $CH_3$ | $(CH_2)_7CH_3$ | Öl |
| 171 | H | Cl | $C_2H_5$ | H | $CH_3$ | $(CH_2)_7CH_3$ | Öl |
| 172 | H | $CF_3$ | H | H | $CH_3$ | $(CH_2)_7CH_3$ | Öl |

| Beisp. Nr. | $R^1$ | $R^2$ | $R^3$ | $R^4$ | $R^5$ | Q | Fp [°C] |
|---|---|---|---|---|---|---|---|
| 173 | H | $CF_3$ | Cl | H | $CH_3$ | $(CH_2)_7CH_3$ | Öl |
| 174 | H | $CH_2OCH_3$ | H | H | $CH_3$ | $(CH_2)_7CH_3$ | Öl |

| Beisp. Nr. | $R^1$ | $R^2$ | $R^3$ | $R^4$ | $R^5$ | Q | Fp [°C] |
|---|---|---|---|---|---|---|---|
| 175 | H | $CH_2OCH_3$ | Cl | H | $CH_3$ | $(CH_2)_7CH_3$ | Öl |
| 176 | H | H | $OCH_3$ | H | H | 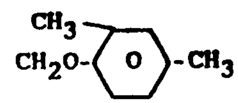 | Öl |
| 177 | H | $CH_2OCH_3$ | $OCH_3$ | H | $CH_3$ | $(CH_2)_3-C(CH_3)_2OH$ | Öl |
| 178 | H | $CH_2OCH_3$ | $OCH_3$ | H | $CH_3$ | $(CH_2)_2-O-CH_2CH(CH_3)_2$ | Öl |
| 179 | H | $CH_2OCH_3$ | $OCH_3$ | H | $C_2H_5$ | $(CH_2)_8CH_3$ | Öl |
| 180 | H | $CH_2OCH_3$ | $OCH_3$ | H | $n-C_3H_7$ | $(CH_2)_8CH_3$ | Öl |
| 181 | H | $CH_2OCH_3$ | $OCH_3$ | H | $n-C_8H_{17}$ | $(CH_2)_7CH_3$ | Öl |

| Beisp. Nr. | $R^1$ | $R^2$ | $R^3$ | $R^4$ | $R^5$ | Q | Fp [°C] |
|---|---|---|---|---|---|---|---|
| 182 | H | $CH_2OCH_3$ | $OCH_3$ | H | $n-C_5H_{11}$ | $(CH_2)_4CH_3$ | Öl |
| 183 | H | $CH_2OCH_3$ | $OCH_3$ | H | $C_2H_5$ | 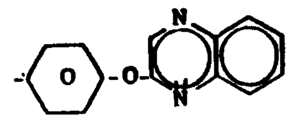 | |
| 184 | H | $CH_2OCH_3$ | $OCH_3$ | H | $C_2H_5$ | 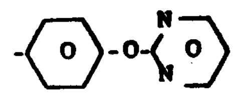 | |
| 185 | H | $CH_2OCH_3$ | $OCH_3$ | H | $C_2H_5$ | 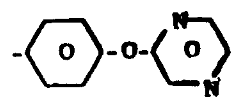 | Öl |
| 186 | H | $CH_2OCH_3$ | $OCH_3$ | H | $C_2H_5$ | 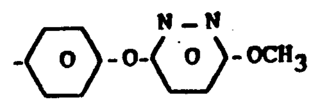 | |

| Beisp. Nr. | $R^1$ | $R^2$ | $R^3$ | $R^4$ | $R^5$ | Q | Fp [°C] |
|---|---|---|---|---|---|---|---|
| 187 | H | $CH_2OCH_3$ | $OCH_3$ | H | $C_2H_5$ | 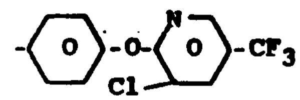 | |
| 188 | H | $CH_2OCH_3$ | $OCH_3$ | H | $C_2H_5$ | 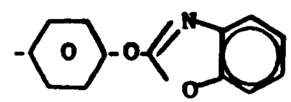 | |
| 189 | H | $CH_2OCH_3$ | $OCH_3$ | H | $C_2H_5$ | 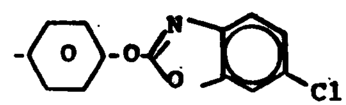 | |
| 190 | H | $CH_2OCH_3$ | $OCH_3$ | H | $C_2H_5$ | 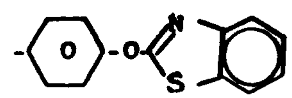 | |

| Beisp. Nr. | $R^1$ | $R^2$ | $R^3$ | $R^4$ | $R^5$ | Q | Fp [°C] |
|---|---|---|---|---|---|---|---|
| 191 | H | $CH_2OCH_3$ | $OCH_3$ | H | $C_2H_5$ | 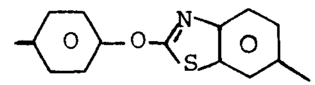 | |
| 192 | H | $CH_2OCH_3$ | $OCH_3$ | H | $C_2H_5$ | 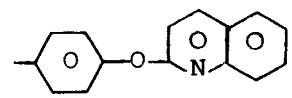 | |
| 192a | H | $CH_2OCH_3$ | $OCH_3$ | H | H | $-CH(CH_3)-CH_2-$ 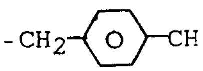 $-CH(CH_3)_2$ | Öl |
| 192b | H | $CH_2OCH_3$ | $OCH_3$ | H | H | $-CH(CH_3)-CH_2-$ 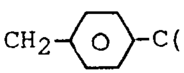 $-C(CH_3)_3$ | Öl |
| 192c | H | $CH_2OCH_3$ | $OCH_3$ | H | $CH_3$ | $-(CH_2)_7-CH_3$ | |

Tabelle B

| Beisp. Nr. | $R^4$ | $R^5$ | $R^{16}$ | $R^{17}$ | Q | Fp[°C] |
|---|---|---|---|---|---|---|
| 193 | H | $CH_3$ | $CH_3$ | H | $-(CH_2)_7CH_3$ | 100-103 |
| 194 | H | $CH_3$ | $CH_3$ | H | $-(CH_2)_8CH_3$ | 95-97 |
| 195 | H | H | $CH_3$ | $CH_3$ | $-(CH_2)_{10}CH_3$ | 67-68 |
| 196 | H | H | $CH_3$ | $CH_3$ | $-(CH_2)_4CH_3$ | 83-85 |
| 197 | H | $C_2H_5$ | $CH_3$ | $CH_3$ | 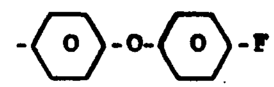 | |
| 198 | H | $CH_3$ | H | H | $(CH_2)_7CH_3$ | |
| 199 | H | $CH_3$ | H | H | $(CH_2)_6CH_3$ | |
| 200 | H | $C_2H_5$ | H | H | 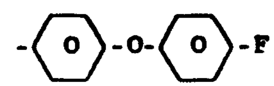 | |

| Beisp. Nr. | $R^4$ | $R^5$ | $R^{16}$ | $R^{17}$ | Q | Fp[°C] |
|---|---|---|---|---|---|---|
| 201 | H | $C_2H_5$ | H | H | 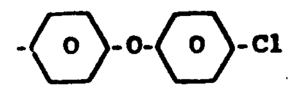 | |
| 202 | H | | H | H | 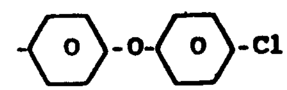 | |

Tabelle C

| Beisp. Nr. | $R^4$ | $R^5$ | $R^{16}$ | $R^{17}$ | Q | Fp[°C] |
|---|---|---|---|---|---|---|
| 203 | H | $CH_3$ | H | H | $-(CH_2)_8CH_3$ | 69-72 |
| 204 | H | $CH_3$ | H | H | $-(CH_2)_7CH_3$ | 100-103 |
| 205 | H | $CH_3$ | H | H | $-(CH_2)_6CH_3$ | |
| 206 | H | $C_2H_5$ | H | H | 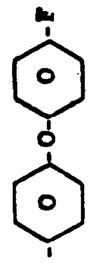 | |
| 207 | H | $C_2H_5$ | H | H | 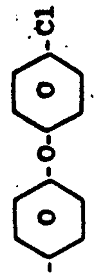 | |
| 208 | H | 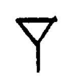 | H | H | $(CH_2)_7CH_3$ | |

C) Biologische Beispiele

Beispiel 1

Weizenpflanzen wurden im 3-Blattstadium mit Konidien des Weizenmehltaus (Erysiphe graminis) stark inokuliert und in einem Gewächshaus bei 20 ° C und einer relativen Luftfeuchte von 90-95% aufgestellt. 3 Tage nach Inokulation wurden die Pflanzen mit den in Tabelle A-C aufgeführten Verbindungen in den angegebenen Wirkstoffkonzentrationen gleichmäßig benetzt. Nach einer Inkubationszelt von 10 Tagen wurden die Pflanzen auf Befall mit Weizenmehltau untersucht. Der Befallsgrad wird ausgedrückt in % befallener Blattfläche, bezogen auf unbehandelt, infizierte Kontrollpflanzen ( = 100% Befall). Das Ergebnis ist in der Tabelle 1 zusammengefaßt.

Tabelle 1

| Verbindungen gemäß Beispiel Nr. | mit Weizenmehltau befallene Blattfläche in % bei mg Wirkstoff/Liter Spritzbrühe | | | |
|---|---|---|---|---|
| | 500 | 250 | 125 | 60 |
| 2 | 0 | 0 | 0 | 0 |
| 3 | 0 | 0 | 0 | 0 |
| 4 | 0 | 0 | 0 | 0 |
| 5 | 0 | 0 | 0 | 0 |
| 6 | 0 | 0 | 0 | 0 |
| 7 | 0 | 0 | 0 | 0 |
| 9 | 0 | 0 | 0 | 0 |
| 10 | 0 | 0 | 0 | 0 |
| 11 | 0 | 0 | 0 | 0 |
| 13 | 0 | 0 | 0 | 0 |
| 16 | 0 | 0 | 0 | 0 |
| 21 | 0 | 0 | 0 | 0 |
| 26 | 0 | 0 | 0 | 0 |
| 27 | 0 | 0 | 0 | 0 |
| 30 | 0 | 0 | 0 | 0 |
| 31 | 0 | 0 | 0 | 0 |
| 34 | 0 | 0 | 0 | 0 |
| 41 | 0 | 0 | 0 | 0 |
| 42 | 0 | 0 | 0 | 0 |
| 53 | 0 | 0 | 0 | 0 |
| 59 | 0 | 0 | 0 | 0 |
| 61 | 0 | 0 | 0 | 0 |
| 62 | 0 | 0 | 0 | 0 |
| unbehandelte, infizierte Pflanzen | 100 | | | |

Beispiel 2

Weizen aus der Sorte "Jubilar" wurde im 2-Blattstadium mit wäßrigen Suspensionen der beanspruchten Verbindung tropfnaß behandelt.

Nach dem Anstrocknen des Spritzbelages wurden die Pflanzen mit einer wäßrigen Sporensuspension von Puccinia recondita inokuliert. Die Pflanzen wurden für ca. 16 Stunden tropfnaß in eine Klimakammer mit 20 °C und ca. 100% rel. Luftfeuchte gestellt. Anschließend wurden die infizierten Pflanzen in einem Gewächshaus bei einer Temperatur von 22 - 25 °C und 50 - 70% rel. Luftfeuchte weiterkultiviert.

Nach einer Inkubationszeit von ca. 2 Wochen sporulierte der Pilz auf der gesamten Blattoberfläche der nicht behandelten Kontrollpflanzen, so daß eine Befallsauswertung der Versuchspflanzen vorgenommen werden konnte. Der Befallsgrad wird in % befallener Blattfläche im Vergleich zu den unbehandelten, infizierten Kontrollpflanzen angegeben und ist in Tabelle 2 wiedergegeben.

Tabelle 2

| Verbindungen gemäß Beispiel Nr. | mit Puccinia recondita befallene Blattfläche in % bei mg/Wirkstoff/Liter Spritzbrühe | | | |
|---|---|---|---|---|
| | 500 | 250 | 125 | 60 |
| 1 | 0 | 0 | 0 | 3 |
| 2 | 0 | 0 | 0 | 0 |
| 4 | 0 | 0 | 0 | 0 |
| 5 | 0 | 0 | 0 | 0 |
| 6 | 0 | 0 | 0 | 0 |
| 7 | 0 | 0 | 0 | 0 |
| 8 | 0 | 0 | 0 | 0 |
| 9 | 0 | 0 | 0 | 0 |

Fortsetzung Tabelle 2

| Verbindungen gemäß Beispiel Nr. | mit Puccinia recondita befallene Blattfläche in % bei mg/Wirkstoff/Liter Spritzbrühe | | | |
|---|---|---|---|---|
| | 500 | 250 | 125 | 60 |
| 10 | 0 | 0 | 0 | 0 |
| 11 | 0 | 0 | 0 | 0 |
| 13 | 0 | 0 | 0 | 0 |
| 15 | 0 | 0 | 0 | 3 |
| 16 | 0 | 0 | 0 | 0 |
| 21 | 0 | 0 | 0 | 0 |
| 26 | 0 | 0 | 0 | 0 |
| 27 | 0 | 0 | 0 | 0 |
| 30 | 0 | 0 | 0 | 0 |
| 31 | 0 | 0 | 0 | 0 |
| 38 | 0 | 0 | 0 | 0 |
| 39 | 0 | 0 | 0 | 0 |
| 41 | 0 | 0 | 0 | 0 |
| 42 | 0 | 0 | 0 | 0 |
| 53 | 0 | 0 | 0 | 0 |
| 55 | 0 | 0 | 0 | 0 |
| 59 | 0 | 0 | 0 | 0 |
| 60 | 0 | 0 | 0 | 3 |
| 61 | 0 | 0 | 0 | 0 |
| 62 | 0 | 0 | 0 | 0 |
| unbehandelte, infizierte Pflanzen | | 100 | | |

Beispiel 3

Apfelunterlagen (BM, IX) wurden im 4-Blattstadium mit den beanspruchten Verbindungen in den in Tab. 3 genannten Anwendungskonzentrationen gleichmäßig benetzt. Nach Antrocknen des Wirkstoffbelages wurden die Pflanzen mit Konidien des Apfelschorfs (Venturia inaequalis) stark infiziert und tropfnaß in eine Klimakammer gestellt, deren Temperatur 22° C und deren relative Luftfeuchtigkeit 100% betrug. Nach einer Infektionszeit von 48 Stunden kamen die Pflanzen in ein Gewächshaus mit 18° C und einer relativen Luftfeuchte von 95 - 100%. Nach einer Inkubationszeit von 14 Tagen wurden die Pflanzen auf Befall mit Apfelschorf (Venturia inaequalis) untersucht. Die Beurteilung des Befalls erfolgte wie üblich nach Augenschein. Der Befallsgrad der Pflanzen mit Apfelschorf wird in % befallener Blattfläche, bezogen auf unbehandelte, infizierte Pflanzen, ausgedrückt und ist in Tabelle 3 wiedergegeben.

Tabelle 3

| Verbindungen gemäß Beispiel Nr. | % Schorfbefall bei mg Wirkstoff/Liter Spritzbrühe | | | |
|---|---|---|---|---|
| | 500 | 250 | 125 | 60 |
| 21 | 0 | 0 | 0 | 0 |
| 27 | 0 | 0 | 0 | 0 |
| 62 | 0 | 0 | 0 | 0 |
| 61 | 0 | 0 | 0 | 0 |
| 26 | 0 | 0 | 0 | 0 |
| 11 | 0 | 0 | 0 | 0 |
| 9 | 0 | 0 | 0 | 0 |
| 6 | 0 | 0 | 0 | 0 |
| 5 | 0 | 0 | 0 | 0 |
| unbehandelte, infizierte Pflanzen | 100 | | | |

Beispiel 4

Weizenpflanzen der Sorte "Jubilar" wurden in 2-Blattstadium mit wäßrigen Suspensionen der beanspruchten Verbindungen tropfnaß behandelt.

Nach dem Antrocknen des Spritzbelages wurden die Pflanzen mit einer wäßrigen Pyknosporen-Suspension von Leptosphaeria nodorum inokuliert und mehrere Stunden bei 100% rel. Luftfeuchte in einer Klimakammer inkubiert. Bis zur Symptomausprägung wurden die Pflanzen im Gewächshaus bei ca. 90% rel. Luftfeuchte weiterkultiviert.

Der Befallsgrad wird in %befallener Blattfläche im Vergleich zu unbehandelten infizierten Kontrollpflanzen ausgedrückt und ist in Tabelle 4 wiedergegeben.

Tabelle 4

| Verbindungen gemäß Beispiel Nr. | mit Leptosphaeria nodorum befallen Blattfläche in % bei mg Wirkstoff/Liter Spritzbrühe | |
|---|---|---|
| | 500 | 250 |
| 4 | 0 | 0 |
| 6 | 0 | 0 |
| 7 | 0 | 0 |
| 8 | 0 | 0 |
| 9 | 0 | 0 |
| 10 | 0 | 0 |
| 13 | 0 | 0 |
| 15 | 0 | 0 |
| 18 | 0 | 0 |
| 20 | 0 | 0 |
| 21 | 0 | 0 |
| 25 | 0 | 0 |
| 27 | 0 | 0 |
| 31 | 0 | 0 |
| 32 | 0 | 0 |
| 34 | 0 | 0 |
| 42 | 0 | 0 |
| 53 | 0 | 0 |
| 55 | 0 | 0 |
| 56 | 0 | 0 |
| 58 | 0 | 0 |
| 62 | 0 | 0 |
| unbehandelte, infizierte Pflanzen | 100 | |

Beispiel 5

Mit Bohnenspinnmilben (Tetranychus urticae, Vollpopulation) stark befallene Bohnenpflanzen (Phaseolus v.) wurden mit der wäßrigen Verdünnung eines Spritzpulverkonzentrates, das 1000 ppm des jeweiligen Wirkstoffes enthielt, gespritzt.

Die Mortalität der Milben wurden nach 7 Tagen kontrolliert. 100% Mortalität wurde mit den Verbindungen gemäß Beispiel 3, 4, 6, 7, 9, 10, 11, 13, 15, 21, 26, 27, 30, 32, 34, 39, 42, 53, 56, 58, 60, 61, 62 und 63 erreicht.

Beispiel 6

Mit schwarzer Bohnenblattlaus (Aphis fabae) stark besetzte Ackerbohnen (Vicia faba) wurden mit wäßrigen Verdünnungen von Spritzpulverkonzentraten mit 1000 ppm Wirkstoffgehalt bis zum Stadium des beginnenden Abtropfens besprüht. Die Mortalität der Blattläuse wurde nach 3 Tagen bestimmt. Eine 100%ige Mortalität konnte mit den Verbindungen gemäß Beispiel 3, 4, 5, 6, 8, 9, 10, 11, 13, 15, 21, 26, 27, 30, 31, 32, 35, 38, 39, 41, 42, 53, 56, 58, 61, 62 und 63 erzielt werden.

Beispiel 7

Auf die Innenseite des Deckels und des Bodens einer Petrischale wurden jeweils 1 ml der zu testenden Formulierung emulgiert, in Wasser gleichmäßig aufgetragen und nach dem Antrocknen des Belages jeweils 10 Imagines der Hausfliege (Musca domestica) eingegeben. Nach dem Verschließen der Schale werden diese bei Raumtemperatur aufbewahrt und nach 3 Stunden die Mortalität der Versuchstiere bestimmt. Bei 250 ppm (bezogen auf den Gehalt an Wirkstoff) zeigen die Präparate 6, 10, 21, 31, 35, 42, 56, 58, 61, 62 und 63 eine gute Wirkung (100% Mortalität) gegenüber der Stubenfliege.

## Patentansprüche

1. Substituierte 4-Aminopyrimidine der allgemeinen Formel I

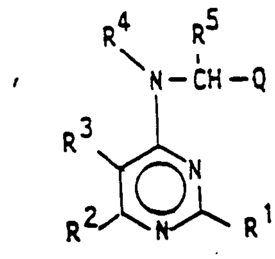

(I)

worin

$R^1$ Wasserstoff, Halogen, $(C_1-C_4)$Alkyl oder $(C_3-C_6)$Cycloalkyl bedeutet,

$R^2$ Wasserstoff, $(C_1-C_4)$Alkyl, Halogen, Trifluormethyl, $(C_1-C_4)$Alkoxy, $(C_1-C_4)$Halogenalkoxy, $(C_1-C_4)$Alkoxy-$(C_1-C_4)$alkyl, $(C_1-C_4)$Alkylthio, $(C_1-C_4)$Alkylthio-$(C_1-C_4)$alkyl, $(C_1-C_4)$Alkylamino, $(C_1-C_4)$Dialkylamino oder $(C_3-C_6)$Cycloalkylamino bedeutet,

$R^3$ Wasserstoff, $(C_1-C_4)$Alkyl, $(C_1-C_4)$Alkoxy, $(C_1-C_4)$Halogenalkoxy, $(C_1-C_4)$Alkylthio, Halogen, Nitro oder $(C_1-C_4)$Dialkylamino bedeutet, mit der Maßgabe, daß wenn Q keine der später aufgeführten Gruppen der Bedeutung $Q^1$ oder der allgemeinen Formeln II', II'', II''' oder II'''' ist und $R^3$ eine $(C_1-C_4)$Alkylgruppe oder ein Halogenatom bedeutet, $R^2$ nicht gleichzeitig $(C_1-C_4)$Alkyl, Halogen, $(C_1-C_4)$Alkoxy-$(C_1-C_4)$alkyl oder $(C_1-C_4)$Alkylthio-$(C_1-C_4)$alkyl bedeutet, oder, falls Q die Bedeutung $Q^1$ hat und $R^2$ eine Ethylgruppe bedeutet, $R^3$ nicht gleichzeitig Halogen bedeutet, oder, falls Q die Bedeutung $Q_1$ hat und $R^4$ einen Rest der Formel

$$-\underset{\underset{O}{\|}}{C}-NR^6R^7$$

und $R^2$ $(C_1-C_4)$Alkyl oder Halogen bedeuten, $R^3$ nicht gleichzeitig $(C_1-C_4)$Alkyl oder Halogen ist, oder,

falls Q ein Rest der allgemeinen Bedeutung $Q^1$ oder der Formel II ist, für die E eine direkte Bindung oder Methylenoxy ist, oder der Formeln II', II'', II''' oder II'''' oder der Bedeutung $Q^3$ ist, für die $R^{14}$ keine Gruppe der allgemeinen Bedeutung IV ist, $R^2$ und $R^3$ auch zusammen mit den Kohlenstoffatomen an das sie gebunden sind, einen ungesättigten 5-gliedrigen Ring bilden können, der ein an den Pyrimidin-Ring gebundenes Sauerstoff- oder Schwefelatom enthält und der gegebenenfalls durch Alkyl oder Halogen substituiert ist,

$R^4$ Wasserstoff oder einen Rest

$$-\underset{\underset{O}{\|}}{C}-NR^6R^7,$$

worin $R^6$ oder $R^7$ gleich oder verschieden sind und jeweils Wasserstoff, $(C_1-C_4)$Alkyl, Phenyl oder Phenyl-$(C_1-C_4)$alkyl bedeuten,

wobei die beiden vorgenannten Phenylgruppen unsubstituiert oder mit einem oder zwei Substituenten versehen sind und diese Substituenten jeweils Halogen, $(C_1-C_4)$Alkyl, $(C_1-C_4)$-Halogenalkyl, $(C_1-C_4)$Alkoxy, $(C_1-C_4)$-Alkylthio oder Nitro sein können, oder $R^6$ und $R^7$ zusammen mit dem Stickstoffatom an das sie gebunden sind einen gesättigten oder ungesättigten 5- oder 6-gliedrigen Ring bilden, der zusätzlich weitere Stickstoffatome oder ein Sauerstoff- oder Schwefelatom enthalten kann, wobei dieser Ring mit einem Benzolring kondensiert und mit einem oder zwei Substituenten versehen sein kann und diese Substituenten jeweils $(C_1-C_4)$Alkyl, Trifluormethyl, Halogen, $(C_1-C_4)$Alkoxy, $(C_1-C_4)$Alkylthio oder Nitro

sein können,

$R^5$ Wasserstoff, $(C_1-C_8)$Alkyl oder $(C_3-C_6)$Cycloalkyl bedeutet,

Q die Bedeutung $Q^1$ hat und

$Q^1$ $(C_1-C_{15})$Alkyl bedeutet, gegebenenfalls substituiert mit einem, zwei oder drei Halogenatomen, einer $(C_1-C_{15})$Alkoxygruppe, einer $(C_4-C_8)$Cycloalkylalkoxygruppe, einer Dioxolanylgruppe, einer $(C_1-C_4)$Alkoxy-$(C_1-C_4)$-alkoxygruppe, einer Hydroxygruppe, einer Methoxycarbonylgruppe, einer $(C_3-C_6)$Cycloalkylgruppe, einer 2-[2-($(C_1-C_4)$Alkoxy)ethoxy]-ethoxygruppe, einer Pyranyloxygruppe, einer Imidazol-1-ylgruppe, einer Triazol-1-ylgruppe, einer Pyrazol-1-ylgruppe oder einer Gruppe -A-B-$R^8$, worin A Sauerstoff, Schwefel oder Imino, B Carbonyl, Thiocarbonyl oder Sulfonyl bedeutet und $R^8$ $(C_1-C_4)$Alkyl, $(C_3-C_6)$-Cycloalkyl, $(C_1-C_4)$Halogenalkyl, $(C_1-C_4)$Alkoxy, eine substituierte oder unsubstituierte Phenoxygruppe, eine substituierte oder unsubstituierte Phenylgruppe, eine substituierte oder unsubstituierte Anilinogruppe, eine 2,6-Dimethylmorpholin-4-yl-, 4-Methylpiperazin-1-yl-, Imidazol-1-yl-, Triazol-1-yl-, Pyrazol-1-ylgruppe oder eine Mono- oder Di-$(C_1-C_4)$-alkylaminogruppe bedeutet, oder

Q die Bedeutung $Q^2$ hat und

$Q^2$ eine Gruppe der allgemeinen Formeln II, II', II'', II''' oder II'''' bedeutet

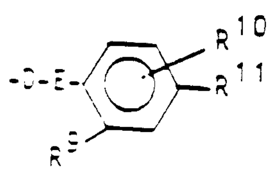

II

,

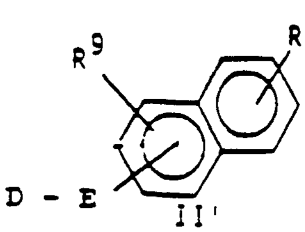

II'

,

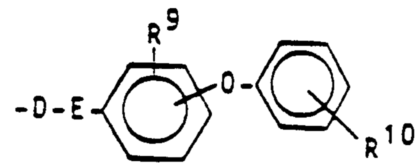

II''

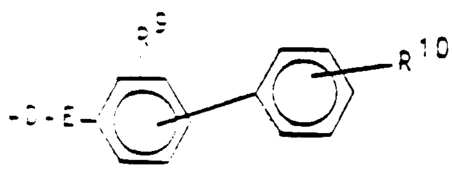

II'''

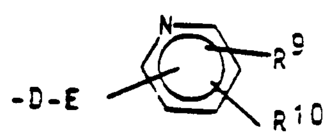

II''''

worin

| | |
|---|---|
| D | eine $(C_1-C_6)$Alkylengruppe bedeutet, |
| E | eine direkte Bindung, Sauerstoff oder Methylenoxy bedeutet, |
| $R^9$ und $R^{10}$ | gleich oder verschieden sind und jeweils Wasserstoff, Halogen, $(C_1-C_4)$Alkyl, $(C_3-C_6)$Cycloalkyl, $(C_1-C_4)$Halogenalkyl, $C_3$- oder $C_4$-Alkenyl, $(C_1-C_4)$Alkoxy, $(C_1-C_4)$-Alkylthio oder Nitro bedeuten, |
| $R^{11}$ | die für $R^9$ und $R^{10}$ angegebenen Bedeutungen hat, sowie für den Fall, daß E Sauerstoff bedeutet, zusätzlich eine Gruppe der Formel X-Y-$(GO)_n$-$R^{12}$ bedeutet, worin |
| X | eine $(C_1-C_8)$Alkylengruppe oder eine $(C_1-C_8)$Alkylengruppe mit einem $(C_1-C_4)$-Alkoxysubstituenten bedeutet, |
| Y | Sauerstoff, Schwefel oder eine Iminogruppe bedeutet, |
| G | eine $(C_1-C_8)$Alkylengruppe oder eine $(C_1-C_4)$Alkylenoxy-$(C_1-C_4)$alkylengruppe bedeutet, |
| n | Null oder 1 ist, |
| $R^{12}$ | $(C_1-C_4)$Alkyl, $C_3$- oder $C_4$-Alkenyl, $(C_4-C_6)$Alkadienyl, $C_3$- oder $C_4$-Alkinyl, Phenyl-$(C_1-C_3)$alkyl oder eine Gruppe der Formel $CH_2$-W bedeutet, worin |
| W | eine Gruppe der Formel CH = N-O$R^{13}$ bedeutet, worin |
| $R^{13}$ | Wasserstoff, $(C_1-C_4)$Alkyl, $C_3$- oder $C_4$-Alkenyl oder Phenyl-$(C_1-C_3)$alkyl bedeutet, |
| W | weiterhin eine Morpholinomethylgruppe oder eine heterocyclische Gruppe mit 5 bis |

8 Ringatomen bedeutet, von denen 2 oder 3 Sauerstoff- oder Schwefelatome sind, und der besagte Heterocyclus gesättigt ist oder eine Doppelbindung im Ring besitzt und der besagte Heterocyclus unsubstituiert ist oder einen oder zwei Alkyl-, $(C_1-C_4)$-Halogenalkyl- oder Phenylsubstituenten trägt, oder

Q die Bedeutung $Q^3$ hat und

$Q^3$ eine Gruppe der allgemeinen Formel III bedeutet

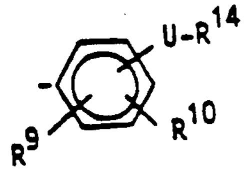

(III),

worin $R^9$ und $R^{10}$ die oben angegebenen Bedeutungen haben, und

U eine direkte Bindung, Sauerstoff, Schwefel, $(C_1-C_3)$Alkylen, oder $(C_1-C_3)$Alkylenoxy bedeutet,

$R^{14}$ Phenyl oder einen Heterocyclus bedeutet, wobei jeder der beiden vorgenannten Reste unsubstituiert oder mit einem oder zwei Substituenten versehen sein kann und diese Substituenten gleich oder verschieden sind und jeweils Halogen, $(C_1-C_4)$alkyl, $(C_1-C_4)$Halogenalkyl, Nitro,
$(C_1-C_4)$Alkoxy, $(C_1-C_4)$Alkylthio, $(C_1-C_4)$Alkoxy-$(C_1-C_4)$alkyl, Phenyl, Phenoxy, Halogenphenoxy oder $(C_1-C_4)$Alkylphenoxy bedeuten,

$R^{14}$ weiterhin, für den Fall, daß U Sauerstoff ist, $(C_5-C_{10})$Alkyl, Allyl, Geranyl, Farnesyl, $(C_1-C_4)$Halogenalkyl, $(C_3-C_6)$Cycloalkylmethyl, eine Ethylgruppe, die in 2-Stellung durch $(C_1-C_4)$Alkoxy, $(C_1-C_4)$Alkoxy-$(C_1-C_4)$alkyl, $(C_1-C_4)$Alkylthio, $(C_1-C_4)$-Alkylsulfonyl oder eine Phenoxygruppe, die gegebenenfalls mit einer oder zwei $(C_1-C_4)$Alkylgruppen substituiert sein kann, Glycidyl, Acetonyl, Halogenphenoxymethyldioxolanyl, 2,2-Diethoxyethyl, 1-Ethoxycarbonylmethyl, Trimethylsilylmethyl, 1-Pyridylethyl oder eine $(C_1-C_4)$Alkylgruppe, die mit einer $(C_1-C_4)$Alkoxyimino- oder einer Benzyloxyiminogruppe substituiert ist, substituiert ist, bedeutet,

$R^{14}$ weiterhin, für den Fall, daß U Sauerstoff ist, eine Gruppe der allgemeinen Formel IV

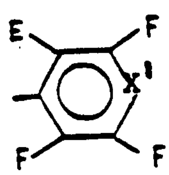

(IV)

bedeutet, worin

X' Stickstoff oder eine Gruppe $CR^{15}$ bedeutet, worin

$R^{15}$ Wasserstoff, Halogen, $(C_1-C_4)$Alkyl, $(C_1-C_4)$Halogenalkyl, $(C_1-C_4)$Alkanoyl, Nitro, Cyano oder 1,3-Dioxolan-2-yl bedeutet, sowie ihre Salze und ihre Stereoisomeren.

**2.** Verbindungen gemäß Anspruch 1, dadurch gekennzeichnet, daß in Formel I

$R^1$ Wasserstoff, Methyl oder Halogen bedeutet,

$R^2$ Wasserstoff, Methyl, Ethyl, Halogen, Trifluormethyl, $(C_1-C_4)$Alkoxy, $(C_1-C_4)$Halogenalkoxy, Methoxymethyl, Dimethylamino oder Diethylamino bedeutet,

$R^3$ Wasserstoff, $(C_1-C_4)$Alkyl, $(C_1-C_4)$Alkoxy, $(C_1-C_4)$Halogenalkoxy oder Halogen bedeutet, mit der Maßgabe, daß wenn Q keine der Gruppen der Bedeutung $Q^1$ oder der allgemeinen Formeln II', II'', II''' oder II'''' ist und $R^3$ eine $(C_1-C_4)$Alkylgruppe oder ein Halogenatom bedeutet, $R^2$ nicht gleichzeitig Methyl, Ethyl, Halogen oder Methoxymethyl bedeutet, oder falls Q die Bedeutung $Q^1$ hat und $R^2$ eine Ethylgruppe bedeutet, $R^3$ nicht gleichzeitig Halogen bedeutet, oder,

falls Q die Bedeutung $Q^1$ hat und $R^4$ ein Rest der Formel

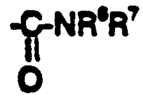

und $R^2$ Methyl, Ethyl oder Halogen bedeuten, $R^3$ nicht gleichzeitig $(C_1-C_4)$Alkyl oder Halogen ist, und

$R^5$ Wasserstoff, $(C_1-C_8)$Alkyl oder Cyclopropyl bedeutet.

**3.** Verbindungen gemäß Anspruch 1 oder 2, dadurch gekennzeichnet, daß in Formel I

$R^3$ Wasserstoff, $(C_1-C_4)$Alkyl, $(C_1-C_4)$Alkoxy, $(C_1-C_4)$Halogenalkoxy oder Halogen bedeutet, mit der Maßgabe, daß wenn Q keine der Gruppen der Bedeutung $Q^1$ oder der allgemeinen Formeln II', II'', II''' oder II'''' ist und $R^3$ eine $(C_1-C_4)$Alkylgruppe oder ein Halogenatom bedeutet, $R^2$ nicht gleichzeitig Methyl, Ethyl oder Methoxymethyl bedeutet, oder, falls Q die Bedeutung $Q_1$ hat und $R^2$ eine Ethylgruppe bedeutet, $R^3$ nicht gleichzeitig Halogen bedeutet, $R^2$ und $R^3$ auch zusammen mit den Kohlenstoffatomen an das sie gebunden sind, einen ungesättigten 5-gliedrigen Ring bilden können, der ein an den Pyrimidin-Ring gebundenes Sauerstoff- oder Schwefelatom enthält und der gegebenenfalls durch alkyl oder Halogen substituiert ist, und

$R^4$ Wasserstoff bedeutet.

**4.** Verbindungen gemäß Anspruch 1 oder 2, dadurch gekennzeichnet, daß in Formel I

$R^2$ Wasserstoff, Methyl, Ethyl, Halogen, Trifluormethyl, $(C_1-C_4)$Alkoxy, $(C_1-C_4)$Halogenalkoxy, Methoxymethyl, Dimethylamino oder Diethylamino bedeutet,

$R^3$ Wasserstoff, $(C_1-C_4)$Alkyl, $(C_1-C_4)$Alkoxy, $(C_1-C_4)$Halogenalkoxy oder, falls $R^3$ nicht Ethyl ist, auch Halogen bedeutet,

$R^4$ Wasserstoff bedeutet,

$R^5$ Wasserstoff, $(C_1-C_4)$Alkyl oder Cyclopropyl bedeutet,

Q die Bedeutung $Q^1$ hat und

$Q^1$ $(C_1-C_{15})$Alkyl bedeutet, gegebenenfalls substituiert mit einem, zwei oder drei Halogenatomen, einer $(C_1-C_{15})$Alkoxygruppe,
einer $(C_4-C_8)$Cycloalkylalkoxygruppe, einer Dioxolanylgruppe, einer $(C_1-C_4)$Alkoxy-$(C_1-C_4)$-alkoxygruppe, einer Hydroxygruppe, einer
Methoxycarbonylgruppe, einer $(C_3-C_6)$Cycloalkylgruppe, einer 2-[2-($(C_1-C_4)$Alkoxy)ethoxy]-ethoxygruppe, einer Pyranyloxygruppe, einer Imidazol-1-ylgruppe, einer Triazol-1-ylgruppe, einer Pyrazol-1-ylgruppe oder einer Gruppe -A-B-$R^8$, worin A Sauerstoff, Schwefel oder Imino, B Carbonyl, Thiocarbonyl oder Sulfonyl bedeutet und $R^8$ $(C_1-C_4)$Alkyl,
$(C_3-C_6)$Cycloalkyl, $(C_1-C_4)$Halogenalkyl, $(C_1-C_4)$Alkoxy, eine substituierte oder unsubstituierte Phenoxygruppe, eine substituierte oder unsubstituierte Phenylgruppe, eine substituierte oder unsubstituierte Anilinogruppe, eine 2,6-Dimethylmorpholin-4-yl-, 4-Methylpiperazin-1-yl-, Imidazol-1-yl-, Triazol-1-yl-, Pyrazol-1-ylgruppe oder eine Mono- oder Di-$(C_1-C_4)$-alkylaminogruppe bedeutet.

**5.** Verbindungen gemäß Anspruch 1 oder 2, dadurch gekennzeichnet, daß in Formel I

$R^2$ Wasserstoff, Methyl, Ethyl, Halogen, Trifluormethyl, Methoxy, Ethoxy, $(C_1-C_4)$-Halogenalkoxy, Methoxymethyl, Dimethylamino oder Diethylamino bedeutet,

$R^3$ Wasserstoff, $(C_1-C_4)$Alkyl, $(C_1-C_4)$Alkoxy, $(C_1-C_4)$Halogenalkoxy oder Halogen bedeutet, mit der Maßgabe, daß wenn Q keine der später aufgeführten Gruppen der allgemeinen Formeln II', II'', II''' oder II'''' ist und $R^3$ eine $(C_1-C_4)$Alkylgruppe oder ein Halogenatom bedeutet, $R^2$ nicht gleichzeitig Methyl, Ethyl, Halogen oder Methoxymethyl bedeutet,

$R^4$ Wasserstoff bedeutet,

$R^5$ Wasserstoff, $(C_1-C_4)$Alkyl oder Cyclopropyl bedeutet,

Q die Bedeutung $Q^2$ hat und

$Q^2$ eine Gruppe der allgemeinen Formeln II, II', II'', II''' oder II'''' bedeutet, worin

D eine $(C_1-C_6)$Alkylengruppe bedeutet,
E eine direkte Bindung, Sauerstoff oder Methylenoxy bedeutet,
$R^9$ und $R^{10}$ gleich oder verschieden sind und jeweils Wasserstoff, Halogen, $(C_1-C_4)$Alkyl, $(C_3-C_6)$Cycloalkyl, $(C_1-C_4)$Halogenalkyl, $C_3$- oder $C_4$-Alkenyl, $(C_1-C_4)$Alkoxy, $(C_1-C_4)$-Alkylthio oder Nitro bedeuten,
$R^{11}$ die für $R^9$ und $R^{10}$ angegebenen Bedeutungen hat, sowie für den Fall, daß E Sauerstoff bedeutet, zusätzlich eine Gruppe der Formel X-Y-$(GO)_n$-$R^{12}$ bedeutet, worin X eine $(C_1-C_8)$Alkylengruppe oder eine $(C_1-C_8)$Alkylengruppe mit einem $(C_1-C_4)$Alkoxysubstituenten bedeutet,
Y Sauerstoff, Schwefel oder eine Iminogruppe bedeutet,
G eine $(C_1-C_8)$Alkylengruppe oder eine $(C_1-C_4)$Alkylenoxy-$(C_1-C_4)$alkylengruppe bedeutet,
n Null oder 1 ist,
$R^{12}$ $(C_1-C_4)$Alkyl, $C_3$- oder $C_4$-Alkenyl, $(C_4-C_6)$Alkadienyl, $C_3$- oder $C_4$-Alkinyl, Phenyl-$(C_1-C_3)$alkyl oder eine Gruppe der Formel $CH_2$-W bedeutet, worin
W eine Gruppe der Formel CH = N-$OR^{13}$ bedeutet, worin
$R^{13}$ Wasserstoff, $(C_1-C_4)$Alkyl, $C_3$- oder $C_4$-Alkenyl oder Phenyl-$(C_1-C_3)$alkyl bedeutet, und
W weiterhin eine Morpholinomethylgruppe oder eine heterocyclische Gruppe mit 5 bis 8 Ringatomen bedeutet, von denen 2 oder 3 Sauerstoff- oder Schwefelatome sind, und der besagte Heterocyclus gesättigt ist oder eine Doppelbindung im Ring besitzt und der besagte Heterocyclus unsubstituiert ist oder einen oder zwei Alkyl-, $(C_1-C_4)$-Halogenalkyl- oder Phenylsubstituenten trägt.

**6.** Verbindungen gemäß Anspruch 1 oder 2, dadurch gekennzeichnet, daß in Formel I
$R^2$ Wasserstoff, Methyl, Ethyl, Halogen, Trifluormethyl, Methoxy, Ethoxy, $(C_1-C_4)$-Halogenalkoxy, Methoxymethyl, Dimethylamino oder Diethylamino bedeutet,
$R^3$ Wasserstoff, $(C_1-C_4)$Alkyl, $(C_1-C_4)$Alkoxy, $(C_1-C_4)$Halogenalkoxy oder Halogen bedeutet, mit der Maßgabe, daß wenn $R^3$ $(C_1-C_4)$Alkyl oder Halogen bedeutet, $R^2$ nicht gleichzeitig Halogen, Methyl, Ethyl oder Methoxymethyl bedeutet,
$R^4$ Wasserstoff bedeutet,
$R^5$ Wasserstoff, $(C_1-C_4)$Alkyl oder Cyclopropyl bedeutet,
Q die Bedeutung $Q^3$ hat und
$Q^3$ eine Gruppe der allgemeinen Formel III bedeutet, worin
$R^9$ und $R^{10}$ die genannten Bedeutungen haben,
U eine direkte Bindung, Sauerstoff, Schwefel, $(C_1-C_3)$Alkylen oder $(C_1-C_3)$Alkylenoxy bedeutet, und
$R^{14}$ Phenyl oder einen Hetercyclus bedeutet, wobei jeder der beiden vorgenannten Rests unsubstituiert oder mit einem oder zwei Substituenten versehen sein kann und diese Substituenten gleich oder verschieden sind und jeweils Halogen, $(C_1-C_4)$Alkyl, $(C_1-C_4)$Halogenalkyl, Nitro,
$(C_1-C_4)$Alkoxy, $(C_1-C_4)$Alkylthio, $(C_1-C_4)$Alkoxy-$(C_1-C_4)$alkyl, Phenyl, Phenoxy, Halogenphenoxy oder $(C_1-C_4)$Alkylphenoxy bedeuten, oder
$R^{14}$ weiterhin, für den Fall, daß U Sauerstoff ist, $(C_5-C_{10})$Alkyl, Allyl, Geranyl, Farnesyl, $(C_1-C_4)$Halogenalkyl, $(C_3-C_6)$Cycloalkylmethyl, eine Ethylgruppe, die In 2-Stellung durch $(C_1-C_4)$Alkoxy, $(C_1-C_4)$Alkoxy-$(C_1-C_4)$alkyl, $(C_1-C_4)$Alkylthio, $(C_1-C_4)$-Alkylsulfonyl oder eine Phenoxygruppe, die gegebenenfalls mit einer oder zwei $(C_1-C_4)$Alkylgruppen substituiert sein kann, Glycidyl, Acetonyl, Halogenphenoxymethyldioxolanyl, 2,2-Diethoxyethyl, 1-Ethoxycarbonylmethyl, Trimethylsilylmethyl, 1-Pyridylethyl oder eine $(C_1-C_4)$Alkylgruppe, die mit einer $(C_1-C_4)$Alkoxyimino- oder einer Benzyloxyiminogruppe substituiert ist, substituiert ist, bedeutet, oder
$R^{14}$ weiterhin, für den Fall, daß U Sauerstoff ist, eine Gruppe der allgemeinen Formel IV bedeutet, worin
X' Stickstoff oder eine Gruppe $CR^{15}$ bedeutet, worin
$R^{15}$ Wasserstoff, Halogen, $(C_1-C_4)$Alkyl, $(C_1-C_4)$Halogenalkyl, $(C_1-C_4)$Alkanoyl, Nitro, Cyano oder 1,3-Dioxolan-2-yl bedeutet.

**7.** Verbindungen gemäß Anspruch 1 oder 2, dadurch gekennzeichnet, daß in Formel I

$R^2$ Wasserstoff, Halogen, Methoxy, Ethoxy, Trifluormethyl, Methoxymethyl, Dimethylamino oder Diethylamino bedeutet,

$R^3$ Wasserstoff, $(C_1-C_3)$Alkyl, Methoxy, Ethoxy, $(C_1-C_2)$Halogenalkoxy oder Halogen bedeutet, mit der Maßgabe, daß wenn Q keine der Gruppen der Bedeutung $Q^1$ oder der allgemeinen Formeln II', II'', II''' oder II'''' ist und $R^3$ eine $(C_1-C_3)$Alkylgruppe oder ein Halogenatom bedeutet, $R^2$ nicht gleichzeitig Halogen oder Methoxymethyl bedeutet, und

$R^4$ Wasserstoff bedeutet.

**8.** Verbindungen gemäß Anspruch 1 oder 2, dadurch gekennzeichnet, daß in Formel I

$R^2$ Wasserstoff, Halogen, Trifluormethyl, Methoxy, Ethoxy, Methoxymethyl< Dimethylamino oder Diethylamino bedeutet,

$R^3$ Wasserstoff, $(C_1-C_3)$Alkyl, Methoxy, Ethoxy, $(C_1-C_2)$Halogenalkoxy oder Halogen bedeutet,

$R^4$ Wasserstoff bedeutet,

Q die Bedeutung $Q^1$ hat und

$Q^1$ $(C_1-C_{15})$Alkyl bedeutet, gegebenenfalls substituiert mit einem, zwei oder drei Halogenatomen, einer $(C_1-C_{15})$Alkoxygruppe, einer $(C_4-C_8)$Cycloalkylalkoxygruppe, einer Dioxolanylgruppe, einer $(C_1-C_4)$Alkoxy-$(C_1-C_4)$-alkoxygruppe, einer Hydroxygruppe, einer Methoxycarbonylgruppe, einer $(C_3-C_6)$Cycloalkylgruppe, einer 2-[2-($(C_1-C_4)$Alkoxy)ethoxy]ethoxygruppe, Pyranyloxygruppe, einer imidazol-1-ylgruppe, einer Triazol-1-ylgruppe, einer Pyrazol-1-ylgruppe oder einer Gruppe -A-B-$R^8$, worin A Sauerstoff, Schwefel oder Imino, B Carbonyl, Thiocarbonyl oder Sulfonyl bedeutet und $R^8$ -$(C_1-C_4)$Alkyl, $(C_3-C_6)$Cycloalkyl, $(C_1-C_4)$Halogenalkyl, $(C_1-C_4)$Alkoxy, eine substituierte oder unsubstituierte Phenoxygruppe, eine substituierte oder unsubstituierte Phenylgruppe, eine substituierte oder unsubstituierte Anilinogruppe, eine 2,6-Dimethylmorpholin-4-yl-, 4-Methylpiperazin-1-yl-, Imidazol-1-yl-, Triazol-1-yl-, Pyrazol-1-ylgruppe oder Mono- oder Di-$(C_1-C_4)$alkylaminogruppe bedeutet.

**9.** Verbindungen gemäß Anspruch 1 oder 2, dadurch gekennzeichnet, daß in Formel I

$R^2$ Wasserstoff, Halogen, Methoxy, Ethoxy, Trifluormethyl, Methoxymethyl, Dimethylamino oder Diethylamino bedeutet,

$R^3$ Wasserstoff, $(C_1-C_3)$Alkyl, Methoxy, Ethoxy, $(C_1-C_2)$Halogenalkoxy oder Halogen bedeutet, mit der Maßgabe, daß wenn Q keine der später aufgeführten Gruppen der allgemeinen Formeln II', II'', II''' oder II'''' ist und $R^3$ eine $(C_1-C_3)$Alkylgruppe oder ein Halogenatom bedeutet, $R^2$ nicht gleichzeitig Halogen oder Methoxymethyl bedeutet,

$R^4$ Wasserstoff bedeutet,

Q die Bedeutung $Q^2$ hat und

$Q^2$ eine Gruppe der allgemeinen Formeln II, II', II'', II''' oder II'''' bedeutet, worin

D eine $(C_1-C_6)$Alkylengruppe bedeutet,

E eine direkte Bindung, Sauerstoff oder Methylenoxy bedeutet,

$R^9$ und $R^{10}$ gleich oder verschieden sind und jeweils Wasserstoff, Halogen, $(C_1-C_4)$Alkyl, $(C_3-C_6)$Cycloalkyl, $(C_1-C_4)$Halogenalkyl, $C_3$- oder $C_4$-Alkenyl, $(C_1-C_4)$Alkoxy, $(C_1-C_4)$-Alkylthio oder Nitro bedeuten,

$R^{11}$ die für $R^9$ und $R^{10}$ angegebenen Bedeutungen hat, sowie für den Fall, daß E Sauerstoff bedeutet, zusätzlich eine Gruppe der Formel $X\text{-}Y\text{-}(GO)_n\text{-}R^{12}$ bedeutet, worin X eine $(C_1-C_8)$Alkylengruppe oder eine $(C_1-C_8)$Alkylengruppe mit einem $(C_1-C_4)$Alkoxysubstituenten bedeutet,

Y Sauerstoff, Schwefel oder eine Iminogruppe bedeutet,

G eine $(C_1-C_8)$Alkylengruppe oder eine $(C_1-C_8)$Alkylenoxy-$(C_1-C_4)$alkylengruppe bedeutet,

n Null oder 1 ist,

$R^{12}$ $(C_1-C_4)$Alkyl, $C_3$- oder $C_4$-Alkenyl, $(C_4-C_6)$Alkadienyl, $C_3$- oder $C_4$-Alkinyl, Phenyl-$(C_1-C_3)$alkyl oder eine Gruppe der Formel $CH_2$-W bedeutet, worin

W eine Gruppe der Formel $CH=N\text{-}OR^{13}$ bedeutet, worin

$R^{13}$ Wasserstoff, $(C_1-C_4)$Alkyl, $C_3$- oder $C_4$-Alkenyl oder Phenyl-$(C_1-C_3)$alkyl bedeutet, und

W weiterhin eine Morpholinomethylgruppe oder eine heterocyclische Gruppe mit 5 bis

8 Ringatomen bedeutet, von denen 2 oder 3 Sauerstoff- oder Schwefelatome sind, und der besagte Heterocyclus gesättigt ist oder eine Doppelbindung im Ring besitzt und der besagte Heterocyclus unsubstituiert ist oder einen oder zwei Alkyl-, $(C_1-C_4)$-Halogenalkyl- oder Phenylsubstituenten trägt.

**10.** Verbindungen gemäß Anspruch 1 oder 2, dadurch gekennzeichnet, daß in Formel I

- $R^2$ Wasserstoff, Halogen, Methoxy, Ethoxy, Trifluormethyl, Methoxymethyl, Dimethylamino oder Diethylamino bedeutet,
- $R^3$ Wasserstoff, $(C_1-C_3)$Alkyl, Methoxy, Ethoxy, $(C_1-C_2)$Halogenalkoxy oder Halogen bedeutet, mit der Maßgabe, daß wenn $R^3$ $(C_1-C_3)$Alkyl oder Halogen bedeutet, $R^2$ nicht gleichzeitig Halogen oder Methoxymethyl bedeutet,
- $R^4$ Wasserstoff bedeutet,
- Q die Bedeutung $Q^3$ hat und
- $Q^3$ eine Gruppe der allgemeinen Formel III bedeutet, worin $R^9$ und $R^{10}$ die genannten Bedeutungen haben,
- U eine direkte Bindung, Sauerstoff, Schwefel, $(C_1-C_3)$Alkylen oder $(C_1-C_3)$Alkylenoxy bedeutet, und
- $R^{14}$ Phenyl oder einen Heterocyclus bedeutet, wobei jeder der beiden vorgenannten Reste unsubstituiert oder mit einem oder zwei Substituenten versehen sein kann und diese Substituenten gleich oder verschieden sind und jeweils Halogen, $(C_1-C_4)$Alkyl, $(C_1-C_4)$-Halogenalkyl, $(C_1-C_4)$Alkoxy, Nitro, $(C_1-C_4)$Alkylthio, $(C_1-C_4)$Alkoxy-$(C_1-C_4)$alkyl, Phenyl, Phenoxy, Halogenphenoxy oder $(C_1-C_4)$Alkylphenoxy bedeuten, oder
- $R^{14}$ weiterhin, für den Fall, daß U Sauerstoff ist, $(C_5-C_{10})$Alkyl, Allyl, Geranyl, Farnesyl, $(C_1-C_4)$-Halogenalkyl, $(C_3-C_6)$Cycloalkylmethyl, eine Ethylgruppe, die in 2-Stellung durch $(C_1-C_4)$-Alkoxy, $(C_1-C_4)$Alkoxy-$(C_1-C_4)$alkyl, $(C_1-C_4)$Alkylthio, $(C_1-C_4)$Alkylsulfonyl oder eine Phenoxygruppe, die gegebenenfalls mit einer oder zwei $(C_1-C_4)$Alkylgruppen substituiert sein kann, Glycidyl, Acetonyl, Halogenphenoxymethyldioxolanyl, 2,2-Diethoxyethyl, 1-Ethoxycarbonylmethyl, Trimethylsilylmethyl, 1-Pyridylethyl oder eine $(C_1-C_4)$Alkylgruppe, die mit einer $(C_1-C_4)$Alkoxyimino- oder eine Benzyloxyiminogruppe substituiert ist, substituiert ist, bedeutet, und
- $R^{14}$ weiterhin, für den Fall, daß U Sauerstoff ist, eine Gruppe der allgemeinen Formel IV bedeutet, worin
- X' Stickstoff oder eine Gruppe $CR^{15}$ bedeutet, worin
- $R^{15}$ Wasserstoff, Halogen, $(C_1-C_4)$Alkyl, $(C_1-C_4)$Halogenalkyl, $(C_1-C_4)$Alkanoyl, Nitro, Cyano oder 1,3-Dioxolan-2-yl bedeutet.

**11.** Verbindungen gemäß einem der Ansprüche 1 bis 10, dadurch gekennzeichnet, daß in Formel I

- $R^1$ Wasserstoff bedeutet,
- $R^2$ Methoxy oder Methoxymethyl bedeutet,
- $R^3$ Methoxy bedeutet und
- $R^4$ Wasserstoff bedeutet.

**12.** Verbindungen gemäß Anspruch 11, dadurch gekennzeichnet, daß in Formel I

- $R^5$ Wasserstoff, Methyl, Ethyl oder Cyclopropyl bedeutet.

**13.** Verbindungen gemäß Anspruch 11 oder 12, dadurch gekennzeichnet, daß

- $R^2$ Methoxymethyl bedeutet,
- $R^5$ Wasserstoff, Methyl oder Cyclopropyl bedeutet,
- Q die Bedeutung $Q^1$ hat und
- $Q^1$ $(C_3-C_{13})$Alkyl bedeutet.

**14.** Verbindungen gemäß Anspruch 11 oder 12, dadurch gekennzeichnet, daß in Formel I

- $R^5$ Wasserstoff, Methyl oder Cyclopropyl bedeutet,
- Q die Bedeutung $Q^2$ hat und
- $Q^2$ eine Gruppe der allgemeinen Formeln II, II', II'', II''' oder II'''' bedeutet, worin
- D eine $(C_1-C_2)$Alkylengruppe bedeutet,
- E eine direkte Bindung oder Sauerstoff bedeutet,
- $R^9$ und $R^{10}$ gleich oder verschieden sind und jeweils Wasserstoff, Halogen, $(C_1-C_4)$Alkyl, $(C_3$-

$C_6$)Cycloalkyl, ($C_1$-$C_4$)Halogenalkyl, ($C_1$-$C_4$)Alkoxy, bedeuten,

$R^{11}$ die für $R^9$ und $R^{10}$ angegebenen Bedeutungen hat, sowie für den Fall, daß E Sauerstoff bedeutet, zusätzlich eine Gruppe der Formel X-Y-$(GO)_n$-$R^{12}$ bedeutet, worin

X eine ($C_1$-$C_8$)Alkylengruppe bedeutet,

Y Sauerstoff bedeutet,

G eine Ethylengruppe bedeutet,

n Null oder 1 ist und

$R^{12}$ ($C_1$-$C_4$)Alkyl, bedeutet.

**15.** Verbindungen gemäß Anspruch 11 oder 12, dadurch gekennzeichnet, daß in Formel I

Q die Bedeutung $Q^3$ hat und

$Q^3$ eine Gruppe der allgemeinen Formel III bedeutet, worin $R^9$ und $R^{10}$ die genannten Bedeutungen haben,

U Sauerstoff bedeutet und

$R^{14}$ Phenyl oder einen Heterocyclus bedeutet, wobei jeder der beiden vorgenannten Reste unsubstituiert oder mit einem oder zwei Substituenten versehen sein kann und diese Substituenten gleich oder verschieden sind und jeweils Halogen, ($C_1$-$C_4$)Alkyl, ($C_1$-$C_4$)-Halogenalkyl, ($C_1$-$C_4$)Alkoxy, Nitro, ($C_1$-$C_4$)Alkylthio, ($C_1$-$C_4$)Alkoxy-($C_1$-$C_4$)alkyl, Phenyl, Phenoxy, Halogenphenoxy oder ($C_1$-$C_4$)Alkylphenoxy bedeuten, oder

$R^{14}$ weiterhin, für den Fall, daß U Sauerstoff ist, ($C_5$-$C_{10}$)Alkyl, Allyl, Geranyl, Farnesyl, ($C_1$-$C_4$)-Halogenalkyl oder ($C_3$-$C_6$)Cycloalkylmethyl, bedeutet oder

$R^{14}$ weiterhin, für den Fall, daß U Sauerstoff ist, eine Gruppe der allgemeinen Formel IV bedeutet, worin

X' Stickstoff oder eine Gruppe der Formel CF bedeutet.

**16.** Verbindungen gemäß Anspruch 1, dadurch gekennzeichnet, daß in Formel I

$R^1$ Wasserstoff, Methyl oder Halogen bedeutet,

$R^2$ und $R^3$ zusammen mit dem Kohlenstoffatom an das sie gebunden sind einen ungesättigten 5-gliedrigen Ring bilden, der ein an den Pyrimidin-Ring gebundenes Sauerstoff- oder Schwefelatom enthält und der gegebenenfalls durch ($C_1$-$C_4$)Alkyl oder Halogen substituiert ist,

$R^4$ Wasserstoff bedeutet und

$R^5$ Wasserstoff, ($C_1$-$C_4$)Alkyl oder Cyclopropyl bedeutet,

Q die genannte Bedeutung für $Q^1$ hat.

**17.** Verbindungen gemäß Anspruch 1, dadurch gekennzeichnet, daß in Formel I

$R^1$ Wasserstoff, Methyl oder Halogen bedeutet,

$R^2$ und $R^3$ zusammen mit dem Kohlenstoffatom an das sie gebunden sind, einen ungesättigten 5-gliedrigen Ring bilden, der ein an den Pyrimidin-Ring gebundenes Sauerstoff- oder Schwefelatom enthält und der gegebenenfalls durch ($C_1$-$C_4$)Alkyl oder Halogen substituiert ist,

$R^4$ Wasserstoff bedeutet,

$R^5$ Wasserstoff, ($C_1$-$C_4$)Alkyl oder Cyclopropyl bedeutet,

Q die Bedeutung $Q^2$ hat und

$Q^2$ eine Gruppe der allgemeinen Formeln II, II', II'', II''' oder II'''' bedeutet, worin

D eine ($C_1$-$C_6$)Alkylengruppe bedeutet,

E eine direkte Bindung oder Methylenoxy bedeutet, und für den Fall, daß Q eine Gruppe der Formeln II', II'', II''' oder II'''' ist, E zusätzlich Sauerstoff bedeutet,

$R^9$ und $R^{10}$ gleich oder verschieden sind und jeweils Wasserstoff, Halogen, ($C_1$-$C_4$)Alkyl, ($C_3$-$C_6$)Cycloalkyl, ($C_1$-$C_4$)Halogenalkyl, $C_3$- oder $C_4$-Alkenyl, ($C_1$-$C_4$)Alkoxy, ($C_1$-$C_4$)-Alkylthio oder Nitro bedeuten,

$R^{11}$ die für $R^9$ und $R^{10}$ angegebenen Bedeutungen hat.

**18.** Verbindungen gemäß Anspruch 1, dadurch gekennzeichnet, daß in Formel I

$R^1$ Wasserstoff, Methyl oder Halogen bedeutet,

$R^2$ und $R^3$ zusammen mit dem Kohlenstoffatom an das sie gebunden sind einen ungesättigten 5-gliedrigen Ring bilden, der ein an den Pyrimidin-Ring gebundenes Sauerstoff- oder

Schwefelatom enthält und der gegebenenfalls durch $(C_1-C_4)$Alkyl oder Halogen substituiert ist,

$R^4$ Wasserstoff bedeutet,

$R^5$ Wasserstoff, $(C_1-C_4)$Alkyl oder Cyclopropyl bedeutet,

Q die Bedeutung $Q^3$ hat und

$Q^3$ eine Gruppe der allgemeinen Formel III bedeutet, worin $R^9$ und $R^{10}$ die genannten Bedeutungen haben,

U eine direkte Bindung, Sauerstoff, Schwefel, $(C_1-C_3)$Alkylen oder $(C_1-C_3)$Alkylenoxy bedeutet, und

$R^{14}$ Phenyl oder einen Heterocyclus bedeutet, wobei jeder der beiden vorgenannten Reste unsubstituiert oder mit einem oder zwei Substituenten versehen sein kann und diese Substituenten gleich oder verschieden sind und jeweils Halogen, $(C_1-C_4)$Alkyl, $(C_1-C_4)$Halogenalkyl, $(C_1-C_4)$Alkoxy, Nitro, $(C_1-C_4)$Alkylthio, $(C_1-C_4)$Alkoxy-$(C_1-C_4)$alkyl, Phenyl, Phenoxy, Halogenphenoxy oder $(C_1-C_4)$Alkylphenoxy bedeuten,

$R^{14}$ weiterhin, für den Fall, daß U Sauerstoff ist, $(C_5-C_{10})$Alkyl, Allyl, Geranyl, Farnesyl, $(C_1-C_4)$Halogenalkyl, $(C_3-C_6)$Cycloalkylmethyl, eine Ethylgruppe, die in 2-Stellung durch $(C_1-C_4)$Alkoxy, $(C_1-C_4)$Alkoxy-$(C_1-C_4)$Alkyl, $(C_1-C_4)$Alkylthio, $(C_1-C_4)$-Alkylsulfonyl oder eine Phenoxygruppe, die gegebenenfalls mit einer oder zwei $(C_1-C_4)$Alkylgruppen substituiert sein kann, Glycidyl, Acetonyl, Halogenphenoxymethyldioxolanyl, 2,2-Diethoxyethyl, 1-Ethoxycabonylmethyl, Trimethylsilylmethyl, 1-Pyridylethyl oder eine $(C_1-C_4)$Alkylgruppe die mit einer $(C_1-C_4)$Alkoxyimino- oder eine Benzyloxyiminogruppe substituiert ist, substituiert ist, bedeutet.

**19.** Verbindungen gemäß Anspruch 16, 17 oder 18, dadurch gekennzeichnet, daß in Formel I

$R^1$ Wasserstoff bedeutet und

$R^2$ und $R^3$ zusammen mit dem Kohlenstoffatom an das sie gebunden sind einen ungesättigten 5-gliedrigen Ring bilden, der ein an den Pyrimidin-Ring gebundenes Schwefelatom enthält.

**20.** Verfahren zur Herstellung von Verbindungen der Formel I gemäß Anspruch 1, dadurch gekennzeichnet, daß man eine Verbindung der allgemeinen Formel V

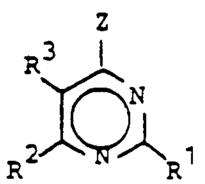

(V),

worin $R^1$, $R^2$ und $R^3$ die unter Formel I angegebenen Bedeutungen haben, und Z die Abgangsgruppe Halogen, Alkylthio, Alkansulfonyloxy,
Arylsulfonyloxy, Alkylsulfonyl oder Arylsulfonyl bedeutet, mit einem Amin der allgemeinen Formel VI umsetzt,

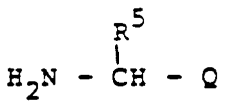

(VI),

worin $R^5$ und Q die unter Formel I angegebenen Bedeutungen haben, und die so erhaltenen Verbindungen der Formel I gegebenenfalls am Stickstoff carbamoyliert oder am $C_5$-Atom des Pyrimidins chloriert oder bromiert.

**21.** Verfahren zur Herstellung von Verbindungen der allgemeinen Formel I',

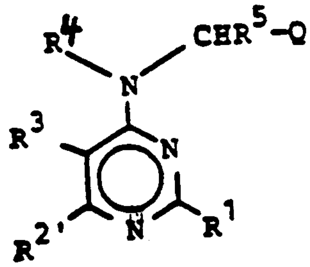

(I'),

worin $R^1$, $R^3$ $R^5$, und Q die unter Formel I angegebenen Bedeutungen haben $R^2$ $(C_1-C_4)$Alkoxy, $(C_1-C_4)$Halogenalkoxy, $(C_1-C_4)$Alkylthio, $(C_1-C_4)$Alkylamino oder $(C_1-C_4)$Dialkylamino bedeutet, dadurch gekennzeichnet, daß man eine Verbindung der allgemeinen Formel VII

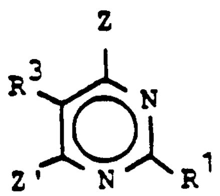

(VII),

worin Z und Z' gleich oder verschieden sein können, und die Abgangsgruppe Halogen, Alkylthio, Alkansulfonyloxy, Arylsulfonyloxy, Alkylsulfonyl oder Arylsulfonyl bedeuten und $R^1$ und $R^3$ die unter Formel I angegebenen Bedeutungen haben, mit einer Verbindung der allgemeinen Formel VI

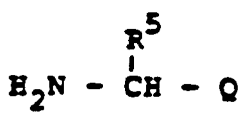

(VI),

worin $R^5$ und Q die unter Formel I angegebenen Bedeutungen haben, zu einer Verbindung der allgemeinen Formel VIII

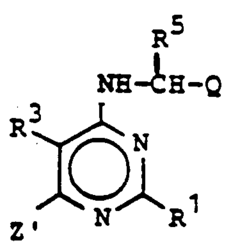

(VIII)

worin, $R^1$, $R^3$, $R^5$, Q und Z' die oben angegebenen Bedeutungen haben, umsetzt, in einem zweiten Reaktionsschritt die Verbindung der allgemeinen Formel VIII mit einer Verbindung der Formel IX umsetzt

$HR^{2'}$ (IX),

worin $R^{2'}$ die oben angegebenen Bedeutungen hat und die so erhaltene Verbindung der Formel I' gegebenenfalls am Stickstoff carbamoyliert oder gegebenenfalls, falls $R^3$ Wasserstoff bedeutet, chloriert oder bromiert.

22. Verfahren zur Herstellung einer Verbindung der Formel I' gemäß Anspruch 21, dadurch gekennzeichnet, daß man eine Verbindung der Formel VII mit einer Verbindung der Formel IX zu einer Verbindung der Formel X

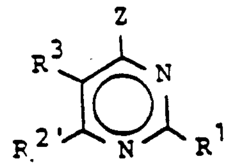

(X),

worin $R^1$ und $R^3$ die unter Formel I angegebenen Bedeutungen haben, $R^{2'}$ ($C_1$-$C_4$)Alkoxy, ($C_1$-$C_4$)-Halogenalkoxy, ($C_1$-$C_4$)Alkylthio, ($C_1$-$C_4$)Alkylamino oder ($C_1$-$C_4$)Dialkylamino bedeutet und Z die unter Formel V angegebenen Bedeutungen hat, umsetzt, die Verbindung der allgemeinen Formel X mit einem Amin der obengenannten Formel VI umsetzt und die so erhaltene Verbindung der Formel I' gegebenenfalls am Stickstoff carbamoyliert oder, falls $R^3$ Wasserstoff bedeutet, chloriert oder bromiert.

23. Insektizide oder akarizide Mittel, dadurch gekennzeichnet, daß sie eine wirksame Menge einer Verbindung der Formel I nach Anspruch 1 zusammen mit den üblichen Zusatz- und/oder Hilfsstoffen enthalten.

24. Fungizide Mittel, dadurch gekennzeichnet, daß sie eine wirksame Menge einer Verbindung der Formel I nach Anspruch 1 zusammen mit den üblichen Zusatz- und/oder Hilfsstoffen enthalten.

25. Nematozide Mittel, dadurch gekennzeichnet, daß sie eine wirksame Menge einer Verbindung der Formel I nach Anspruch 1 zusammen mit den üblichen Zusatz- und/oder Hilfsstoffen enthalten.

26. Verwendung der Verbindungen der Formeln I nach Anspruch 1 zur Bekämpfung von Schadinsekten, Akariden.

27. Verwendung der Verbindungen der Formel I nach Anspruch 1 zur Bekämpfung von Schadpilzen.

28. Verwendung der Verbindung der Formel I nach Anspruch 1 zur Bekämpfung von Nematoden.

29. Verfahren zur Bekämpfung von Schadinsekten, Akariden, dadurch gekennzeichnet, daß man auf diese oder die von ihren befallenen Pflanzen, Flächen oder Substrate eine wirksame Menge einer Verbindung der Formel I nach Anspruch 1 appliziert.

30. Verfahren zur Bekämpfung von Schadpilzen, dadurch gekennzeichnet, daß man auf diese oder die von ihren befallenen Pflanzen, Flächen oder Substrate eine wirksame Menge einer Verbindung der Formel I nach Anspruch 1 appliziert.

31. Verfahren zur Bekämpfung von Nematoden, dadurch gekennzeichnet, daß man auf diese oder die von ihren befallenen Pflanzen, Flächen oder Substrate eine wirksame Menge einer Verbindung der Formel I nach Anspruch 1 appliziert.

**Patentansprüche für folgenden Vertragsstaat : ES**

1. Verfahren zur Herstellung substituierter 4-Aminopyrimidine der allgemeinen Formel I

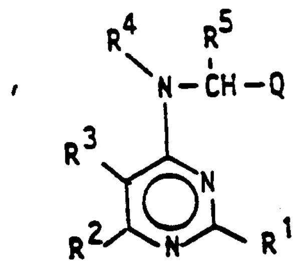

(I)

worin

$R^1$ Wasserstoff, Halogen, $(C_1\text{-}C_4)$Alkyl oder $(C_3\text{-}C_6)$Cycloalkyl bedeutet,

$R^2$ Wasserstoff, $(C_1\text{-}C_4)$Alkyl, Halogen, Trifluormethyl, $(C_1\text{-}C_4)$Alkoxy, $(C_1\text{-}C_4)$Halogenalkoxy, $(C_1\text{-}C_4)$Alkoxy-$(C_1\text{-}C_4)$alkyl, $(C_1\text{-}C_4)$Alkylthio, $(C_1\text{-}C_4)$Alkylthio-$(C_1\text{-}C_4)$alkyl, $(C_1\text{-}C_4)$Alkylamino, $(C_1\text{-}C_4)$Dialkylamino oder $(C_3\text{-}C_6)$Cycloalkylamino bedeutet,

$R^3$ Wasserstoff, $(C_1\text{-}C_4)$Alkyl, $(C_1\text{-}C_4)$Alkoxy, $(C_1\text{-}C_4)$Halogenalkoxy, $(C_1\text{-}C_4)$Alkylthio, Halogen, Nitro oder $(C_1\text{-}C_4)$Dialkylamino bedeutet, mit der Maßgabe, daß wenn Q keine der später aufgeführten Gruppen der Bedeutung $Q^1$ oder der allgemeinen Formeln II', II'', II''' oder II'''' ist und $R^3$ eine $(C_1\text{-}C_4)$Alkylgruppe oder ein Halogenatom bedeutet, $R^2$ nicht gleichzeitig $(C_1\text{-}C_4)$Alkyl, Halogen, $(C_1\text{-}C_4)$Alkoxy-$(C_1\text{-}C_4)$alkyl oder $(C_1\text{-}C_4)$Alkylthio-$(C_1\text{-}C_4)$alkyl bedeutet, oder, falls Q die Bedeutung $Q^1$ hat und $R^2$ eine Ethylgruppe bedeutet, $R^3$ nicht gleichzeitig Halogen bedeutet, oder, falls Q die Bedeutung $Q_1$ hat und $R^4$ einen Rest der Formel

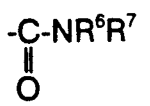

und $R^2$ $(C_1\text{-}C_4)$Alkyl oder Halogen bedeuten, $R^3$ nicht gleichzeitig $(C_1\text{-}C_4)$Alkyl oder Halogen ist, oder,

falls Q ein Rest der allgemeinen Bedeutung $Q^1$ oder der Formel II ist, für die E eine direkte Bindung oder Methylenoxy ist, oder der Formeln II', II'', II''' oder II'''' oder der Bedeutung $Q^3$ ist, für die $R^{14}$ keine Gruppe der allgemeinen Bedeutung IV ist, $R^2$ und $R^3$ auch zusammen mit den Kohlenstoffatomen an das sie gebunden sind, einen ungesättigten 5-gliedrigen Ring bilden können, der ein an den Pyrimidin-Ring gebundenes Sauerstoff- oder Schwefelatom enthält und der gegebenenfalls durch Alkyl oder Halogen substituiert ist,

$R^4$ Wasserstoff oder einen Rest

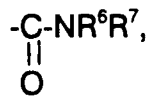

worin $R^6$ oder $R^7$ gleich oder verschieden sind und jeweils Wasserstoff, $(C_1\text{-}C_4)$Alkyl, Phenyl oder Phenyl$(C_1\text{-}C_4)$alkyl bedeuten,

wobei die beiden vorgenannten Phenylgruppen unsubstituiert oder mit einem oder zwei Substituenten versehen sind und diese Substituenten jeweils Halogen, $(C_1\text{-}C_4)$Alkyl, $(C_1\text{-}C_4)$-Halogenalkyl, $(C_1\text{-}C_4)$Alkoxy, $(C_1\text{-}C_4)$-Alkylthio oder Nitro sein können, oder $R^6$ und $R^7$ zusammen mit dem Stickstoffatom an das sie gebunden sind einen gesättigten oder ungesättigten 5- oder 6-gliedrigen Ring bilden, der zusätzlich weitere Stickstoffatome oder ein Sauerstoff- oder Schwefelatom enthalten kann, wobei dieser Ring mit einem Benzolring kondensiert und mit einem oder zwei Substituenten versehen sein kann und diese Substituenten jeweils $(C_1\text{-}C_4)$Alkyl, Trifluormethyl, Halogen, $(C_1\text{-}C_4)$Alkoxy, $(C_1\text{-}C_4)$Alkylthio oder Nitro sein können,

$R^5$ Wasserstoff, $(C_1\text{-}C_8)$Alkyl oder $(C_3\text{-}C_6)$Cycloalkyl bedeutet,

Q die Bedeutung $Q^1$ hat und

$Q^1$ $(C_1\text{-}C_{15})$Alkyl bedeutet, gegebenenfalls substituiert mit einem, zwei oder drei Halogenatomen, einer $(C_1\text{-}C_{15})$Alkoxygruppe, einer $(C_4\text{-}C_8)$Cycloalkylalkoxygruppe, einer Dioxolanylgruppe, einer $(C_1\text{-}C_4)$Alkoxy-$(C_1\text{-}C_4)$-alkoxygruppe, einer Hydroxygruppe, einer Methoxycarbonylgruppe, einer $(C_3\text{-}C_6)$-Cycloalkylgruppe, einer 2-[2-($(C_1\text{-}C_4)$Alkoxy)ethoxy]ethoxygruppe, einer Pyranyloxygruppe, einer Imidazol-1-ylgruppe, einer Triazol-1-ylgruppe, einer Pyrazol-1-ylgruppe oder einer Gruppe -A-B-$R^8$, worin A Sauerstoff, Schwefel oder Imino, B Carbonyl, Thiocarbonyl oder Sulfonyl bedeutet und $R^8$ $(C_1\text{-}C_4)$Alkyl, $(C_3\text{-}C_6)$Cycloalkyl, $(C_1\text{-}C_4)$Halogenalkyl, $(C_1\text{-}C_4)$Alkoxy, eine substituierte oder unsubstituierte Phenoxygruppe, eine substituierte oder unsubstituierte Phenylgruppe, eine substituierte oder unsubstituierte Anilinogruppe, eine 2,6-Dimethylmorpholin-4-yl-, 4-Methylpiperazin-1-yl-, Imidazol-1-yl-, Triazol-1-yl-, Pyrazol-1-ylgruppe oder eine Mono- oder Di-$(C_1\text{-}C_4)$alkylaminogruppe bedeutet, oder

Q die Bedeutung $Q^2$ hat und

$Q^2$ eine Gruppe der allgemeinen Formeln II, II', II'', II''' oder II'''' bedeutet

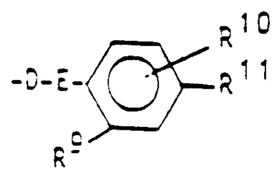

II ,

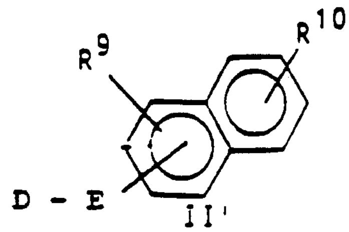

II' ,

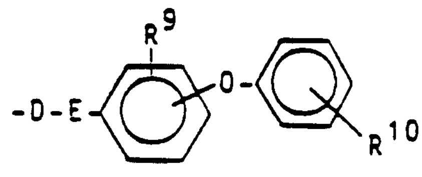

II''

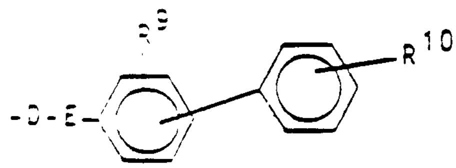

II'''

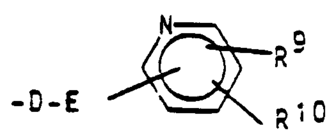

II''''

worin

D eine $(C_1\text{-}C_6)$Alkylengruppe bedeutet,

E eine direkte Bindung, Sauerstoff oder Methylenoxy bedeutet,

$R^9$ und $R^{10}$ gleich oder verschieden sind und jeweils Wasserstoff, Halogen, $(C_1\text{-}C_4)$Alkyl, $(C_3\text{-}C_6)$Cycloalkyl, $(C_1\text{-}C_4)$Halogenalkyl, $C_3$- oder $C_4$-Alkenyl, $(C_1\text{-}C_4)$Alkoxy, $(C_1\text{-}C_4)$-Alkylthio oder Nitro bedeuten,

$R^{11}$ die für $R^9$ und $R^{10}$ angegebenen Bedeutungen hat, sowie für den Fall, daß E Sauerstoff bedeutet, zusätzlich eine Gruppe der Formel X-Y-$(GO)_n$-$R^{12}$ bedeutet, worin

X eine $(C_1\text{-}C_8)$Alkylengruppe oder eine $(C_1\text{-}C_8)$Alkylengruppe mit einem $(C_1\text{-}C_4)$-Alkoxysubsituenten bedeutet,

Y Sauerstoff, Schwefel oder eine Iminogruppe bedeutet,

G eine $(C_1\text{-}C_8)$Alkylengruppe oder eine $(C_1\text{-}C_4)$Alkylenoxy-$(C_1\text{-}C_4)$alkylengruppe bedeutet,

n Null oder 1 ist,

$R^{12}$ $(C_1\text{-}C_4)$Alkyl, $C_3$- oder $C_4$-Alkenyl, $(C_4\text{-}C_6)$Alkadienyl, $C_3$- oder $C_4$-Alkinyl, Phenyl-$(C_1\text{-}C_3)$alkyl oder eine Gruppe der Formel $CH_2$-W bedeutet, worin

W eine Gruppe der Formel CH = N-$OR^{13}$ bedeutet, worin

$R^{13}$ Wasserstoff, $(C_1\text{-}C_4)$Alkyl, $C_3$- oder $C_4$-Alkenyl oder Phenyl$(C_1\text{-}C_3)$alkyl bedeutet,

W weiterhin eine Morpholinomethylgruppe oder eine heterocyclische Gruppe mit 5 bis 8 Ringatomen bedeutet, von denen 2 oder 3 Sauerstoff- oder Schwefelatome sind, und der besagte Heterocyclus gesättigt ist oder eine Doppelbindung im Ring besitzt und der besagte Heterocyclus unsubstituiert ist oder einen oder zwei Alkyl-, $(C_1\text{-}C_4)$-

Halogenalkyl- oder Phenylsubstituenten trägt, oder

Q die Bedeutung $Q^3$ hat und

$Q^3$ eine Gruppe der allgemeinen Formel III bedeutet

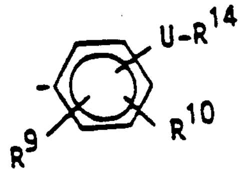

(III),

worin $R^9$ und $R^{10}$ die oben angegebenen Bedeutungen haben, und

U eine direkte Bindung, Sauerstoff, Schwefel, $(C_1-C_3)$Alkylen, oder $(C_1-C_3)$Alkylenoxy bedeutet,

$R^{14}$ Phenyl oder einen Heterocyclus bedeutet, wobei jeder der beiden vorgenannten Reste unsubstituiert oder mit einem oder zwei Substituenten versehen sein kann und diese Substituenten gleich oder verschieden sind und jeweils Halogen, $(C_1-C_4)$Alkyl, $(C_1-C_4)$Halogenalkyl, Nitro, $(C_1-C_4)$Alkoxy, $(C_1-C_4)$Alkylthio, $(C_1-C_4)$Alkoxy-$(C_1-C_4)$-alkyl, Phenyl, Phenoxy, Halogenphenoxy oder $(C_1-C_4)$Alkylphenoxy bedeuten,

$R^{14}$ weiterhin, für den Fall, daß U Sauerstoff ist, $(C_5-C_{10})$Alkyl, Allyl, Geranyl, Farnesyl, $(C_1-C_4)$Halogenalkyl,$(C_3-C_6)$Cycloalkylmethyl, eine Ethylgruppe, die in 2-Stellung durch $(C_1-C_4)$Alkoxy, $(C_1-C_4)$Alkoxy-$(C_1-C_4)$alkyl, $(C_1-C_4)$Alkylthio, $(C_1-C_4)$-Alkylsulfonyl oder eine Phenoxygruppe, die gegebenenfalls mit einer oder zwei $(C_1-C_4)$Alkylgruppen substituiert sein kann, Glycidyl, Acetonyl, Halogenphenoxymethyldioxolanyl, 2,2-Diethoxyethyl, 1-Ethoxycarbonylmethyl, Trimethylsilylmethyl, 1-Pyridylethyl oder eine $(C_1-C_4)$Alkylgruppe, die mit einer $(C_1-C_4)$Alkoxyimino- oder einer Benzyloxyiminogruppe substituiert ist, substituiert ist, bedeutet,

$R^{14}$ weiterhin, für den Fall, daß U Sauerstoff ist, eine Gruppe der allgemeinen Formel IV

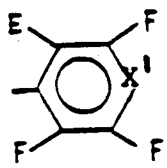

(IV)

bedeutet, worin

X' Stickstoff oder eine Gruppe $CR^{15}$ bedeutet, worin

$R^{15}$ Wasserstoff, Halogen, $(C_1-C_4)$Alkyl, $(C_1-C_4)$Halogenalkyl, $(C_1-C_4)$Alkanoyl, Nitro, Cyano oder 1,3-Dioxolan-2-yl bedeutet, sowie ihre Salze und ihre Stereoisomeren, dadurch gekennzeichnet, daß man eine Verbindung der allgemeinen Formel V

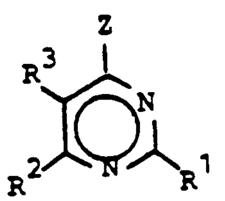

(V),

worin $R^1$, $R^2$ und $R^3$ die unter Formel I angegebenen Bedeutungen haben, und Z die Abgangsgruppe Halogen, Alkylthio, Alkansulfonyloxy, Arylsulfonyloxy, Alkylsulfonyl oder Arylsulfonyl bedeutet, mit einem Amin der allgemeinen Formel VI umsetzt,

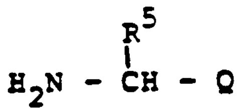

(VI),

worin $R^5$ und Q die unter Formel I angegebenen Bedeutungen haben, und die so erhaltenen Verbindungen der Formel I gegebenenfalls am Stickstoff carbamoyliert oder am $C_5$-Atom des Pyrimidins chloriert oder bromiert, und die so erhaltenen Verbindungen der Formel I gegebenenfalls in ihr Salz überführt.

**2.** Verfahren gemäß Anspruch 1, dadurch gekennzeichnet, daß in Formel I

$R^1$ Wasserstoff, Methyl oder Halogen bedeutet,

$R^2$ Wasserstoff, Methyl, Ethyl, Halogen, Trifluormethyl, $(C_1$-$C_4)$Alkoxy, $(C_1$-$C_4)$Halogenalkoxy, Methoxymethyl, Dimethylamino, oder Diethylamino bedeutet,

$R^3$ Wasserstoff, $(C_1$-$C_4)$Alkyl, $(C_1$-$C_4)$Alkoxy, $(C_1$-$C_4)$Halogenalkoxy oder Halogen bedeutet, mit der Maßgabe, daß wenn Q keine der Gruppen der Bedeutung $Q^1$ oder der allgemeinen Formeln II', II'', II''' oder II'''' ist und $R^3$ eine ,$(C_1$-$C_4)$Alkylgruppe oder ein Halogenatom bedeutet, $R^2$ nicht gleichzeitig Methyl, Ethyl, Halogen oder Methoxymethyl bedeutet, oder falls Q die Bedeutung $Q^1$ hat und $R^2$ eine Ethylgruppe bedeutet, $R^3$ nicht gleichzeitig Halogen bedeutet, oder,

falls Q die Bedeutung $Q^1$ hat und $R^4$ ein Rest der Formel

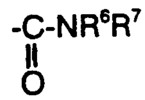

und $R^2$ Methyl, Ethyl oder Halogen bedeuten, $R^3$ nicht gleichzeitig $(C_1$-$C_4)$Alkyl oder Halogen ist, und

$R^5$ Wasserstoff, $(C_1$-$C_8)$Alkyl oder Cyclopropyl bedeutet.

**3.** Verfahren gemäß Anspruch 1 oder 2, dadurch gekennzeichnet, daß in Formel I

$R^3$ Wasserstoff, $(C_1$-$C_4)$Alkyl, $(C_1$-$C_4)$Alkoxy, $(C_1$-$C_4)$Halogenalkoxy oder Halogen bedeutet, mit der Maßgabe, daß wenn Q keine der Gruppen der Bedeutung $Q^1$ oder der allgemeinen Formeln II', II'', II''' oder II'''' ist und $R^3$ eine $(C_1$-$C_4)$Alkylgruppe oder ein Halogenatom bedeutet, $R^2$ nicht gleichzeitig Methyl, Ethyl oder Methoxymethyl bedeutet, oder,

falls Q die Bedeutung $Q_1$ hat und $R^2$ eine Ethylgruppe bedeutet, $R^3$ nicht gleichzeitig Halogen bedeutet, $R^2$ und $R^3$ auch zusammen mit den Kohlenstoffatomen an das sie gebunden sind, einen ungesättigten 5-gliedrigen Ring bilden können, der ein an den Pyrimidin-Ring gebundenes Sauerstoff- oder Schwefelatom enthält und der gegebenenfalls durch Alkyl oder Halogen substituiert ist, und

$R^4$ Wasserstoff bedeutet.

**4.** Verfahren gemäß Anspruch 1 oder 2, dadurch gekennzeichnet, daß in Formel I

$R^2$ Wasserstoff, Methyl, Ethyl, Halogen, Trifluormethyl, $(C_1$-$C_4)$Alkoxy, $(C_1$-$C_4)$Halogenalkoxy, Methoxymethyl, Dimethylamino oder Diethylamino bedeutet,

$R^3$ Wasserstoff, $(C_1$-$C_4)$Alkyl, $(C_1$-$C_4)$Alkoxy, $(C_1$-$C_4)$Halogenalkoxy oder, falls $R^3$ nicht Ethyl ist, auch Halogen bedeutet,

$R^4$ Wasserstoff bedeutet,

$R^5$ Wasserstoff, $(C_1$-$C_4)$Alkyl oder Cyclopropyl bedeutet,

Q die Bedeutung $Q^1$ hat und

$Q^1$ $(C_1$-$C_{15})$Alkyl bedeutet, gegebenenfalls substituiert mit einem, zwei oder drei Halogenatomen, einer $(C_1$-$C_{15})$Alkoxygruppe,

einer $(C_4$-$C_8)$Cycloalkylalkoxygruppe, einer Dioxolanylgruppe, einer $(C_1$-$C_4)$Alkoxy$(C_1$-$C_4)$-alkoxygruppe, einer Hydroxygruppe, einer

Methoxycarbonylgruppe, einer $(C_3$-$C_6)$Cycloalkylgruppe, einer 2-[2-($(C_1$-$C_4)$Alkoxy)ethoxy]-

ethoxygruppe, einer Pyranyloxygruppe, einer Imidazol-1-ylgruppe, einer Triazol-1-ylgruppe, einer Pyrazol-1-ylgruppe oder einer Gruppe -A-B-$R^8$, worin A Sauerstoff, Schwefel oder Imino, B Carbonyl, Thiocarbonyl oder Sulfonyl bedeutet und $R^8$ ($C_1$-$C_4$)Alkyl, ($C_3$-$C_6$)-Cycloalkyl, ($C_1$-$C_4$)Halogenalkyl, ($C_1$-$C_4$)Alkoxy, eine substituierte oder unsubstituierte Phenoxygruppe, eine substituierte oder unsubstituierte Phenylgruppe, eine substituierte oder unsubstituierte Anilinogruppe, eine 2,6-Dimethylmorpholin-4-yl-, 4-Methylpiperazin-1-yl-, Imidazol-1-yl-, Triazol-1-yl-, Pyrazol-1-ylgruppe oder eine Mono- oder Di-($C_1$-$C_4$)-alkylaminogruppe bedeutet.

**5.** Verfahren gemäß Anspruch 1 oder 2, dadurch gekennzeichnet, daß in Formel I

$R^2$ Wasserstoff, Methyl, Ethyl, Halogen, Trifluormethyl, Methoxy, Ethoxy, ($C_1$-$C_4$)-Halogenalkoxy, Methoxymethyl, Dimethylamino oder Diethylamino bedeutet,

$R^3$ Wasserstoff, ($C_1$-$C_4$)Alkyl, ($C_1$-$C_4$)Alkoxy, ($C_1$-$C_4$)Halogenalkoxy oder Halogen bedeutet, mit der Maßgabe, daß wenn Q keine der später aufgeführten Gruppen der allgemeinen Formeln II', II'', II''' oder II'''' ist und $R^3$ eine ($C_1$-$C_4$)Alkylgruppe oder ein Halogenatom bedeutet, $R^2$ nicht gleichzeitig Methyl, Ethyl, Halogen oder Methoxymethyl bedeutet,

$R^4$ Wasserstoff bedeutet,

$R^5$ Wasserstoff, ($C_1$-$C_4$)Alkyl oder Cyclopropyl, bedeutet,

Q die Bedeutung $Q^2$ hat und

$Q^2$ eine Gruppe der allgemeinen Formeln II, II', II'', II''' oder II'''' bedeutet, worin

D eine ($C_1$-$C_6$)Alkylengruppe bedeutet,

E eine direkte Bindung, Sauerstoff oder Methylenoxy bedeutet,

$R^9$ und $R^{10}$ gleich oder verschieden sind und jeweils Wasserstoff, Halogen, ($C_1$-$C_4$)Alkyl, ($C_3$-$C_6$)Cycloalkyl, ($C_1$-$C_4$)Halogenalkyl, $C_3$- oder $C_4$-Alkenyl, ($C_1$-$C_4$)Alkoxy, ($C_1$-$C_4$)-Alkylthio oder Nitro bedeuten,

$R^{11}$ die für $R^9$ und $R^{10}$ angegebenen Bedeutungen hat, sowie für den Fall, daß E Sauerstoff bedeutet, zusätzlich eine Gruppe der Formel X-Y-$(GO)_n$-$R^{12}$ bedeutet, worin X eine ($C_1$-$C_8$)Alkylengruppe oder eine ($C_1$-$C_8$)Alkylengruppe mit einem ($C_1$-$C_4$)Alkoxysubsituenten bedeutet,

Y Sauerstoff, Schwefel oder eine Iminogruppe bedeutet,

G eine($C_1$-$C_8$)Alkylengruppe oder eine ($C_1$-$C_4$)Alkylenoxy-($C_1$-$C_4$)alkylengruppe bedeutet,

n Null oder 1 ist,

$R^{12}$ ($C_1$-$C_4$)Alkyl, $C_3$- oder $C_4$-Alkenyl, ($C_4$-$C_6$)Alkadienyl, $C_3$- oder $C_4$-Alkinyl, Phenyl($C_1$-$C_3$)alkyl oder eine Gruppe der Formel $CH_2$-W bedeutet, worin

W eine Gruppe der Formel CH = N-$OR^{13}$ bedeutet, worin

$R^{13}$ Wasserstoff, ($C_1$-$C_4$)Alkyl, $C_3$- oder $C_4$-Alkenyl oder Phenyl-($C_1$-$C_3$)alkyl bedeutet, und

W weiterhin eine Morpholinomethylgruppe oder eine heterocyclische Gruppe mit 5 bis 8 Ringatomen bedeutet, von denen 2 oder 3 Sauerstoff- oder Schwefelatome sind, und der besagte Heterocyclus gesättigt ist oder eine Doppelbindung im Ring besitz und der besagte Heterocyclus unsubstituiert ist oder einen oder zwei Alkyl-, ($C_1$-$C_4$)-Halogenalkyl- oder Phenylsubstituenten trägt.

**6.** Verfahren gemäß Anspruch 1 oder 2, dadurch gekennzeichnet, daß in Formel I

$R^2$ Wasserstoff, Methyl, Ethyl, Halogen, Trifluormethyl, Methoxy, Ethoxy, ($C_1$-$C_4$)-Halogenalkoxy, Methoxymethyl, Dimethylamino oder Diethylamino bedeutet,

$R^3$ Wasserstoff, ($C_1$-$C_4$)Alkyl, ($C_1$-$C_4$)Alkoxy, ($C_1$-$C_4$)Halogenalkoxy oder Halogen bedeutet, mit der Maßgabe, daß wenn $R^3$ ($C_1$-$C_4$)Alkyl oder Halogen bedeutet, $R^2$ nicht gleichzeitig Halogen, Methyl, Ethyl oder Methoxymethyl bedeutet,

$R^4$ Wasserstoff bedeutet,

$R^5$ Wasserstoff, ($C_1$-$C_4$)Alkyl oder Cyclopropyl bedeutet,

Q die Bedeutung $Q^3$ hat und

$Q^3$ eine Gruppe der allgemeinen Formel III bedeutet, worin

$R^9$ und $R^{10}$ die genannten Bedeutungen haben,

U eine direkte Bindung, Sauerstoff, Schwefel, ($C_1$-$C_3$)Alkylen oder ($C_1$-$C_3$)Alkylenoxy bedeutet, und

$R^{14}$ Phenyl oder einen Hetercyclus bedeutet, wobei jeder der beiden vorgenannten Reste unsubstituiert oder mit einem oder zwei Substituenten versehen sein kann und diese Substituenten gleich oder verschieden sind und jeweils Halogen, $(C_1-C_4)$Alkyl, $(C_1-C_4)$Halogenalkyl, Nitro,
$(C_1-C_4)$Alkoxy, $(C_1-C_4)$Alkylthio, $(C_1-C_4)$Alkoxy-$(C_1-C_4)$alkyl, Phenyl, Phenoxy, Halogenphenoxy oder $(C_1-C_4)$Alkylphenoxy bedeuten, oder

$R^{14}$ weiterhin, für den Fall, daß U Sauerstoff ist, $(C_5-C_{10})$Alkyl, Allyl, Geranyl, Farnesyl, $(C_1-C_4)$Halogenalkyl, $(C_3-C_6)$Cycloalkylmethyl, eine Ethylgruppe, die in 2-Stellung durch $(C_1-C_4)$Alkoxy, $(C_1-C_4)$Alkoxy-$(C_1-C_4)$alkyl, $(C_1-C_4)$Alkylthio, $(C_1-C_4)$-Alkylsulfonyl oder eine Phenoxygruppe, die gegebenenfalls mit einer oder zwei $(C_1-C_4)$Alkylgruppen substituiert sein kann, Glycidyl, Acetonyl, Halogenphenoxymethyldioxolanyl, 2,2-Diethoxyethyl, 1-Ethoxycarbonylmethyl, Trimethylsilylmethyl, 1-Pyridylethyl oder eine $(C_1-C_4)$Alkylgruppe, die mit einer $(C_1-C_4)$Alkoxyimino- oder einer Benzyloxyiminogruppe substituiert ist, substituiert ist, bedeutet, oder

$R^{14}$ weiterhin, für den Fall, daß U Sauerstoff ist, eine Gruppe der allgemeinen Formel IV bedeutet, worin

X' Stickstoff oder eine Gruppe $CR^{15}$ bedeutet, worin

$R^{15}$ Wasserstoff, Halogen, $(C_1-C_4)$Alkyl, $(C_1-C_4)$Halogenalkyl, $(C_1-C_4)$Alkanoyl, Nitro, Cyano oder 1,3-Dioxolan-2-yl bedeutet.

**7.** Verfahren gemäß Anspruch 1 oder 2, dadurch gekennzeichnet, daß in Formel I

$R^2$ Wasserstoff, Halogen, Methoxy, Ethoxy, Trifluormethyl, Methoxymethyl, Dimethylamino oder Diethylamino bedeutet,

$R^3$ Wasserstoff, $(C_1-C_3)$Alkyl, Methoxy, Ethoxy, $(C_1-C_2)$Halogenalkoxy oder Halogen bedeutet, mit der Maßgabe, daß wenn Q keine der Gruppen der Bedeutung $Q^1$ oder der allgemeinen Formeln II', II'', II''' oder II'''' ist und $R^3$ eine $(C_1-C_3)$Alkylgruppe oder ein Halogenatom bedeutet, $R^2$ nicht gleichzeitig Halogen oder Methoxymethyl bedeutet, und

$R^4$ Wasserstoff bedeutet.

**8.** Verbindungen gemäß Anspruch 1 oder 2, dadurch gekennzeichnet, daß in Formel I

$R^2$ Wasserstoff, Halogen, Trifluormethyl, Methoxy, Ethoxy, Methoxymethyl, Dimethylamino oder Diethylamino bedeutet,

$R^3$ Wasserstoff, $(C_1-C_3)$Alkyl, Methoxy, Ethoxy, $(C_1-C_2)$Halogenalkoxy oder Halogen bedeutet,

$R^4$ Wasserstoff bedeutet,

Q die Bedeutung $Q^1$ hat und

$Q^1$ $(C_1-C_{15})$Alkyl bedeutet, gegebenenfalls substituiert mit einem, zwei oder drei Halogenatomen, einer $C_1-C_{15})$Alkoxygruppe, einer
$(C_4-C_8)$Cycloalkylalkoxygruppe, einer Dioxolanylgruppe, einer $(C_1-C_4)$Alkoxy-$(C_1-C_4)$-alkoxygruppe, einer Hydroxygruppe, einer Methoxycarbonylgruppe, einer $(C_3-C_6)$-Cycloalkylgruppe, einer 2-[2-($(C_1-C_4)$Alkoxy)ethoxy]ethoxygruppe, Pyranyloxygruppe, einer Imidazol-1-ylgruppe, einer Triazol-1-ylgruppe, einer Pyrazol-1-ylgruppe oder einer Gruppe -A-B-$R^8$, worin A Sauerstoff, Schwefel oder Imino, B Carbonyl, Thiocarbonyl oder Sulfonyl bedeutet und $R^8$ $(C_1-C_4)$Alkyl,
$(C_3-C_6)$Cycloalkyl, $(C_1-C_4)$Halogenalkyl, $(C_1-C_4)$Alkoxy, eine substituierte oder unsubstituierte Phenoxygruppe, eine substituierte oder unsubstituierte Phenylgruppe, eine substituierte oder unsubstituierte Anilinogruppe, eine 2,6-Dimethylmorpholin-4-yl-, 4-Methylpiperazin-1-yl-, Imidazol-1-yl-, Triazol-1-yl-, Pyrazol-1-ylgruppe oder Mono- oder Di-$(C_1-C_4)$alkylaminogruppe bedeutet.

**9.** Verfahren gemäß Anspruch 1 oder 2, dadurch gekennzeichnet daß in Formel I

$R^2$ Wasserstoff, Halogen, Methoxy, Ethoxy, Trifluormethyl, Methoxymethyl, Dimethylamino oder Diethylamino bedeutet,

$R^3$ Wasserstoff, $(C_1-C_3)$Alkyl, Methoxy, Ethoxy, $(C_1-C_2)$Halogenalkoxy oder Halogen bedeutet, mit der Maßgabe, daß wenn Q keine der später aufgeführten Gruppen der allgemeinen Formeln II', II'', II''' oder II'''' ist und $R^3$ eine $(C_1-C_3)$Alkylgruppe oder ein Halogenatom bedeutet, $R^2$ nicht gleichzeitig Halogen oder Methoxymethyl bedeutet,

$R^4$ Wasserstoff bedeutet,

Q die Bedeutung $Q^2$ hat und
$Q^2$ eine Gruppe der allgemeinen Formeln II, II', II'', II''' oder II'''' bedeutet, worin
D eine $(C_1-C_6)$Alkylengruppe bedeutet,
E eine direkte Bindung, Sauerstoff oder Methylenoxy bedeutet,
$R^9$ und $R^{10}$ gleich oder verschieden sind und jeweils Wasserstoff, Halogen, $(C_1-C_4)$Alkyl, $(C_3-C_6)$Cycloalkyl, $(C_1-C_4)$Halogenalkyl, $C_3$- oder $C_4$-Alkenyl, $(C_1-C_4)$Alkoxy, $(C_1-C_4)$-Alkylthio oder Nitro bedeuten,
$R^{11}$ die für $R^9$ und $R^{10}$ angegebenen Bedeutungen hat, sowie für den Fall, daß E Sauerstoff bedeutet, zusätzlich eine Gruppe der Formel $X-Y-(GO)_n-R^{12}$ bedeutet, worin X eine $(C_1-C_8)$Alkylengruppe oder eine $(C_1-C_8)$Alkylengruppe mit einem $(C_1-C_4)$Alkoxysubstituenten bedeutet,
Y Sauerstoff, Schwefel oder eine Iminogruppe bedeutet,
G eine $(C_1-C_8)$Alkylengruppe oder eine $(C_1-C_8)$Alkylenoxy-$(C_1-C_4)$alkylengruppe bedeutet,
n Null oder 1 ist,
$R^{12}$ $(C_1-C_4)$Alkyl, $C_3$- oder $C_4$-Alkenyl, $(C_4-C_6)$Alkadienyl, $C_3$- oder $C_4$-Alkinyl, Phenyl-$(C_1-C_3)$alkyl oder eine Gruppe der Formel $CH_2$-W bedeutet, worin
W eine Gruppe der Formel $CH=N-OR^{13}$ bedeutet, worin
$R^{13}$ Wasserstoff, $(C_1-C_4)$Alkyl, $C_3$- oder $C_4$-Alkenyl oder Phenyl-$(C_1-C_3)$alkyl bedeutet, und
W weiterhin eine Morpholinomethylgruppe oder eine heterocyclische Gruppe mit 5 bis 8 Ringatomen bedeutet, von denen 2 oder 3 Sauerstoff- oder Schwefelatome sind, und der besagte Heterocyclus gesättigt ist oder eine Doppelbindung im Ring besitzt und der besagte Heterocyclus unsubstituiert ist oder einen oder zwei Alkyl-, $(C_1-C_4)$-Halogenalkyl- oder Phenylsubstituenten trägt.

**10.** Verfahren gemäß Anspruch 1 oder 2, dadurch gekennzeichnet, daß in Formel I
$R^2$ Wasserstoff, Halogen, Methoxy, Ethoxy, Trifluormethyl, Methoxymethyl, Dimethylamino oder Diethylamino bedeutet,
$R^3$ Wasserstoff, $(C_1-C_3)$Alkyl, Methoxy, Ethoxy, $(C_1-C_2)$Halogenalkoxy oder Halogen bedeutet, mit der Maßgabe, daß wenn $R^3$ $(C_1-C_3)$Alkyl oder Halogen bedeutet, $R^2$ nicht gleichzeitig Halogen oder Methoxymethyl bedeutet,
$R^4$ Wasserstoff bedeutet,
Q die Bedeutung $Q^3$ hat und
$Q^3$ eine Gruppe der allgemeinen Formel III bedeutet,worin $R^9$ und $R^{10}$ die genannten Bedeutungen haben,
U eine direkte Bindung, Sauerstoff, Schwefel, $(C_1-C_3)$Alkylen oder $(C_1-C_3)$Alkylenoxy bedeutet, und
$R^{14}$ Phenyl oder einen Heterocyclus bedeutet, wobei jeder der beiden vorgenannten Reste unsubstituiert oder mit einem oder zwei Substituenten versehen sein kann und diese Substituenten gleich oder verschieden sind und jeweils Halogen, $(C_1-C_4)$Alkyl, $(C_1-C_4)$-Halogenalkyl, $(C_1-C_4)$Alkoxy, Nitro, $(C_1-C_4)$Alkylthio, $(C_1-C_4)$Alkoxy-$(C_1-C_4)$alkyl, Phenyl, Phenoxy, Halogenphenoxy oder $(C_1-C_4)$Alkylphenoxy bedeuten, oder
$R^{14}$ weiterhin, für den Fall, daß U Sauerstoff ist, $(C_5-C_{10})$Alkyl, Allyl, Geranyl, Farnesyl, $(C_1-C_4)$-Halogenalkyl, $(C_3-C_6)$Cycloalkylmethyl, eine Ethylgruppe, die in 2-Stellung durch $(C_1-C_4)$-Alkoxy, $(C_1-C_4)$Alkoxy-$(C_1-C_4)$alkyl, $(C_1-C_4)$Alkylthio, $(C_1-C_4)$Alkylsulfonyl oder eine Phenoxygruppe, die gegebenenfalls mit einer oder zwei $(C_1-C_4)$Alkylgruppen substituiert sein kann, Glycidyl, Acetonyl, Halogenphenoxymethyldioxolanyl, 2,2-Diethoxyethyl, 1-Ethoxycarbonylmethyl, Trimethylsilylmethyl, 1-Pyridylethyl oder eine $(C_1-C_4)$Alkylgruppe, die mit einer $(C_1-C_4)$Alkoxyimino- oder eine Benzyloxyiminogruppe substituiert ist, substituiert ist, bedeutet, und
$R^{14}$ weiterhin, für den Fall, daß U Sauerstoff ist, eine Gruppe der allgemeinen Formel IV bedeutet, worin
X' Stickstoff oder eine Gruppe der $CR^{15}$ bedeutet, worin
$R^{15}$ Wasserstoff, Halogen, $(C_1-C_4)$Alkyl, $(C_1-C_4)$Halogenalkyl, $(C_1-C_4)$Alkanoyl, Nitro, Cyano oder 1,3-Dioxolan-2-yl bedeutet.

**11.** Verfahren gemäß einem der Ansprüche 1 bis 10, dadurch gekennzeichnet, daß in Formel I

$R^1$ Wasserstoff bedeutet,
$R^2$ Methoxy oder Methoxymethyl bedeutet,
$R^3$ Methoxy bedeutet und
$R^4$ Wasserstoff bedeutet.

**12.** Verfahren gemäß Anspruch 11, dadurch gekennzeichnet, daß in Formel I
$R^5$ Wasserstoff, Methyl, Ethyl oder Cyclopropyl bedeutet.

**13.** Verfahren gemäß Anspruch 11 oder 12, dadurch gekennzeichnet, daß
$R^2$ Methoxymethyl bedeutet,
$R^5$ Wasserstoff, Methyl oder Cyclopropyl bedeutet,
Q die Bedeutung $Q^1$ hat und
$Q^1$ $(C_3-C_{13})$Alkyl bedeutet.

**14.** Verfahren gemäß Anspruch 11 oder 12, dadurch gekennzeichnet, daß in Formel I
$R^5$ Wasserstoff, Methyl oder Cyclopropyl bedeutet,
Q die Bedeutung $Q^2$ hat und
$Q^2$ eine Gruppe der allgemeinen Formeln II, II', II'', II''' oder II'''' bedeutet, worin
D eine $(C_1-C_2)$Alkylengruppe bedeutet,
E eine direkte Bindung oder Sauerstoff bedeutet,
$R^9$ und $R^{10}$ gleich oder verschieden sind und jeweils Wasserstoff, Halogen, $(C_1-C_4)$Alkyl, $(C_3-C_6)$Cycloalkyl, $(C_1-C_4)$Halogenalkyl, $(C_1-C_4)$Alkoxy, bedeuten,
$R^{11}$ die für $R^9$ und $R^{10}$ angegebenen Bedeutungen hat, sowie für den Fall, daß E Sauerstoff bedeutet, zusätzlich eine Gruppe der Formel $X-Y-(GO)_n-R^{12}$ bedeutet, worin
X eine $(C_1-C_8)$Alkylengruppe bedeutet,
Y Sauerstoff bedeutet,
G eine Ethylengruppe bedeutet,
n Null oder 1 ist und
$R^{12}$ $(C_1-C_4)$Alkyl, bedeutet.

**15.** Verfahren gemäß Anspruch 11 oder 12, dadurch gekennzeichnet, daß in Formel I
Q die Bedeutung $Q^3$ hat und
$Q^3$ eine Gruppe der allgemeinen Formel III bedeutet, worin $R^9$ und $R^{10}$ die genannten Bedeutungen haben,
U Sauerstoff bedeutet und
$R^{14}$ Phenyl oder einen Heterocyclus bedeutet, wobei jeder der beiden vorgenannten Reste unsubstituiert oder mit einem oder zwei Substituenten versehen sein kann und diese Substituenten gleich oder verschieden sind und jeweils Halogen, $(C_1-C_4)$Alkyl, $(C_1-C_4)$-Halogenalkyl, $(C_1-C_4)$Alkoxy, Nitro, $(C_1-C_4)$Alkylthio, $(C_1-C_4)$Alkoxy-$(C_1-C_4)$alkyl, Phenyl, Phenoxy, Halogenphenoxy oder $(C_1-C_4)$Alkylphenoxy bedeuten, oder
$R^{14}$ weiterhin, für den Fall, daß U Sauerstoff ist, $(C_5-C_{10})$Alkyl, Allyl, Geranyl, Farnesyl, $(C_1-C_4)$-Halogenalkyl oder $(C_3-C_6)$Cycloalkylmethyl, bedeutet oder
$R^{14}$ weiterhin, für den Fall, daß U Sauerstoff ist, eine Gruppe der allgemeinen Formel IV bedeutet, worin
X' Stickstoff oder eine Gruppe der Formel CF bedeutet.

**16.** Verfahren gemäß Anspruch 1, dadurch gekennzeichnet, daß in Formel I
$R^1$ Wasserstoff, Methyl oder Halogen bedeutet,
$R^2$ und $R^3$ zusammen mit dem Kohlenstoffatom an das sie gebunden sind einen ungesättigten 5-gliedrigen Ring bilden, der ein an den Pyrimidin-Ring gebundenes Sauerstoff- oder Schwefelatom enthält und der gegebenenfalls durch $(C_1-C_4)$Alkyl oder Halogen substituiert ist,
$R^4$ Wasserstoff bedeutet und
$R^5$ Wasserstoff, $(C_1-C_4)$Alkyl oder Cyclopropyl bedeutet,
Q die genannte Bedeutung für $Q^1$ hat.

**17.** Verfahren gemäß Anspruch 1, dadurch gekennzeichnet, daß in Formel I

$R^1$ Wasserstoff, Methyl oder Halogen bedeutet,
$R^2$ und $R^3$ zusammen mit dem Kohlenstoffatom an das sie gebunden sind, einen ungesättigten 5-gliedrigen Ring bilden, der ein an den Pyrimidin-Ring gebundenes Sauerstoff- oder Schwefelatom enthält und der gegebenenfalls durch $(C_1-C_4)$Alkyl oder Halogen substituiert ist,
$R^4$ Wasserstoff bedeutet,
$R^5$ Wasserstoff, $(C_1-C_4)$Alkyl oder Cyclopropyl bedeutet,
Q die Bedeutung $Q^2$ hat und
$Q^2$ eine Gruppe der allgemeinen Formeln II, II', II'', II''' oder II'''' bedeutet, worin
D eine $(C_1-C_6)$Alkylengruppe bedeutet,
E eine direkte Bindung oder Methylenoxy bedeutet, und für den Fall, daß Q eine Gruppe der Formeln II', II'', II''' oder II'''' ist, E zusätzlich Sauerstoff bedeutet,
$R^9$ und $R^{10}$ gleich oder verschieden sind und jeweils Wasserstoff, Halogen, $(C_1-C_4)$Alkyl, $(C_3-C_6)$Cycloalkyl, $(C_1-C_4)$Halogenalkyl, $C_3$- oder $C_4$-Alkenyl, $(C_1-C_4)$Alkoxy, $(C_1-C_4)$-Alkylthio oder Nitro bedeuten,
$R^{11}$ die für $R^9$ und $R^{10}$ angegebenen Bedeutungen hat.

**18.** Verfahren gemäß Anspruch 1, dadurch gekennzeichnet, daß in Formel I
$R^1$ Wasserstoff, Methyl oder Halogen bedeutet,
$R^2$ und $R^3$ zusammen mit dem Kohlenstoffatom an das sie gebunden sind einen ungesättigten 5-gliedrigen Ring bilden, der ein an den Pyrimidin-Ring gebundenes Sauerstoff- oder Schwefelatom enthält und der gegebenenfalls durch $(C_1-C_4)$Alkyl oder Halogen substituiert ist,
$R^4$ Wasserstoff bedeutet,
$R^5$ Wasserstoff, $(C_1-C_4)$Alkyl oder Cyclopropyl bedeutet,
Q die Bedeutung $Q^3$ hat und
$Q^3$ eine Gruppe der allgemeinen Formel III bedeutet, worin $R^9$ und $R^{10}$ die genannten Bedeutungen haben,
U eine direkte Bindung, Sauerstoff, Schwefel, $(C_1-C_3)$Alkylen oder $(C_1-C_3)$Alkylenoxy bedeutet, und
$R^{14}$ Phenyl oder einen Heterocyclus bedeutet, wobei jeder der beiden vorgenannten Reste unsubstituiert oder mit einem oder zwei Substituenten versehen sein kann und diese Substituenten gleich oder verschieden sind und jeweils Halogen, $(C_1-C_4)$Alkyl, $(C_1-C_4)$Halogenalkyl, $(C_1-C_4)$Alkoxy, Nitro, $(C_1-C_4)$Alkylthio, $(C_1-C_4)$Alkoxy-$(C_1-C_4)$alkyl, Phenyl, Phenoxy, Halogenphenoxy oder $(C_1-C_4)$Alkylphenoxy bedeuten,
$R^{14}$ weiterhin, für den Fall, daß U Sauerstoff ist, $(C_5-C_{10})$Alkyl, Allyl, Geranyl, Farnesyl, $(C_1-C_4)$Halogenalkyl, $(C_3-C_6)$Cycloalkylmethyl, eine Ethylgruppe, die in 2-Stellung durch $(C_1-C_4)$Alkoxy, $(C_1-C_4)$Alkoxy-$(C_1-C_4)$Alkyl, $(C_1-C_4)$Alkylthio, $(C_1-C_4)$-Alkylsulfonyl oder eine Phenoxygruppe, die gegebenenfalls mit einer oder zwei $(C_1-C_4)$Alkylgruppen substituiert sein kann, Glycidyl, Acetonyl, Halogenphenoxymethyldioxolanyl, 2,2-Diethoxyethyl, 1-Ethoxycabonylmethyl, Trimethylsilylmethyl, 1-Pyridylethyl oder eine $(C_1-C_4)$Alkylgruppe, die mit einer $(C_1-C_4)$Alkoxyimino- oder eine Benzyloxyiminogruppe substituiert ist, substituiert ist, bedeutet.

**19.** Verfahren gemäß Anspruch 16, 17 oder 18, dadurch gekennzeichnet, daß in Formel I
$R^1$ Wasserstoff bedeutet und
$R^2$ und $R^3$ zusammen mit dem Kohlenstoffatom an das sie gebunden sind einen ungesättigten 5-gliedrigen Ring bilden, der ein an den Pyrimidin-Ring gebundenes Schwefelatom enthält, dadurch gekennzeichnet, daß man eine Verbindung der allgemeinen Formel V

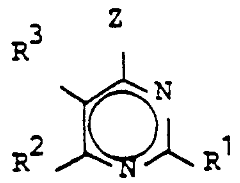
(V),

worin $R^1$, $R^2$ und $R^3$ die unter Formel I angegebenen Bedeutungen haben, und Z die Abgangsgruppe Halogen, Alkylthio, Alkansulfonyloxy Arylsulfonyloxy, Alkylsulfonyl oder Arylsulfonyl bedeutet, mit einem Amin der allgemeinen Formel VI umsetzt,

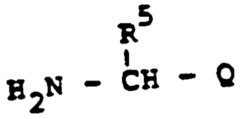

(VI),

worin $R^5$ und Q die unter Formel I angegebenen Bedeutungen haben, und die so erhaltenen Verbindungen der Formel I gegebenenfalls am Stickstoff carbamoyliert oder am $C_5$-Atom des Pyrimidins chloriert oder bromiert, und die so erhaltenen Verbindungen der Formel I gegebenenfalls in ihr Salz überführt.

**20.** Verfahren zur Herstellung von Verbindungen der allgemeinen Formel I',

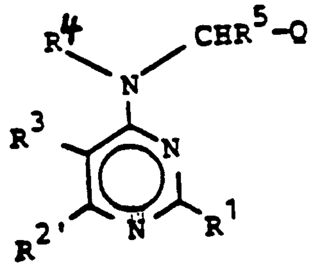

(I'),

worin $R^1$, $R^3$ $R^5$, und Q die unter Formel I angegebenen Bedeutungen haben $R^{2'}$ $(C_1\text{-}C_4)$Alkoxy, $(C_1\text{-}C_4)$Halogenalkoxy, $(C_1\text{-}C_4)$Alkylthio, $(C_1\text{-}C_4)$Alkylamino oder $(C_1\text{-}C_4)$Dialkylamino bedeutet, dadurch gekennzeichnet, daß man eine Verbindung der allgemeinen Formel VII

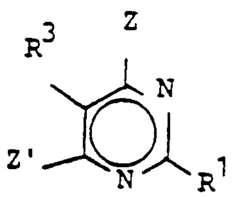

(VII),

worin Z und Z' gleich oder verschieden sein können, und die Abgangsgruppe Halogen, Alkylthio, Alkansulfonyloxy, Arylsulfonyloxy, Alkylsulfonyl oder Arylsulfonyl bedeuten und $R^1$ und $R^3$ die unter Formel I angegebenen Bedeutungen haben, mit einer Verbindung der allgemeinen Formel VI

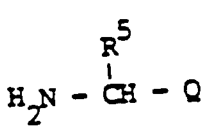

(VI)

worin $R^5$ und Q die unter Formel I angegebenen Bedeutungen haben, zu einer Verbindung der allgemeinen Formel VIII

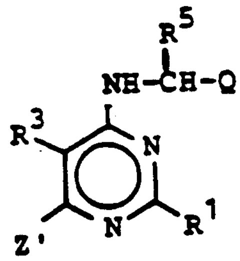

(VIII)

worin, $R^1$, $R^3$, $R^5$, Q und Z' die oben angegebenen Bedeutungen haben, umsetzt, in einem zweiten Reaktionsschritt die Verbindung der allgemeinen Formel VIII mit einer Verbindung der Formel IX umsetzt

$HR^{2'}$ (IX),

worin $R^{2'}$ die oben angegebenen Bedeutungen hat, umsetzt und die so erhalten Verbindung der Formel I' gegebenenfalls am Stickstoff carbamoyliert oder gegebenenfalls, falls $R^3$ Wasserstoff bedeutet, chloriert oder bromiert.

**21.** Verfahren zur Herstellung einer Verbindung der Formel I' gemäß Anspruch 20, dadurch gekennzeichnet, daß man eine Verbindung der Formel VII mit einer Verbindung der Formel IX zu einer Verbindung der Formel X

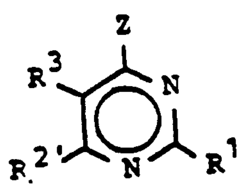

(X),

worin $R^1$ und $R^3$ die unter Formel I angegebenen Bedeutungen haben, $R^{2'}$ ($C_1$-$C_4$)Alkoxy, ($C_1$-$C_4$)-Halogenalkoxy, ($C_1$-$C_4$)Alkylthio, ($C_1$-$C_4$)Alkylamino oder ($C_1$-$C_4$)Dialkylamino bedeutet und Z die unter Formel V angegebenen Bedeutungen hat, umsetzt, die Verbindung der allgemeinen Formel X mit einem Amin der obengenannten Formel VI umsetzt und die so erhaltene Verbindung der Formel I' gegebenenfalls am Stickstoff carbamoyliert oder, falls $R^3$ Wasserstoff bedeutet, chloriert oder bromiert.

**22.** Verfahren zur Herstellung eins insektiziden oder akariziden Mittels, dadurch gekennzeichnet, daß man eine wirksame Menge einer Verbindung der Formel 1 nach Anspruch 1 zusammen mit den üblichen Zusatz- und/oder Hilfsstoffen in eine für diese Anwendung geeignete Form bringt.

**23.** Verfahren zur Herstellung eines fungiziden Mittels, dadurch gekennzeichnet, daß man eine wirksame Menge einer Verbindung der Formel I nach Anspruch I zusammen mit den üblichen Zusatz- und/oder Hilfsstoffen in eine für diese Anwendung geeignete Form bringt.

**24.** Verfahren zur Herstellung nematoziden Mittels, dadurch gekennzeichnet, daß man eine wirksame Menge einer Verbindung der Formel I nach Anspruch 1 zusammen mit den üblichen Zusatz- und/oder Hilfsstoffen in eine für diese Anwendung geeignete Form bringt.

**25.** Verwendung der Verbindungen der Formeln I nach Anspruch 1 zur Bekämpfung von Schadinsekten, Akariden.

**26.** Verwendung der Verbindungen der Formel I nach Anspruch 1 zur Bekämpfung von Schadpilzen.

**27.** Verwendung der Verbindung der Formel I nach Anspruch 1 zur Bekämpfung von Nematoden.

**28.** Verfahren zur Bekämpfung von Schadinsekten, Akariden, dadurch gekennzeichnet, daß man auf diese oder die von ihren befallenen Pflanzen, Flächen oder Substrate eine wirksame Menge einer Verbindung der Formel I nach Anspruch 1 appliziert.

**29.** Verfahren zur Bekämpfung von Schadpilzen, dadurch gekennzeichnet, daß man auf diese oder die von ihren befallenen Pflanzen, Flächen oder Substrate eine wirksame Menge einer Verbindung der Formel I nach Anspruch 1 appliziert.

**30.** Verfahren zur Bekämpfung von Nematoden, dadurch gekennzeichnet, daß man auf diese oder die von ihren befallenen Pflanzen, Flächen oder Substrate eine wirksame Menge einer Verbindung der Formel I nach Anspruch 1 appliziert.

Europäisches Patentamt

# EUROPÄISCHER RECHERCHENBERICHT

Nummer der Anmeldung

EP 92 10 8143

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 331 529 (UBE INDUSTRIES)<br>* Seite 7; Seite 13, Beispiel 4; Seiten 16-22; Ansprüche *<br>--- | 1,5,6, 20-31 | C 07 D 239/42<br>C 07 D 239/46<br>C 07 D 239/48<br>C 07 D 239/52<br>C 07 D 401/12<br>A 01 N 43/54 |
| D,X | EP-A-0 264 217 (UBE INDUSTRIES)<br>* Seiten 6,13-39; Ansprüche *<br>--- | 1,10,20 -31 | |
| D,X | EP-A-0 356 158 (UBE INDUSTRIES)<br>* Seiten 2,6,7; Seite 11 - Seite 27 (Tabelle I); Ansprüche *<br>--- | 1,2,20-31 | |
| D,X | EP-A-0 370 704 (UBE INDUSTRIES)<br>* Das ganze Dokument *<br>--- | 1,5,6, 15,18, 20-31 | |
| X | CHEMICAL ABSTRACTS, Band 69, 1968, Seiten 9049-9050, Zusammenfassung Nr. 96644m, Columbus, Ohio, US; V.N. SOKOLOVA et al: "N-(4-Pyrimidyl)ethylamine derivates. II. 5-Allyl- and 5-p-chlorophenylpyrimidines", & KHIM. GETEROTSIKL. SOEDIN. 1968, (3), 519-23<br>* Abstract *<br>--- | 1,20 | **RECHERCHIERTE SACHGEBIETE (Int. Cl.5)** |
| P,X | EP-A-0 470 600 (CIBA-GEIGY)(12-02-1992)<br>* Seiten 23-48; Ansprüche *<br>--- | 1,20-31 | C 07 D 239/00<br>C 07 D 401/00 |
| X | GB-A-2 043 061 (ICI)<br>* Seiten 1-7; Ansprüche *<br>--- | 1,20-31 | |
| X | DE-A-2 263 052 (WACKER-CHEMIE)<br>* Das ganze Dokument *<br>--- -/- | 1,4,8, 20-31 | |

Der vorliegende Recherchenbericht wurde für alle Patentansprüche erstellt

| Recherchenort | Abschlußdatum der Recherche | Prüfer |
|---|---|---|
| DEN HAAG | 26-08-1992 | FRANCOIS J.C.L. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 92 10 8143

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | CHEMICAL ABSTRACTS, Band 101, 1984, Seite 712, Zusammenfassung Nr. 130700t, Columbus, Ohio, US; & JP-A-59 36 666 (SANKYO) 28-02-1984 * Abstract * --- | 1,23,24 | |
| X | CHEMICAL ABSTRACTS, Band 101, 1984, Seite 660, Zusammenfassung Nr. 110939z, Columbus, Ohio, US; & JP-A-59 36 667 (SANKYO) 28-02-1984 * Abstract * --- | 1,23,24 | |
| P,A | EP-A-0 453 137 (NIHON NOHYAKU)(23-10-1991) * Das ganze Dokument * --- | 1,5,9, 20-31 | |
| D,A | EP-A-0 276 406 (BAYER) * Ansprüche * --- | 1,20-31 | |
| D,A | EP-A-0 323 757 (UBE INDUSTRIES) * Ansprüche * --- | 1,20-31 | RECHERCHIERTE SACHGEBIETE (Int. Cl.5) |
| D,A | EP-A-0 196 524 (SANKYO CO., LTD et al.) * Ansprüche * --- | 1,20-31 | |
| A | US-A-5 002 949 (S.M. PESECKIS et al.) * Spalten 1-8 * ----- | 1 | |

Der vorliegende Recherchenbericht wurde für alle Patentansprüche erstellt

| Recherchenort | Abschlußdatum der Recherche | Prüfer |
|---|---|---|
| DEN HAAG | 26-08-1992 | FRANCOIS J.C.L. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)